(19) Europäisches Patentamt
European Patent Office
Office européen des brevets

(11) EP 1 809 279 B1

(12) **EUROPEAN PATENT SPECIFICATION**

(45) Date of publication and mention of the grant of the patent:
**29.05.2013 Bulletin 2013/22**

(21) Application number: **05851355.7**

(22) Date of filing: **03.11.2005**

(51) Int Cl.:
***A61K 31/404*** *(2006.01)* ***A61K 9/00*** *(2006.01)*
***A61P 35/00*** *(2006.01)*

(86) International application number:
**PCT/US2005/039922**

(87) International publication number:
**WO 2006/052712 (18.05.2006 Gazette 2006/20)**

(54) **PARTICULATE COMPOSITIONS OF TUBULIN INHIBITOR**

PARTIKELZUSAMMENSETZUNGEN EINES TUBULINHEMMERS

COMPOSITIONS PARTICULAIRES D'INHIBITEURS DE LA TUBULINE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **08.11.2004 US 626036 P**
**11.01.2005 US 642878 P**

(43) Date of publication of application:
**25.07.2007 Bulletin 2007/30**

(73) Proprietors:
- **Baxter International Inc.
Deerfield, Illinois 60015 (US)**
- **Baxter Healthcare S.A.
8152 Glattpark (Opfikon) (CH)**

(72) Inventors:
- **PAPADOPOULOS, Pavlos
Antioch, Illinois 60002 (US)**
- **DOTY, Mark
Grayslake, Illinois 60030 (US)**
- **KIPP, James E.
Wauconda, Illinois 60084 (US)**
- **ROESSLER, Berthold
Halle , Westfalen 33790 (DE)**

(74) Representative: **Crowhurst, Charlotte Waveney
Potter Clarkson LLP
The Belgrave Centre
Talbot Street
Nottingham NG1 5GG (GB)**

(56) References cited:
**EP-A1- 1 044 683 EP-A2- 1 269 994
US-B1- 6 232 327**

- **LAVELLE F: "AMERICAN ASSOCIATION FOR CANCER RESEARCH 1999: 10-14 APRIL, PHILADELPHIA, PENNSYLVANIA" EXPERT OPINION ON INVESTIGATIONAL DRUGS, ASHLEY PUBLICATIONS LTD., LONDON, GB, vol. 8, no. 6, 1999, pages 903-909, XP001014727 ISSN: 1354-3784**
- **BACHER GERALD ET AL: "D-24851, a novel synthetic microtubule inhibitor, exerts curative antitumoral activity in vivo, shows efficacy toward multidrug-resistant tumor cells, and lacks neurotoxicity" CANCER RESEARCH, AMERICAN ASSOCIATION FOR CANCER RESEARCH, BALTIMORE, MD, US, vol. 61, no. 1, 2001, pages 392-399, XP002173885 ISSN: 0008-5472**
- **BROXTERMAN H J ET AL: "CANCER RESEARCH 2001: DRUG RESISTANCE, NEW TARGETS AND DRUG COMBINATIONS" DRUG RESISTANCE UPDATES, CHURCHILL LIVINGSTONE, EDINBURGH, GB, vol. 4, 2001, pages 197-209, XP008011921 ISSN: 1368-7646**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 809 279 B1

## Description

The present invention relates to nano- and micro-particulate formulations of indole tubulin inhibitors, methods of manufacture and methods of use. Preferred indole tubulin inhibitors comprise N-substituted indol-3-glyoxyamides and, more preferably, N-(Pyridin-4-yl)-[1-(4-chlorobenzyl)-indol-3-yl]glyoxylic acid amide (D-24851), also known as "Indibulin." While particulate compositions of the indole tubulin inhibitors can be prepared by a variety of methods, preferred methods involve precipitating the tubulin inhibitor compound in an aqueous medium in the presence of surfactant(s) to form a pre-suspension, followed by adding energy to yield a desired size distribution of nanoparticles in a suspension. The compositions are useful for various treatments and preferably for the treatment of anti-tumor agent resistant cancers and other diseases.

### Background of the Invention

A. Background Regarding Nanoparticles of Poorly Soluble Drugs

There is an ever increasing number of drugs being formulated that are poorly soluble or insoluble in aqueous solutions. Such drugs are a challenge to formulate in an injectable form for parenteral administration. Drugs that are insoluble in water, however, can provide the significant benefit of stability when formulated as a suspension of sub-micron particles in an aqueous medium. Accurate control of particle size is essential for safe and efficacious use of these formulations. Particles generally must be less than seven microns in diameter to safely pass through capillaries without causing emboli (Allen et al., 1987; Davis and Taube, 1978; Schroeder et al., 1978; Yokel et al., 1981).

One approach to delivering an insoluble drug is disclosed in U.S. Pat. No. 2,745,785. This patent discloses a method for preparing tabular or plate-like crystals of penicillin G, N,N'-dibenzylethylenediamine salts suitable for parenteral administration. The method includes the step of re-crystallizing the penicillin G from a formamide solution by adding water to reduce the solubility of the penicillin G. The '785 patent further provides that the penicillin G salt particles can be coated with wetting agents such as lecithin, emulsifiers, surface-active, de-foaming agents, partial higher fatty acid esters of sorbitan, polyoxyalkylene derivatives thereof, and aryl alkyl polyether alcohols or salts thereof. The '785 patent further discloses micronizing the penicillin G with an air blast under pressure to form crystals ranging from about 5 to 20 microns.

Another approach, disclosed in U.S. Pat. No. 5,118,528, describes a process for preparing nanoparticles. The process includes the steps of: (1) preparing a liquid phase of a substance in a solvent or a mixture of solvents to which may be added one or more surfactants, (2) preparing a second liquid phase of a non-solvent or a mixture of non-solvents, the non-solvent is miscible with the solvent or mixture of solvents for the substance, (3) adding together the solutions of (1) and (2) with stirring; and (4) removing of unwanted solvents to produce a colloidal suspension of nanoparticles. The '528 patent discloses particles smaller than 500 nm prepared without the supply of energy. In particular the '528 patent states that it is undesirable to use high-energy equipment such as sonicators and homogenizers.

U.S. Pat. No. 4,826,689 discloses a method for making uniformly sized particles from water-insoluble drugs or other organic compounds. First, a suitable solid organic compound is dissolved in an organic solvent, and the solution can be diluted with a non-solvent. Then, an aqueous precipitating liquid is infused, precipitating non-aggregated particles with substantially uniform mean diameter. The particles are then separated from the organic solvent. Depending on the organic compound and the desired particle size, the parameters of temperature, ratio of non-solvent to organic solvent, infusion rate, stir rate, and volume can be varied according to the invention. This process forms a drug in a metastable state which is thermodynamically unstable and which eventually converts to a more stable crystalline state. The drug is trapped in a metastable state in which the free energy lies between that of the starting drug solution and the stable crystalline form. The '689 patent discloses utilizing crystallization inhibitors (e.g., polyvinylpyrrolidinone) and surface-active agents (e.g., poly(oxyethylene)-co-oxypropylene)) to render the precipitate stable enough to be isolated by centrifugation, membrane filtration or reverse osmosis.

U.S. Pat. Nos. 5,091,188; 5,091,187 and 4,725,442 disclose (a) either coating small drug particles with natural or synthetic phospholipids or (b) dissolving the drug in a suitable lipophilic carrier and forming an emulsion stabilized with natural or semisynthetic phospholipids. One disadvantage of these approaches is they rely on the quality of the raw material of the drug and do not disclose steps of changing the morphology of the raw material to render the material in a friable, more easily processed form.

Another approach to providing formulations of insoluble drugs for parenteral delivery is disclosed in U.S. Pat. No. 5,145,684. The '684 patent discloses the wet milling of an insoluble drug in the presence of a surface modifier to provide a drug particle having an average effective particle size of less than 400 nm. The surface modifier is adsorbed on the surface of the drug particle in an amount sufficient to prevent agglomeration into larger particles.

Yet another attempt to provide insoluble drug formulations for parenteral delivery is disclosed in U.S. Pat. No. 5,922,355. The '355 patent discloses providing submicron sized particles of insoluble drugs using a combination of

surface modifiers and a phospholipid, followed by particle size reduction using techniques such as sonication, homogenization, milling, microfluidization, precipitation or recrystallization. There is no disclosure in the ’355 patent of changing process conditions to make crystals in a more friable form.

U.S. Pat. No. 5,780,062 discloses a method of preparing small particles of insoluble drugs by (1) dissolving the drug in a water-miscible first solvent, (2) preparing a second solution of a polymer and an amphiphile in an aqueous second solvent in which the drug is substantially insoluble whereby a polymer/amphiphile complex is formed and (3) mixing the solutions from the first and second steps to precipitate an aggregate of the drug and polymer/amphiphile complex.

U.S. Pat. No. 5,858,410 discloses a pharmaceutical nanosuspension suitable for parenteral administration. The ’410 patent describes a method of subjecting at least one solid, therapeutically active compound dispersed in a solvent to high pressure homogenization in a piston-gap homogenizer. The particles formed have an average diameter, determined by photon correlation spectroscopy (PCS), of 10 nm to 1000 nm, and the proportion of particles larger than 5 microns in the total population being less than 0.1% (number distribution determined with a Coulter counter), without prior conversion into a melt. The examples in the ’410 patent disclose jet milling prior to homogenization. Use of solvents is discouraged in that such use results in the formation of crystals that are too large.

U.S. Pat. No. 4,997,454 discloses a method for making uniformly sized particles from solid compounds. The method includes the steps of dissolving the solid compound in a suitable solvent followed by infusing precipitating liquid, thereby precipitating non-aggregated particles with substantially uniform mean diameter. The particles are then separated from the solvent. The ’454 patent discourages forming particles in a crystalline state because during the precipitating procedure the crystal can dissolve and recrystallize, thereby broadening the particle size distribution range. The ’454 patent encourages trapping the particles in a metastable particle state during the precipitating procedure.

U.S. Pat. No. 5,605,785 discloses a process for forming nanoamorphous dispersions of photographically useful compounds. The process of forming nanoamorphous dispersions includes any known process of emulsification that produces a disperse phase having amorphous particulates.

U.S. 2002/0127278A1 discloses a method for preparing submicron-sized particles of organic compounds.

U.S. Patent No. 6,607,784 discloses a method for preparing submicron sized particles of an organic compound, the solubility of which is greater in a water-miscible first solvent than in a second solvent which is aqueous, the process including the steps of (i) dissolving the organic compound in the water-miscible first solvent to form a solution, (ii) mixing the solution with the second solvent to define a pre-suspension; and (iii) adding energy to the pre-suspension to form particles having an average effective particle size of 400 nm to 2 microns.

B. Background Regarding Indole Derivatives and Their Use as Antitumor Agents

U.S. Publication No. 2002/0091124A1 discloses indole and heteroindole derivatives and their use as antitumor agents.

U.S. Pat. Nos. 6,008,231; 6,232,327 and 6,693,119 disclose N-substituted indole-3-glyoxylamides, methods of preparation and their use for the treatment of cancer, asthma, allergy, and for use as immunosuppressants. The compounds are particularly useful in the treatment of antitumor agent resistant tumors, metastasizing carcinoma including development and spread of metastases, tumors sensitive to angiogenesis inhibitors or tumors that are both antitumor agent resistant and sensitive to angiogenesis inhibitors.

U.S. Publication No. 2003/0195244A1 discloses indole compounds and their use for treatment of cancer and angiogenesis-related disorders. There is no disclosure in 2003/0195244A1 describing the preparation or use of nanoparticulate formulations of such derivatives.

U.S. Publication No. 2004/0033267A1 discloses nanoparticulate compositions comprising angiogenesis inhibitors. C. Background Regarding Tubulin Inhibitors.

During mitosis, a cell’s DNA is replicated and then divided into two new cells. The process of separating the newly replicated chromosomes into the two forming cells involves spindle fibers constructed with microtubules, which themselves are formed by long chains of smaller protein subunits called tubulins. Spindle microtubules attach to replicated chromosomes and pull one copy to each side of the dividing cell. Without these microtubules, cell division is not possible. See Cancerquest (2003): "Cancer Treatments - Chemotherapy" www.cancerquest.org/index.cfm?page=520 or similar webiste.

Microtubules therefore are among the most important sub-cellular targets of anticancer chemotherapeutics because they are present in all cells and are necessary for mitotic, interphase and cell maintenance functions (e.g. intracellular transport, development and maintenance of cell shape, cell motility, and possibly distribution of molecules on cell membranes). Compounds that interact with tubulin can interfere with the cell cycle by causing tubulin precipitation and sequestration, thereby interrupting many important biologic functions that depend on the microtubular class of subcellular organelles. Therefore, such compounds can potentially inhibit the proliferation of tumor cell lines derived from various organs. See, e.g., Bacher et al. (2001) Pure Appl. Chem. 73:91459-1464 and Rowinsky & Donehower

(1991) Pharmac. Ther. 52:35-84.

One class of well-characterized and clinically used antimitotic drugs is of natural origin, namely, the taxanes (paclitaxel, docetaxel), vinca alkaloids (vincristine, vinblastine, vinorelbine) and podophyllotoxins/colchicine. These agents either inhibit the polymerization of tubulin (vinca alkaloids/cholchicine) or prevent the disassembly of microtubules (taxanes). A major drawback of taxanes and vinca alkaloids is the development of neurotoxicity since the drugs interfere with the function of microtubules in axons, which mediate the neuronal vesicle transport.

Epothilone A and B and their analogs exhibit high cytotoxicity and good stabilization of microtubules. These natural products were originally isolated from myxobacteria. Their unique capability to inhibit taxol-resistant tumor cell lines and their good solubility are the biggest advantages as compared to taxanes. However, the complicated chemical structures and limited access to the natural resources, in combination with the development of drug resistance, limit the potential of these natural products in general.

Other natural products or derived analogs are characterized by increased solubility or potency, but still are complicated in chemical structure.

D. Background Regarding Indibulin

New, synthetic, small-molecule chemical entities that bind to tubulin, but are neither a substrate of transmembrane pumps nor interfere with the function of axonal microtubules, would strongly increase the therapeutic index in the treatment of malignancies.

A series of synthetic molecules that bind to tubulin are currently being evaluated in the preclinical or early clinical stage. Among them is a synthetic compound, *N*-(Pyridin-4-yl)-[1-(4-chlorobenzyl)-indol-3-yl]glyoxylic acid amide, named D-24851, and also known as "Indibulin."

D-24851 is a synthetic small molecule indole tubulin inhibitor with significant antitumor activity *in vitro* and *in vivo.* It destabilizes microtubules in tumor cells, as well as in a cell-free system. The binding site of D-24851 does not appear to overlap with the tubulin-binding sites of the well-characterized microtubule-destabilizing agents vincristine or colchicine. Futhermore, the molecule selectively blocks cell cycle progression at metaphase.

*In vitro,* D-24851 exerts significant antitumor activity against a variety of malignancies (e.g., prostate, brain, breast, pancreas, and colon). D-24851 displays high in-*vivo* antineoplastic efficacy in animals. Based on its mechanism of action it is expected to target all types of solid tumors. It also is expected to exhibit antiasthmatic, antiallergic, immunosuppресsant and immunomodulating actions. No neurological symptoms have so far been found in animal experiments. In preclinical experiments in rodents the compound was very well tolerated at highly effective doses. Another advantage for further development is, in contrast to other tubulin-inhibitory compounds, its easy synthesis.

Other tubulin inhibiting compounds from the indole chemical class have also been identified as potential drug candidates having similar modes of action to Indibulin including D-64131, a 2-arylindole derivative, as described in "New Small-Molecule Tubulin Ishibitors", Pure Appl. Chem., Vol. 73, No. 9, 2001.

Summary of the Invention

The present invention provides a particulate composition according to claim 1 and a method of making a particulate composition according to claim 26.

The present invention is directed to particulate compositions of specific indole-based, tubulin inhibitors. Preferred compositions comprise an aqueous suspension of nanoparticles of indole-based, tubulin inlubitors coated with at least one surfactant selected from the group consisting of ionic surfactants, non-ionic surfactants, zwitterionic surfactants, biologically derived surfactants and amino acids.

The compositions can be administered to animals, particularly human beings. The compositions and their associated methods of administration provide numerous benefits including the agility to deliver the compositions via parenteral or oral administration, reduced toxicity and improved bioavailability. Further, since the particles (e.g., nanoparticles) of the present invention constitute a high proportion of autitubulin agents, the nanosuspensions of the present invention contain a significantly reduced concentration of excipients, such as surfactants or other solubilizers, that otherwise would be needed in larger amounts to solubilize the agent for administration. The reduction in excipient levels allows for significantly higher dosing of active agent (since complications caused by excipients are reduced with reduced concentrations of excipients). Moreover, preferred suspensions of the present invention contain little to no solvents, allowing for greater dosing of the active agent while reducing solvent toxicity to the subject

In providing the present formulations, many disadvantages of the prior art can be avoided. Such disadvantages include toxicity, ineffectiveness against multi-drug resistant (MDR) tumors, low absorption rate, poor bioavailability and complicated chemical structure (making synthesis difficult).

The present invention is also directed to methods of making particulate compositions of the tubulin inhibitors, by preparing particles of at least one tubulin inhibitor compound and, optionally, at least one surfactant, and formulating

the resulting particles in a suitable vehicle for administration Preferred methods are directed to the preparation of aqueous based, nanosuspensions of tubulin inhibitors for parenteral administration.

The composition of the invention can be used to treat a mammal, preferably a human subject, by administering a therapeutically effective amount of the anti-tubulin suspension. Preferably, the administered composition will provide anticancer, antiasthmatic, antiallergic, immunouppresant, or immunomodulating activity. Most preferred uses are directed to the administration of Indibulin nanosupensions for the treatment of cancer.

Other advantages and aspects of the present invention will become apparent upon reading the following detailed description of the invention.

Brief Description of the Figures

FIG. 1 is a graph comparing D-24851 plasma levels after intravenous injection of Compositions 4 and 5;

FIG. 2 is a graph showing the mean plasma concentrations of D-24851 following intravenous administration in dogs - Day 1 (Composition 4);

FIG. 3 is a graph showing the mean plasma concentrations of D-24851 following intravenous administration to dogs - Day 27 (Composition 4);

FIG. 4 depicts Method "A," a preferred process for making particle suspensions; and

FIG. 5 depicts schematically Method "B," a preferred process for making particle suspensions.

FIG. 6. is a graph comparing D-24851 nanosuspension (Composition 4) dose dependency in Rat AH13 tumor model with a control solution.

FIG. 7. is a graph showing the plasma concentrations after intravenous administration of different doses of D-24851 nanosuspension (Composition 4) in rats.

FIG. 8. is a graph showing the plasma concentrations after intravenous administration of D-24851 nanosuspension (Composition 4) on Day 1 and Day 15. in rats.

Detailed Description of The Invention

The present invention is described herein using several definitions, as set forth below and throughout the application.

"About" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

"Bioavailability" with respect to the pharmocokinetic performance of pharmaceutical compositions is commonly used in the art to describe the in vivo performance of a formulation. The parameters that are commonly used in the art to describe the in vivo performance of a formulation (or the bioavailbility) are $C_{max}$, the maximum concentration of the active in the blood; $T_{max}$, the elapsed time after dosing that the drug reaches the $C_{max}$; and AUC (area under curve), a measure of the total amount of drug absorbed by the patient. Thus, "improved bioavailability," with respect to a nanosuspension of the present invention, refers to an improved performance (e.g., improved $C_{max}$, $T_{max}$, AUC or other performance criteria) of such nanosuspension relative to formulations other than nanoparticulate compositions for a given indole tubulin inhibitor of the present invention. This improved bioavailability also applies to multiple dosing regimens of the nanosuspensions of the present invention relative to multiple dosing regimens of other formulations containing the same drug. Depending on the drug dosed, the patient being dosed and the severity of condition of the patient to be treated, the $C_{max}$, $T_{max}$, AUC or other performance criteria values may be either increased or decreased in order to obtain improved bioavailability. For example, if the $C_{max}$ for a given drug needed to be reduced in order to improve the effectiveness of the drug (i.e., efficacy and safety), then nanosuspensions of the present invention that, when administered, reduced the $C_{max}$ relative to other administered formulations containing the same drug would have improved bioavailability. Likewise, if $T_{max}$ needs to be increased in order to improve effectiveness of a drug, then nanosuspensions of the present invention increasing that parameter would have improved bioavailability.

"Coated," with respect to a surfactant or other excipient of a particulate (e.g., nano- or micro-particulate) composition, refers to the presence of such compound at, or approximately on, the surface of the particle. A particle "coated" with such compound may be partially or fully covered with the compound and such compound may or may not be partially entrained within the particle.

"Friable" refers to particles that are fragile and are more easily broken down into smaller particles.

"Microsuspension" refers to a suspension of microparticles, and "microparticles" refers to particles of active agent having a mean particle size of about 200nm to about 5 microns, unless otherwise specified.

"Nanosuspension" refers to a suspension of nanoparticles, and "nanoparticles" and "nanoparticulate" refer to

particles of active agent having a mean particle size of about 15 nm to about 2 microns, unless otherwise specified. "Particle suspension" refers to a suspension of particles that can be of various size distributions.

As used herein, "particle size" or "size" (with reference to particles) is determined on the basis of volume-weighted average particle size as measured by conventional particle size measuring techniques well known to those skilled in the art. Such techniques include, for example, sedimentation field flow fractionation, photon correlation spectroscopy, light scattering, disk centrifugation, light microscopy or electron microscopy.

"Presuspension" refers to a solid dispersion that may be amorphous, semi-crystalline, or crystalline, and which has not be reduced sufficiently in size to the desired range and/or requires an input of energy to stabilize the solid dispersion.

"Poorly water soluble" means that the water solubility of the compound is less than about 10 mg/ml.

With reference to stable drug particles, "stable" means that tubulin inhibitor particles do not appreciably flocculate or agglomerate or otherwise increase in particle size.

"Sustained-release" refers to the administration of a nanosuspension of the present invention wherein the effective concentration of the active pharmaceutical ingredient in the bloodstream following such administration is maintained for a relatively long period of time, or a longer period relative to the period of effective concentration following administration of other formulations containing the same active pharmaceutical ingredient.

"Therapeutically effective amount" refers to drug dosage amounts that generally provide an ameliorative effect on the dosed subject. It is emphasized that, due to the variability of disease state and individual response, a "therapeutically effective amount" of a composition of the present invention administered to a particular subject in a particular instance will not always be effective in treating the diseases described herein, even though such dosage is deemed a "therapeutically effective amount" by those skilled in the art. It is to be further understood that drug dosages are, in particular instances, measured as parenteral or oral dosages, or with reference to drug levels as measured in either blood or plasma.

"Tolerability" refers to an individual's ability to receive administration of a nanosuspension of the present invention (containing an active pharmaceutical ingredient) continuously, in bolus, in multiple doses or in doses larger than those administered through other formulations of the same active pharmaceutical ingredient, without injurious or undesired effects, or with reduced injurious or undesired effects relative to the effects of administration of such other formulations on the individual, whether such formulations are dosed continuously, in bolus or in a multiple dosing regimen.

<u>Compounds/Particles</u>

The following terms shall have meaning in the description of the invention:

The term "free hydroxy group" means an OH group. The term "functionally modified hydroxy group" means an OH group that has been functionalized to form: an ether, in which an alkyl, aryl, cycloalkyl, heterocycloalkyl, alkenyl, cycloalkenyl, heterocycloalkenyl, acylalkyl, alkynyl, or heteroaryl group is substituted for the hydrogen; an ester, in which an acyl group is substituted for the hydrogen; a carbamate, in which an aminocarbonyl group is substituted for the hydrogen; or a carbonate, in which an aryloxy-, heteroaryloxy-, alkoxy-, cycloalkoxy-, heterocycloalkoxy-, alkenyloxy-, cycloalkenyloxy-, heterocycloalkenyloxy-, or alkynyloxy-carbonyl group is substituted for the hydrogen. Preferred moieties include OH, $OCH_2 C(O)CH_3$, $OCH_2 C(O)C_2 H_5$, $OCH_3$, $OCH_2 CH_3$, $OC(O)CH_3$, and $OC(O)C_2 H_5$.

The term "acyl" represents a group that is linked by a carbon atom that has a double bond to an oxygen atom and a single bond to another carbon atom.

The term "alkyl" includes straight or branched chain aliphatic hydrocarbon groups that are saturated, that is, they contain no carbon-carbon double bonds. The alkyl groups may be interrupted by one or more heteroatoms, such as oxygen, nitrogen, or sulfur, and may be substituted with other groups, such as halogen, hydroxyl, aryl, cycloalkyl, aryloxy, or alkoxy. Preferred straight or branched alkyl groups include methyl, ethyl, propyl, isopropyl, butyl, sec-butyl, isobutyl, and t-butyl.

The term "carbonyl group" represents a carbon atom double bonded to an oxygen, atom, wherein the carbon atom has two free valencies.

The term "halogen" represents fluoro, chloro, bromo, or iodo.

The term "aryl" refers to carbon-based rings that are aromatic. The rings may be isolated, such as phenyl, or fused, such as naphthyl. The ring hydrogens may be substituted with other groups, such as alkyl, halogen, free or functonalized hydroxy, trihalomethyl, etc.

Examples of aryl groups include phenyl, and substituted phenyl groups such as 2-, 3-, or 4-balophenyl, alkylphenyl, and 3-(trifluoromethyl)phenyl..

The term "arylalkyl" refers to an alkyl group in which at least one of the hydrogens on the alkyl substituent is replaced by an aryl group. Examples include benzyl groups, and substituted benzyl groups such as 2-, 3-, or (4-halophenyl) methyl, and (4-alkylphenyl)methyl.

The term "heteroaryl" refers to aromatic hydrocarbon rings which contain at least one heteroatom such as O, S, or N in the ring. Heteroaryl rings may be isolated, with 5 to 6 ring atoms, or fused, with 8 to 10 atoms. The heteroaryl ring(s) hydrogens or heteroatoms with open valency may be substituted with other groups, such as alkyl or halogen. Examples of heteroaryl groups include imidazole, pyridine, indole, quinoline, furan, thiophene, benzothiophene, pyrrole, pyrazole, oxazole, isoxazole, thiazole, tetrahydroquinoline, benzofuran, dihydrobenzofuran, and dihydrobenzindole.

The terms "aryloxy", "heteroaryloxy", "alkoxy", "cycloalkoxy", "heterocycloalkoxy" "alkenyloxy", "cycloalkenyloxy" and "heterocycloalkenyloxy" represent an aryl, heteroaryl, alkyl, cycloalkyl, heterocycloalkyl, alkenyl, cycloalkenyl, heterocycloalkenyl, or alkynyl group, respectively, attached through an oxygen linkage.

The terms "alkoxycarbonyl", "aryloxycarbonyl", "heteroaryloxycarbonyl", "cycloalkoxycarbonyl", "heterocycloalkoxycarbonyl", "alkenyloxycarbonyl", "Cycloalkenyloxycarbonyl" and "heterocycloalkenyloxycarbonyl" represent an alkoxy, aryloxy, heteroaryloxy, cycloalkoxy, heterocycloalkoxy, alkenyloxy, cycloalkenyloxy or heterocycloalkenyloxy, respectively, bonded from its oxygen atom to the carbon of a carbonyl group, the carbonyl group itself being bonded to another atom through its carbon atom.

The indole tubulin inhibitor compounds of the present invention are of the general Formula (1):

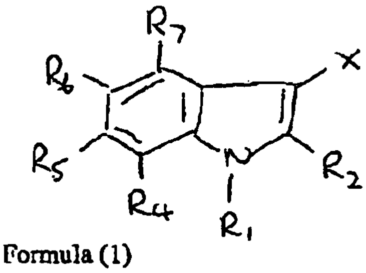

**Formula (1)**

wherein:

X is

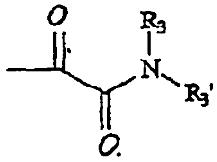

,

$R_3$ is aryl or heteroaryl, $R_{3'}$ is hydrogen,

$R_4$, $R_5$, $R_6$ and $R_7$ are independently hydrogen, halogen, or alkyl;

$R_1$ is arylalkyl ; and

$R_2$ is hydrogen, alkyl, acyl, aryl, alkoxycarbonyl, aryloxycarbonyl, heteroaryloxycarbonyl, cycloalkoxycarbonyl, heterocycloalkoxycarbonyl, alkenyloxycarbonyl, cycloalkenyloxycarbonyl and heterocycloalkenyloxycarbonyl.

Preferably, $R_1$ is a substituted benzyl group, more preferably a halogenated benzyl group (2-, 3-, or (4-halophenyl)methyl), and most preferably a (4-chlorophenyl)methyl group.

Preferably, $R_4$, $R_5$, $R_6$, and $R_7$ are hydrogen atoms.

Preferably, $R_3$ is a pyridinyl group (pyridine ring). More preferably, $R_3$ is a 4-pyridinyl group.

A preferred species of indole tubulin inhibitors of the present invention are those described in U.S. Patent No. 2003/0195244 (particularly N-substituted and 3-substituted).

A preferred species of indole tubulin inhibitors of the present invention are those described in U.S. Publication No. 2002/0091124A1 (2-acyl indoles).

A most preferred species of indoles of the present invention are those described in U.S. Pat. Nos. 6,008,231; 6,232,327 and 6,693,119 (N-substituted indole-3-glyoxylamides).

The most preferred indole of the present invention is D-24851, having the chemical structure of Formula 2:

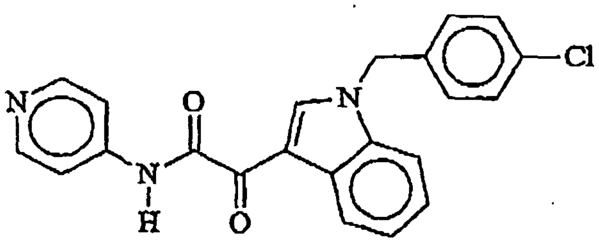

Formula (2).

The indoles of the present invention can be synthesized by methods known to those skilled in the art and as disclosed in the foregoing patents and publications.

One or more tubulin inhibitors are present in a composition of the present invention in an amount of from about 0.01% to about 20% weight to volume (w/v), preferably from about 0.05% to about 15% w/v, and more preferably from about 0.1 % to about 10% w/v.

The particles of the present invention will vary in size distribution depending on a number of factors including the active agent, surfactants present, route of administration and dosing regimen. In general, the particles will have a size distribution of from about 15 nm to 50 microns, preferably from about 50 nm to 10 microns and more preferably from about 50 nm to 2 microns. When the particles are prepared for injectable administration, they will have an effective particle size. Preferably, such particles will be less than about 5 microns in size (microparticles), and more preferably, less than about 2 microns in size (nanoparticles).

Surfactants/Suspensions

Suitable surfactants for coating the particles in the present invention can be selected from ionic surfactants, nonionic surfactants, zwitterionic surfactants, phospholipids, biologically derived surfactants or amino acids and their derivatives. Ionic surfactants can be anionic or cationic. The surfactants are present in the compositions in an amount of from about 0.01% to 10% w/v, and preferably from about 0.05% to about 5% w/v.

Suitable anionic surfactants include : alkyl sulfonates, aryl sulfonates, alkyl phosphates, alkyl phosphonates, potassium laurate, sodium lauryl sulfate, sodium dodecylsulfate, alkyl polyoxyethylene sulfates, sodium alginate, phosphatidic acid and their salts, sodium carboxymethylcellulose, bile acids and their salts (e.g., salts of cholic acid, deoxycholic acid, glycocholic acid, taurocholic acid, and glycodeoxycholic acid), and calcium carboxymethylcellulose, stearic acid and its salts (e.g., sodium and calcium stearate), phosphates, sodium dodecylsulfate, carboxymethylcellulose calcium, carboxymethylcellulose sodium, dioctyl sodium sulfosuccinate (DOSS), dialkylesters of sodium sulfosuccinic acid, sodium lauryl sulfate and phospholipids.

Suitable cationic surfactants include: quaternary ammonium compounds, benzalkonium chloride, cetyltrimethylammonium bromide, chitosans, lauryldimethylbenzylammonium chloride, acyl carnitine hydrochlorides, alkyl pyridinium halides, cetyl pyridinium chloride, cationic lipids, polymethylmethacrylate trimethylammonium bromide, sulfonium compounds, pnlyvinylpynolidone-2-dimethylaminoethyl methacrylate dimethyl sulfate, hexadecyltrimethyl ammonium bromide, phosphonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, $C_{12-15}$-dimethyl hydroxyethyl ammonium chloride, $C_{12-15}$-dimethyl hydroxyethyl ammonium bromide, coconut dimethyl hydroxyethyl ammonium chloride, coconut dimethyl hydroxyethyl ammonium bromide, myristyl trimethyl ammonium methyl sulfate, lauryl dimethyl benzyl ammonium chloride, lauryl dimethyl benzyl ammonium bromide, lauryl dimethyl $(ethenoxy)_4$ ammonium chloride, lauryl dimethyl $(ethenoxy)_4$ ammonium bromide, N-alkyl $(C_{12-18})$climethylbenzyl ammonium chloride, N-alkyl $(C_{14-18})$dimethyl-benzyl ammonium chloride, N-tetradecyldimethylbenzyl ammonium chloride monohydrate, dimethyl didecyl ammonium chloride, N-alkyl $(C_{12-14})$ dimethyl 1-naphthylmethyl ammonium chloride, trimethylammonium halide alkyl-trimethylammonium salts, dialkyldimethylammonium salts, lauryl trimethyl ammonium chloride, ethoxylated alkylamidoalkylammonium salts, ethoxylated trialkyl ammonium salts, dinlkylbenzene dialkylammonium chloride, N-didecyldimethyl ammonium chloride, N-tatradecyldimethylbenzyl ammonium chloride monohydrale, N-alkyl$(C_{12-14})$ dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, $C_{12}$ trimethyl ammonium bromides, $C_{15}$ trimethyl ammonium bromides, $C_{17}$ trimethyl ammonium bromides, dodecylbenzyl triethyl ammonium chloride, poly-diallyldanethylammonium chloride (DADMAC™). dimethyl aminonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, "POLYQUAT 10" (a mixture of polymeric quaternary ammonium compounds),, tetrabutylammonium bromide, benzyl triraethylammonium bromide, choline esters, stearalkonium chloride, cetyl pyridinium bromide, cetyl pyridinium chloride, halide salts of quaternized polyoxyethylalkylamines, alkyl pyridinium salts, amines, amine salts, imide azolinium salts, protonated quaternary acrylamides, methylated quaternary polymers, cationic guar gum, dodecyl trimethyl ammonium bromide, triethanolamine, and poloxamines.

Suitable nonionic surfactants include: polyoxyethylene fatty alcohol ethers, polyoxyethylene sorbitan fatty acid esters, polyoxyethylene fatty acid esters, sorbitan esters, glyceryl esters, glycerol monostearate, polyethylene glycols, polypropylene glycols, polypropylene glycol esters, cetyl alcohol, cetostearyl alcohol, stearyl alcohol, aryl alkyl polyether alcohols, polyoxyethylene-polyoxypropylene copolymers, poloxamers, poloxamines, methylcellulose, hydroxycellulose, hydroxymethylcellulose, hydroxypropylcellulose, hydroxypropylmethylcellulose, noncrystalline cellulose, polysaccharides, starch, hydroxyethylstarch, polyvinyl alcohol, polyvinylpyrrolidone, triethanolamine stearate, amine oxides, dextran, glycerol, gum acacia, cholesterol, tragacanth, cetomacrogol emulsifying wax, sorbitan esters, polyoxyethylene alkyl ethers, polyoxyethylene stearates, hydroxypropyl methylcellulose, methylcellulose, hydroxyethylcellulose, hydroxypropylmethylcellulose phthalate, noncrystalline cellulose, polyviryl alcohol, polyvinylpyrrolidone, 4-(1,1,3,3-tetramethylbutyl) phenol polymer with ethylene oxide and formaldehyde, poloxamers, alkyl aryl polyether sulfonates, mixtures of sucrose stearate and sucrose distearate, $C_{18}H_{37}CH_2C(O)N(CH_3)CH_2(CHOH)_4(CH_2OH)_2$, p-isononylphenoxypoly(glycidol), decanoyl-N-methylglucamide, n-decyl-B-D-glucopyranoside, n-decyl-B-D-maltopyranoside, n-dodecyl-B-D-glucopyranoside, n-dodecyl-B-D-maltoside, heptanoyl-N-methylglucamide, n-heptyl-B-D-glucopy-ranoside, n-heptyl-B-D-thioglucoside, n-hexyl-B-D-glucopyranoside; nonanoyl-N-methylglucarnide, n-nonyl-B-D-glucopyranoside, octanoyl-N-methylglucamice, n-octyl-B-D-glucopyranoside, octyl-B-D-thioglucopyranoside, PEG-cholesterol, PEG-vitamin A, PEG-vitamin E, and random copolymers of vinyl acetate and vinyl pyrrolidone.

Zwitterionic-surfactants are electrically neutral but possess local positive and negative charges within the same molecule. The net charge on the molecule may depend on the pH, and therefore at low pH some zwitterionic surfactants may act as cationic surfactants while at high pH they may also act an anionic surfactants. Suitable zwitterionic surfactants include zwitterionic phospholipids. These phospholipids include phosphatidylcholine, phosphatidylethanolamine, dimyristoyl-glycexo-phosphoethanolamine (DMPE), dipalmitoyl-glycero-phosphoethanolamine (DPPE), distearoyl-glycero-phosphoethanolamine (DSPE), and dioleolyl-glycero-phosphoethanolamine (DOPE), pegylated phospholipids, PEG- phosphatidylcholine, PEG-diacyl-glycero-phosphoethanolamine, PEG-phosphatidylethanolamine, PEG-dimyristoyl-glycero-phosphoethanolamine, PEG-dipalmitoyl-glycero-phosphoethanolemine, PEG-distearoyl-glycero-phosphoethanolamine, PEG-dioleolyl-glycero-phosphoethanolamine, methoxy polyethylene glycol (mPEG)-phospholipids, mPEG-phosphatidylcholine, mPEG-phosphatidylethanolamine, nPEG-diacyl-glycero-phosphoethanolamine, mPEG-dimyristoyl-glycero-phosphoethanolamine, mPEG-dipalmitoyl-glycero-phosphoethanolamine, mPEG-distearoyl-glycero-phosphoethanolamine, and mPEG-dioleolyl-glycero-phosphoethanolamine.

Mixtures of phospholipids that include anionic and zwitterionic phospholipids may be employed in this invention. Such mixtures include lysophospholipids, egg or soybean phospholipid or any combination thereof.

Suitable biologically derived surfactants include: lipoproteins, gelatin, casein, lysozyme, albumin, casein, heparin, hirudin, or other proteins.

A preferred ionic surfactant is a bile salt, and a preferred bile salt is sodium deoxycholate. A preferred nonionic surfactant is a polyalkoxyether, and preferred polyalkoxyethers are polyoxyethylene-polyoxypropylene triblock copolymers such as Poloxamer 188 and Poloxamer 407. Another preferred surfactant is a lipid in which a polyalkoxyether is covalently attached to a lipid through an ether linkage. A preferred surfactant of this class is a pegylated phospholipid. Another preferred surfactant is a pegylated phospholipid methyl ether (for example, mPEG-DSPE).

In a preferred embodiment of the present invention, the particles are suspended in an aqueous medium further including a pH adjusting agent. Suitable pH adjusting agents include, sodium hydroxide, hydrochloric acid, tris buffer, mono-, di-, tricarboxylic acids and their salts, citrate buffer, phosphate, glycerol-1-phosphate, glycercol-2-phosphate, acetate, lactate, tris(hydroxymethyl)aminomethane, aminosaccharides, mono-, di- and trialkylated amines, meglumine (N-methylglucosamine), and amino acids. The aqueous medium may additionally include an osmotic pressure adjusting agent, such as glycerin, a monosaccharide such as dextrose, a disaccharide such as sucrose, trehalose and maltose, a trisaccharide such as raffinose, and sugar alcohols such as mannitol and sorbitol.

In an embodiment of the present invention, the aqueous mediun of the particle suspension composition is removed to form dry particles. The method to remove the aqueous medium can be any method known in the art. One example is evaporation. Another example is freeze-drying or lyophilization. The dry particles may then be formulated into any acceptable physical form including solutions, tablets, capsules, suspensions, creams, lotions, emulsions, aerosols, powders, incorporation into reservoir or matrix devices for sustained release (such as implants or transdermal patches), and the like. The aqueous suspension of the present invention may also be frozen to improve stability upon storage. Freezing of an aqueous suspension to improve stability is disclosed in the commonly assigned and co-pending US 2003/0077329 (U.S Patent Application Serial No.10/270,267).

Preferred compositions comprise an aqueous suspension of particles of tubulin inhibitor present at 0.05% to

10% w/v, the particles are coated with 0.05% to 5% w/v of an ionic surfactant (e.g., deoxycholate) or a zwitterionic surfactant (e.g., mPEG-DSPE), and 0.05% to 5% w/v polyalkoxyether (for example, Poloxamer 188), and glycerin added to adjust osmotic pressure of the formulation.

The particle suspensions of the present invention can be prepared by methods known to those skilled in the art and those methods described below.

Methods of particle/suspension preparation

Energy addition methods for preparing particle suspensions of the present invention are disclosed in commonly assigned and co-pending US 2002/0176935; US 2002/0127278; US 2002/0168402; US 2005/0037083; US 2003/0044433; US 2003/0031719; US 2003/0059472; US 2003/0100568; US 2003/0096013; US 2003/0072807; US 2003/77329; and US 2004/022862. (U.S. Patent Application. Serial Nos. 60/258,160; 09/874,799; 09/874,637; 09/874,499; 09/964,273; 10/035,821, 60/347,548; 10/021,692; 10/183,035; 10/213,352; 10/246,802; 10/270,268; 10/270,267, and 10/390,333). A general procedure for preparing the suspension useful in the practice of this invention follows.

The processes can be separated into three general categories. Each of the categories of processes share the steps of: (1) dissolving a tubulin inhibitor compound in a water miscible first organic solvent to create a first solution; (2) mixing the first solution with a second solvent of water to precipitate the tubulin inhibitor to create a pre-suspension; and (3) adding energy to the pre-suspension in the form of high-shear mixing or heat to provide a stable form of the tubulin inhibitor having the desired size ranges defined above.

The three categories of processes are distinguished based upon the physical properties of the tubulin inhibitor as determined through x-ray diffraction studies, differential scanning calorimetry (DSC) studies or other suitable study conducted prior to the energy-addition step and after the energy-addition step.

L First Process Category

The methods of the first process category generally include the step of dissolving the tubulin inhibitor in a water miscible first solvent followed by the step of mixing this solution with an aqueous solution to form a pre-suspension wherein the tubulin inhibitor is in an amorphous form, a semi-crystalline form or in a super-cooled liquid form as determined by x-ray diffraction studies, DSC, light or electron microscopy or other analytical techniques and has an average effective particle size within one of the effective particle size ranges set forth above. The mixing step is followed by an energy-addition step and, in a preferred form of the invention is an annealing step.

II. Second Process Category

The methods of the second process category include essentially the same steps as in the steps of the first process category but differ in the following respect. An x-ray diffraction, DSC or other suitable analysis of the pre-suspension shows the tubulin inhibitor in a crystalline form and having an average effective particle size. The tubulin inhibitor after the energy-addition step has essentially the same average effective particle size as prior to the energy-addition step but has less of a tendency to aggregate into larger particles when compared to that of the particles of the pre-suspension. Without being bound to a theory, it is believed the differences in the particle stability may be due to a reordering of the surfactant molecules at the solid-liquid interface.

III. Third Process Category

The methods of the third category modify the first two steps of those of the first and second processes categories to ensure the tubulin inhibitor in the pre-suspension is in a friable form having an average effective particle size (e.g., such as slender needles and thin plates). Friable particles can be formed by selecting suitable solvents, surfactants or combination of surfactants, the temperature of the individual solutions, the rate of mixing and rate of precipitation and the like. Friability may also be enhanced by the introduction of lattice defects (e.g., cleavage planes) during the steps of mixing the first solution with the aqueous solution. This would arise by rapid crystallization such as that afforded in the precipitation step. In the energy-addition step these friable crystals are converted to crystals that are kinetically stabilized and having an average effective particle size smaller than those of the presuspension. Kinetically stabilized means particles have a reduced tendency to aggregate when compared to particles that are not kinetically stabilized. In such instance the energy-addition step results in a breaking up and coating of the friable particles. By ensuring the particles of the presuspension are in a friable state, the organic compound can more easily and more quickly be prepared into a particle within the desired size ranges when compared to processing an organic compound where the steps have not been taken to render it in a friable form.

The energy-addition step can be carried out in any fashion wherein the pre-suspension is exposed to cavitation, shearing or impact forces. In one preferred form of the invention, the energy-addition step is an annealing step. Annealing is defined in this invention as the process of converting matter that is thermodynamically unstable into a more stable form by single or repeated application of energy (direct heat or mechanical stress), followed by thermal relaxation. This lowering of energy may be achieved by conversion of the solid form from a less ordered to a more ordered lattice structure. Alternatively, this stabilization may occur by a reordering of the surfactant molecules at the solid-liquid interface.

These three process categories will be discussed separately below. It should be understood, however, that the process conditions such as choice of surfactants or combination of surfactants, amount of surfactant used, temperature of reaction, rate of mixing of solutions, rate of precipitation and the like can be selected to allow for any drug to be processed under any one of the categories discussed next

The first process category, as well as the second and third process categories, can be further divided into two subcategories, Method A, and B shown diagrammatically in FIG. 4 and FIG. 5, respectively.

The first solvent according to the present invention is a solvent or mixture of solvents in which the organic compound of interest is relatively soluble and which is miscible with the second solvent. Such solvents include water-miscible protic compounds, in which a hydrogen atom in the molecule is bound to an electronegative atom such as oxygen, nitrogen, or other Group VA, VIA and VII A in the Periodic Table of elements. Examples of such solvents include alcohols, amines (primary or secondary), oximes, hydroxamic acids, carboxylic acids, sulfonic acids, phosphonic acids, phosphoric acids, amides and ureas.

Other examples of the first solvent also include aprotic organic solvents. Some of these aprotic Solvents can form hydrogen bonds with water, but can only act as proton acceptors because they lack effective proton donating groups. One class of aprotic solvents is a dipolar aprotic solvent, as defined by the International Union of Pure and Applied Chemistry (IUPAC Compendium of Chemical Terminology, 2nd Ed., 1997):

> *A solvent with a comparatively high relative permittivity (or dielectric constant), greater than ca. 15, and a sizable permanent dipole moment, that cannot donate suitably labile hydrogen atoms to form strong hydrogen bonds, e.g. dimethyl sulfoxide.*

Dipolar aprotic solvents can be selected from the group consisting of amides (fully substituted, with nitrogen lacking attached hydrogen atoms), ureas (fully substituted, with no hydrogen atoms attached to nitrogen), ethers, cyclic ethers, nitriles, ketones, sulfones, sulfoxides, fully substituted phosphates, phosphonate esters, phosphoramides, nitro compounds, and the like. Dimethylsulfoxide (DMSO), N-methyl-2-pyrrolidinone (NMP), 2-pyrrolidinone, 1,3-dimethyl-2-imidazolidinone (DMI), dimethylacetamide (DMA), dimethylformamide (DMF), dioxane, acetone, tetrahydrofuran (THF), tetramethylenesulfone (sulfolane), acetonitrile, and hexamethylphosphoramide (HMPA), nitromethane, 1,2-propylene glycol carbonate, among others, are members of this class.

Solvents may also be chosen that are generally water-immiscible, but have sufficient water solubility at low volumes (less than 10%) to act as a water-miscible first solvent at these reduced volumes. Examples include aromatic hydrocarbons, alkenes, alkanes, and halogenated aromatics, halogenated alkenes and halogenated alkanes. Aromatics include benzene (substituted or unsubstituted), and monocyclic or polycyclic arenes. Examples of substituted benzenes include xylenes (ortho, meta, or para), and toluene. Examples of alkanes include hexane, neopentane, heptane, isooctane, and cyclohexane. Examples of halogenated aromatics include chlorobenzene, bromobenzene, and chlorotoluene. Examples of halogenated alkanes and alkenes include trichloroethane, methylene chloride, ethylenedichloride (EDC), and the like.

Examples of the all of the above solvent classes include: N-methyl-2-pyrrolidinone (N-methyl-2-pyrrolidone), 2-pyrrolidinone (2-pyrrolidone), 1,3-dimethyl-2-imidazolidinone (DMI), dimethylsulfoxide, dimethylacetamide, carboxylic acids (such as acetic acid and lactic acid), aliphatic alcohols (such as ethanol, ethanol, isopropanol, 3-pentanol, and n-propanol), benzyl alcohol, glycerol, butylene glycol (1,2-butanediol, 1,3-butanediol, 1,4-butanediol, and 2,3-butanediol), ethylene glycol, propylene glycol, mono- and diacylated glycerides, dimethyl isosorbide, acetone, dimethylsulfone, dimethylformamide, 1,4-dioxane, tetramethylenesulfone (sulfolane), acetonitrile, nitromethane, tetramethylurea, hexamethylphosphoramide (HMPA), tetrahydrofuran (THF), diethylether, tert-butylmethyl ether (TBME), aromatic hydrocarbons, alkenes, alkanes, halogenated aromatics, halogenated alkenes, halogenated alkanes, xylene, toluene, benzene, substituted benzene, ethyl acetate, methyl acetate, butyl acetate, chlorobenzene, bromobenzene, chlorotoluene, trichloroethane, methylene chloride, ethylenedichloride (EDC), hexane, neopentane, heptane, isooctane, cyclohexane, polyethylene glycol (PEG), PEG esters, PEG-4, PEG-8, PEG-9, PEG-12, PEG-14, PEG-16, PEG-120, PEG-75, PEG-150, polyethylene glycol esters, PEG-4 dilaurate, PEG-20 dilaurate, PEG-6 isostearate, PEG-8 palmitostearate, PEG-150 palmitostearate, polyethylene glycol sorbitans, PEG-20 sorbitan isostearate, polyethylene glycol monoalkyl ethers, PEG-3 dimethyl ether, PEG-4 dimethyl ether, polypropylene glycol (PPG), polypropylene alginate, PPG-10 butanediol, PPG-10 methyl glucose ether, PPG-20 methyl glucose ether, PPG-15 stearyl ether, propylene glycol dicaprylate/dicaprate, propylene glycol laurate, and glycofurol (tetrahydrofurfuryl alcohol polyethylene glycol ether).

A preferred first solvent is N-methyl-2-pyrrolidinone (NMP). Another preferred first solvent is lactic acid.

The second solvent is an aqueous solvent This aqueous solvent may be water by itself. This solvent may also contain buffers, salts, surfactant(s), water-soluble polymers, and combinations of these excipients.

Method A

In Method A, the tubulin inhibitor is first dissolved in the first solvent to create a first solution. The tubulin inhibitor can be added from about 0.01% to about 20% weight to volume (w/v) depending on the solubility of the tubulin inhibitor in the first solvent. Heating of the concentrate from about 30°C to about 100°C may be necessary to ensure total dissolution of the tubulin inhibitor in the first solvent.

A second aqueous solution is provided with one or more surfactants added thereto. The surfactants can be selected from an ionic surfactant, a nonionic surfactant, a cationic surfactant, a zwitterionic surfactant, a phospholipid, or a biologically derived surfactant set forth above.

It may also be desirable to add a pH adjusting agent to the second solution such as sodium hydroxide, hydrochloric acid, amino acid such as glycine, tris buffer or citrate, acetate, lactate, meglumine, or the like. The second solution should have a pH within the range of from about 2 to about 12.

The first and second solution are then combined. Preferably, the first solution is added to the second solution in a controlled rate. The addition rate is dependent on the batch size, and precipitation kinetics for the tubulin inhibitor. Typically, for a small-scale laboratory process (preparation of 1 liter), the addition rate is from about 0.05 cc per minute to about 50 cc per minute. During the addition, the solutions should be under constant agitation. It has been observed using light microscopy that amorphous particles, semi-crystalline solids, or a super-cooled liquid are formed to create a pre-suspension. The method further includes the step of subjecting the pre-suspension to an annealing step to convert the amorphous particles, super-cooled liquid or semi-crystalline solid to a crystalline more stable solid state. The resulting particles will have an average effective particles size as measured by dynamic light scattering methods (e.g., photocorrelation spectroscopy, laser diffraction, low-angle laser light scattering (LALLS), medium-angle laser light scattering (MALLS)), light obscuration methods (Coulter method, for example), rheology, or microscopy (light or electron) within the ranges set forth above.

The energy-addition step involves adding energy through sonication, homogenization, counter current flow homogenization (e.g., the Mini DeBEE 2000 homogenizer, available from BEE Incorporated, NC, in which a jet of fluid is directed along a first path, and a structure is interposed in the first path to cause the fluid to be redirected in a controlled flow path along a new path to cause emulsification or mixing of the fluid), microfluidization, or other methods of providing impact, shear or cavitation forces. The sample may be cooled or heated during this stage. In one preferred form of the invention the annealing step is effected by homogenization. In another preferred form of the invention the annealing may be accomplished by ultrasonication. In yet another preferred form of the invention the annealing may be accomplished by use of an emulsification apparatus as described in U.S. Patent No. 5,720,551.

Depending upon the rate of annealing, it may be desirable to adjust the temperature of the processed sample to within the range of from approximately 0°C to 30°C. Alternatively, in order to effect a desired phase change in the processed solid, it may also be necessary to adjust the temperature of the pre-suspension to a temperature within the range of from about - 30°C to about 100°C during the annealing step.

Method B

Method B differs from Method A in the following respects. The first difference is a surfactant or combination of surfactants are added to the first solution. The surfactants may be selected from ionic surfactants, nonionic surfactants, cationic surfactants, zwitterionic surfactants, phospholipids, or biologically derived as set forth above. A drug suspension resulting from application of the processes described in this invention may be administered directly as an injectable solution, provided that an appropriate means for solution sterilization is applied.

Sterilization

Sterilization may be accomplished by separate sterilization of the drug concentrate (drug, solvent, and optional surfactant) and the diluent medium (water, and optional buffers and surfactants) prior to mixing to form the pre-suspension. Sterilization methods include pre-filtration first through a 3.0 micron filter followed by filtration through a 0.45-micron particle filter, followed by steam or heat sterilization or sterile filtration through two redundant 0.2-micron membrane filters.

Preparation of Solvent-free Suspension

Optionally, a solvent-free suspension may be produced by solvent removal after precipitation. This can be

accomplished by centrifugation, dialysis, diafiltration, force-field fractionation, high-pressure filtration or other separation techniques well known in the art. Complete removal of lactic acid or N-methyl-2-pyrrolidinone was typically carried out by one to three successive centrifugation runs; after each centrifugation the supernatant was decanted and discarded- A fresh volume of the suspension vehicle without the organic solvent was added to the remaining solids and the mixture was dispersed by homogenization. It will be recognized by others skilled in the art that other high-shear mixing techniques could be applied in this reconstitution step.

Replacement of Excipients

Furthermore, any undesired excipients such as surfactants may be replaced by a more desirable excipient by use of the separation methods described in the above paragraph. The solvent and first excipient may be discarded with the supernatant after centrifugation or filtration. A fresh volume of the suspension vehicle without the solvent and without the first excipient may then be added. Alternatively, a new surfactant may be added. For example, a suspension consisting of drug, N-methyl-2-pyrrolidinone (solvent), Poloxamer 188 (first excipient), sodium deoxycholate, glycerol and water may be replaced with phospholipids (new surfactant), glycerol and water after centrifugation and removal of the supernatant.

Lyophilization

The suspension may be dried by lyophilization (freeze-drying) to form a lyophilized suspension for reconstitution into a suspension suitable for administration. For the purpose of preparing a stablized, dry solid, bulking agents such as mannitol, sorbitol, sucrose, starch, lactose, trehalose or raffinose may be added prior to lyophilization. The suspension may be lyophilized using any applicable program for lyophilization, for example:

loading at +25°C
cooling down to -45 °C in 1 hour
holding time at -45°C for 3.5 hours
mean drying for 33 hours with continual increase of temperature to +15°C at a pressure of 0.4 mbar
final drying for 10 hours at +20 °C at a pressure of 0.03 mbar cryo protectant: mannitol

In addition to the microprecipitation methods described above, any other known precipitation methods for preparing particles of active agent (and more preferably, nanoparticles) in the art can be used in conjunction with the present invention. The following is a description of examples of other precipitation methods. The examples are for illustration purposes, and are not intended to limit the scope of the present invention.

Emulsion Precipitation Methods

One suitable emulsion precipitation technique is disclosed in the co-pending and commonly assigned U.S. Application No. 2005/0037083. In this approach, the process includes the steps of: (1) providing a multiphase system having an organic phase and an aqueous phase, the organic phase having a pharmaceutically effective compound therein; and (2) sonicating the system to evaporate a portion of the organic phase to cause precipitation of the compound in the aqueous phase and having an average effective particle size of less than about 2 μm. The step of providing a multiphase system includes the steps of: (1) mixing a water immiscible solvent with the pharmaceutically effective compound to define an organic solution, (2) preparing an aqueous based solution with one or more surface active compounds, and (3) mixing the organic solution with the aqueous solution to form the multiphase system. The step of mixing the organic phase and the aqueous phase can include the use of piston gap homogenizers, colloidal mills, high speed stirring equipment, extrusion equipment, manual agitation or shaking equipment, microfluidizer, or other equipment or techniques for providing high shear conditions. The crude emulsion will have oil droplets in the water of a size of approximately less than 1 μm in diameter. The crude emulsion is sonicated to define a microemulsion and eventually to define a submicron sized particle suspension.

Another approach to preparing submicron-sized particles is disclosed in co-pending and commonly assigned U.S. 2003/0059472, incorporated herein by reference and made a part hereof. The process includes the steps of: (1) providing a crude dispersion of a multiphase system having an organic phase and an aqueous phase, the organic phase having a pharmaceutical compound therein; (2) providing energy to the crude dispersion to form a fine dispersion; (3) freezing the fine dispersion; and (4) lyophilizing the fine dispersion to obtain submicron sized particles of the pharmaceutical compound. The step of providing a multiphase system includes the steps of: (1) mixing a water immiscible solvent with the pharmaceutically effective compound to define an organic solution; (2) preparing an aqueous based solution with one or more surface active compounds; and (3) mixing the organic solution with the aqueous solution to

form the multiphase system. The step of mixing the organic phase and the aqueous phase includes the use of piston gap homogenizers, colloidal mills, high speed stirring equipment, extrusion equipment, manual agitation or shaking equipment, microfluidizer, or other equipment or techniques for providing high shear conditions.

Solvent Anti-Solvent Precipitation

A suitable solvent anti-solvent precipitation technique is disclosed in U.S. Pat. Nos. 5,118,528 and 5,100,591. The process includes the steps of: (1) preparing a liquid phase of a biologically active substance in a solvent or a mixture of solvents to which may be added one or more surfactants; (2) preparing a second liquid phase of a non-solvent or a mixture of non-solvents, the non-solvent is miscible with the solvent or mixture of solvents for the substance; (3) adding together the solutions of (1) and (2) with stirring; and (4) removing of unwanted solvents to produce a colloidal suspension of nanoparticles. The '528 Patent discloses that it produces particles of the substance smaller than 500 nm without the supply of energy.

Phase Inversion Precipitation

One suitable phase inversion precipitation is disclosed in U.S. Pat. Nos. 6,235,224, 6, 143,211 and U.S. patent application No. 2001/0042932. Phase inversion is a term used to describe the physical phenomena by which a polymer dissolved in a continuous phase solvent system inverts into a solid macromolecular network in which the polymer is the continuous phase. One method to induce phase inversion is by the addition of a nonsolvent to the continuous phase. The polymer undergoes a transition from a single phase to an unstable two phase mixture: polymer rich and polymer poor fractions. Micellar droplets of nonsolvent in the polymer rich phase serve as nucleation sites and become coated with polymer. The '224 patent discloses that phase inversion of polymer solutions under certain conditions can bring about spontaneous formation of discrete microparticles, including nanoparticles. The'224 patent discloses dissolving or dispersing a polymer in a solvent. A pharmaceutical agent is also dissolved or dispersed in the solvent. For the crystal seeding step to be effective in this process it is desirable the agent is dissolved in the solvent. The polymer, the agent and the solvent together form a mixture having a continuous phase, wherein the solvent is the continuous phase. The mixture is then introduced into at least tenfold excess of a miscible nonsolvent to cause the spontaneous formation of the microencapsulated microparticles of the agent having an average particle size of between 10 nm and 10 μm. The particle size is influenced by the solvent:nonsolvent volume ratio, polymer concentration, the viscosity of the polymer-solvent solution, the molecular weight of the polymer, and the characteristics of the solvent-nonsolvent pair. The process eliminates the step of creating microdroplets, such as by forming an emulsion, of the solvent. The process also avoids the agitation and/or shear forces.

pH Shift Precipitation

pH shift precipitation techniques typically include a step of dissolving a drug in a solution having a pH where the drug is soluble, followed by the step of changing the pH to a point where the drug is no longer soluble. The pH can be acidic or basic, depending on the particular pharmaceutical compound. The solution is then neutralized to form a presuspension of submicron sized particles of the pharmaceutically active compound. One suitable pH shifting precipitation process is disclosed in U.S. Pat. No. 5,665,331. The process includes the step of dissolving of the pharmaceutical agent together with a crystal growth modifier (CGM) in an alkaline solution and then neutralizing the solution with an acid in the presence of suitable surface-modifying surface-active agent or agents to form a fine particle dispersion of the pharmaceutical agent. The precipitation step can be followed by steps of diafiltration clean-up of the dispersion and then adjusting the concentration of the dispersion to a desired level. This process of reportedly leads to microcrystalline particles of Z-average diameters smaller than 400 nm as measured by photon correlation spectroscopy.

Other examples of pH shifting precipitation methods are disclosed in U.S. Pat. Nos. 5,716,642; 5,662,883; 5,560,932; and 4,608,278.

Infusion Precipitation Method

Suitable infusion precipitation techniques are disclosed in the U.S. Pat. Nos. 4,997,454 and 4,826,689. First, a suitable solid compound is dissolved in a suitable organic solvent to form a solvent mixture. Then, a precipitating nonsolvent miscible with the organic solvent is infused into the solvent mixture at a temperature between about -10°C and about 100°C and at an infusion rate of from about 0.01 ml per minute to about 1000 ml per minute per volume of 50 ml to produce a suspension of precipitated non-aggregated solid particles of the compound with a substantially uniform mean diameter of less than 10 μm. Agitation (e.g., by stirring) of the solution being infused with the precipitating nonsolvent is preferred. The nonsolvent may contain a surfactant to stabilize the particles against aggregation. The

particles are then separated from the solvent. Depending on the solid compound and the desired particle size, the parameters of temperature, ratio of nonsolvent to solvent, infusion rate, stir rate, and volume can be varied according to the invention. The particle size is proportional to the ratio of nonsolvent: solvent volumes and the temperature of infusion and is inversely proportional to the infusion rate and the stirring rate. The precipitating nonsolvent may be aqueous or non-aqueous, depending upon the relative solubility of the compound and the desired suspending vehicle.

Temperature Shift Precipitation

Temperature shift precipitation technique, also known as the hot-melt technique, is disclosed in U.S. Pat No. 5,188,837 to Domb. In an embodiment of the invention, lipospheres are prepared by the steps of: (1) melting or dissolving a substance such as a drug to be delivered in a molten vehicle to form a liquid of the substance to be delivered; (2) adding a phospholipid along with an aqueous medium to the melted substance or vehicle at a temperature higher than the melting temperature of the substance or vehicle; (3) mixing the suspension at a temperature above the melting temperature of the vehicle until a homogenous fine preparation is obtained; and then (4) rapidly cooling the preparation to room temperature or below.

Solvent Evaporation Precipitation

Solvent evaporation precipitation techniques are disclosed in U.S. Pat No. 4,973,465. The ’465 Patent discloses methods for preparing microcrystals including the steps of: (1) providing a solution of a pharmaceutical composition and a phospholipid dissolved in a common organic solvent or combination of solvents, (2) evaporating the solvent or solvents and (3) suspending the film obtained by evaporation of the solvent or solvents in an aqueous solution by vigorous stirring. The solvent can be removed by adding energy to the solution to evaporate a sufficient quantity of the solvent to cause precipitation of the compound. The solvent can also be removed by other well known techniques such as applying a vacuum to the solution or blowing nitrogen over the solution.

Reaction Precipitation

Reaction precipitation includes the steps of dissolving the pharmaceutical compound into a suitable solvent to form a solution. The compound should be added in an amount at or below the saturation point of the compound in the solvent. The compound is modified by reacting with a chemical agent or by modification in response to adding energy such as heat or UV light or the like to such that the modified compound has a lower solubility in the solvent and precipitates from the solution.

Compressed Fluid Precipitation

A suitable technique for precipitating by compressed fluid is disclosed in United States Patent No. 6,576,264. The method includes the steps of dissolving a water-insoluble drug in a solvent to form a solution. The solution is then sprayed into a compressed fluid, which can be a gas, liquid or supercritical fluid. The addition of the compressed fluid to a solution of a solute in a solvent causes the solute to attain or approach supersaturated state and to precipitate out as fine particles. In this case, the compressed fluid acts as an anti-solvent which lowers the cohesive energy density of the solvent in which the drug is dissolved.

Alternatively, the drug can be dissolved in the compressed fluid which is then sprayed into an aqueous phase. The rapid expansion of the compressed fluid reduces the solvent power of the fluid, which in turn causes the solute to precipitate out as fine particles in the aqueous phase. In this case, the compressed fluid acts as a solvent

Other Methods for Preparing Particles

The particles of the present invention can also be prepared by mechanical grinding of the active agent Mechanical grinding include such techniques as jet milling, pearl milling, ball milling, hammer milling, fluid energy milling or wet grinding techniques such as those disclosed in U.S. Pat. No. 5,145,684.

Another method to prepare the particles of the present invention is by suspending an active agent. In this method, particles of the active agent are dispersed in an aqueous medium by adding the particles directly into the aqueous medium to derive a pre-suspension. The particles are normally coated with a surface modifier to inhibit the aggregation of the particles. One or more other excipients can be added either to the active agent or to the aqueous medium.

Example 1: Small-scale preparation (300 g) of a suspension of the D-24851 (Composition 1)

An aqueous surfactant solution containing 0.1% sodium deoxycholate, 2.2% glycerin (tonicity agent), and 0.142% sodium phosphate dibasic (buffer) was cooled to low temperature (< 10°C). A solution of D-24851 and Poloxamer 188 in lactic acid was added to the above surfactant solution A suspension formed upon mixing of the two solutions. The total suspension weight was 300 g, with a drug concentration of approximately 1% (w/w). High-pressure homogenization was carried out immediately after precipitation, at a pressure of approximately 10,000 psi and a temperature of <70°C. The lactic acid was removed by centrifugation and the suspension was homogenized again at approximately 10,000 psi and a temperature of <70°C. After homogenization, the particle size of the suspension was examined using light scattering. The mean particle size was approximately 190 nm.

Example 2: Preparation of 2,000 g of a suspension of D-24851 (Composition 2)

An aqueous surfactant solution containing 0.1% sodium deoxycholate, 2.2% glycerin (tonicity agent), and 0.142% sodium phosphate dibasic (buffer) was cooled to low temperature (< 10°C). A solution of D-24851 and poloxamer 188 in lactic acid was added to the above surfactant solution. A suspension formed upon mixing of the two solutions. The total suspension weight was 2,000 g, with a drug concentration of approximately 1% (w/w). High-pressure homogenization was carried out immediately after precipitation, at a pressure of approximately 10,000 psi and a temperature of <70°C. The lactic acid was removed by centrifugation and the suspension was homogenized again at approximately 10,000 psi and a temperature of <70°C. After homogenization, the particle size of the suspension was examined using light scattering. The mean particle size was approximately 325 nm.

Example 3: Large-scale preparation (6,000 g) of a suspension of D-24851 (Composition 3)

An aqueous surfactant solution containing 0.1% sodium deoxycholate, 2.2% glycerin (tonicity agent), and 0.142% sodium phosphate dibasic (buffer) was cooled to low temperature (< 10°C). A solution of D-24851 and poloxamer 188 in lactic acid was added to the above surfactant solution. A suspension formed upon mixing of the two solutions. The total suspension weight was 6,000 g, with a drug concentration of approximately 1% (w/w). High-pressure homogenization was carried out immediately after precipitation, at a pressure of approximately 10,000 psi and a temperature of <70°C. The lactic acid was removed by centrifugation and the suspension was homogenized again at approximately 10,000 psi and a temperature of <70°C. After homogenization, the particle size of the suspension was examined using light scattering. The mean particle size was approximately 370 nm.

Example 4: Stability of a nanosuspension of the present invention

Stability of the suspensions was tested using accelerated stress (thermal cycling, agitation, freeze-thaw, and centrifugation) as well as storage at 5°C for up to 6 months. There were no significant changes in the particle size mean, $99^{th}$ percentile and $100^{th}$ percentile values (for Composition 3). Furthermore, no aggregation was observed in any of the stress tests. Aggregation was estimated by measuring particle size before and after sonication for one minute, and computing the percent aggregation by use of the following equation:

$$\%\ \text{Aggregation} = \frac{(P_{99} - P_{99S})\ X 100}{P_{99S}}$$

where $P_{99}$ represents the $99^{th}$ percentile of the particle size distribution before sonication, and $P_{99s}$ represents the $99^{th}$ percentile of the particle size distribution after sonication.

Example 5: D-24851 (Composition 4)

*A preferred composition of the present invention:*

| Ingredient | Concentration |
|---|---|
| D-24851 | 10 mg/g |
| Poloxamer 188 | 1 mg/g |
| Deoxycholic acid, sodium salt | 1 mg/g |

(continued)

| Ingredient | Concentration |
|---|---|
| Glycerin | 22 mg/g |
| Sodium phosphate, dibasic | 1.42 mg/g |
| NaOH sol., HCl sol. | for pH adjustment |
| Water for injection | adjust to total weight of 100 g |
| PH | 8.5 |

Example 6: Solutol/Propanediol Formulation (Composition 5)

The following composition was prepared for comparison with compositions of the present invention. Composition per 500 g solution:

| | |
|---|---|
| D-24851) | 1.0 g (0.2%, w/w) |
| Solutol HS15 | 375.0 g |
| 1,2 Propanediol | 125.0 g |

Example 7: Lactic Acid Formulation (Composition 6)

The following composition was prepared for comparison with compositions of the present invention. The lactic acid formulation is an oversaturated solution of D-24851 for oral administration. Because of the oversaturated drug concentration and physical instability, it is important that the solution must be freshly prepared prior to administration. The drug is provided as a preparation set. These sets comprise 3 vials or a 3 compartment device as follows:

Content of the Drug Vial (Vial 1)

| | |
|---|---|
| 1 Vial/Compartment (100 mL container) contains: | |
| Indibulin (D-24851) | 60.0 mg |

Content of Solvent Vial A (Vial 2)

| | |
|---|---|
| 1 Vial/Compartment (10 mL container) contains: | |
| Lactic acid 90 % | 9041.3 mg |

Content of Solvent Vial B (Vial 3)

| | |
|---|---|
| 1 Vial/Compartment (75 mL container) contains: | |
| Glucose | 5705.5 mg |
| Passion fruit flavor | 10.0 mg |
| Water pur. | 51347.0 mg |

Composition of D-24851-lactic acid drinking solution after preparation
1 Vial/Container contains:

| Ingredient | Amount |
|---|---|
| D-24851 | 60.0 mg |
| Lactic acid | 7269.2 mg |
| Glucose | 5601.8 mg |
| Passion fruit flavor | 9.8 mg |
| Water pur. | 50413.4 mg |

Example 8: Preferred Compositions

| Ingredient | Concentration |
|---|---|
| Compound of Formula 1 | Range<br>0.1% - 10% w/w |
| 1st Preferred Surfactant (or class)<br>Non-ionic surfactant, e.g. poloxamer | Range<br>0.01% - 5% w/w |
| 2nd Preferred Surfactant (or class)<br>Anionic or zwiterionic surfactant, e.g. bile acid salt, phospholipids, or mixture | Range<br>0.01% - 5% w/w |
| Excipient 1<br>Buffer agent, e.g. sodium phosphate | Range<br>1 - 50 mM |
| Excipient 2<br>Tonicity agent, e.g. glycerin or trehalose | Range<br>1% - 5% w/w |

Example 9: Preferred Compositions

**Table 1 - Batches of D-24851 Suspension Formulations Compounded by Direct Homogenization**

| Batch No. | Surfactant 1 | Surfactant 2 | Tonicity Agent | Buffer |
|---|---|---|---|---|
| 1 | Phospholipids E80, 1.2% | - | Trehalose, 4% | $Na_2HPO_4$, 0.142% |
| 2 | Phospholipids E80, 1.2% | - | Glycerin, 2.2% | $Na_2HPO_4$, 0.142% |
| 3 | Phospholipids E80, 1.2% | DMPG, 0.1% | Trehalose, 4% | $Na_2HPO_4$, 0.142% |
| 4 | DMPC, 1.2% | DMPG, 0.1% | Trehalose, 4% | $Na_2HPO_4$, 0.142% |
| 5 | Phospholipon 100H, 1.2% | DMPG, 0.1% | Trehalose, 4% | $Na_2HPO_4$, 0.142% |
| 6 | Phospholipids E80, 1.2% | Na Deoxycholate, 0.1% | Glycerin, 2.2% | $Na_2HPO_4$, 0.142% |
| 7 | Phospholipids E80, 0.6% | Na Deoxycholate, 0.05% | Glycerin, 2.2% | $Na_2HPO_4$, 0.142% |
| 8 | Phospholipids E80, 2.4% | - | Glycerin, 2.2% | $Na_2HPO_4$, 0.142% |
| 9 | Phospholipids E80, 2.4% | Na Deoxycholate, 0.1 % | Glycerin, 2.2% | $Na_2HPO_4$, 0.142% |

**Table 2 - Batches of D-24851 Suspension Formulations Compounded by Microprecipitation/Direct Homogenization**

| Batch No. | Surfactant 1 | Surfactant 2 | Tonicity Agent | Buffer |
|---|---|---|---|---|
| 10 | Phospholipids E80, 1.2% | - | Glycerin, 2.2% | $Na_2HPO_4$, 0.142% |
| 11 | Phospholipids E80, 1.2% | Na Deoxycholate, 0.1% | Glycerin, 2.2% | $Na_2HPO_4$, 0.142% |
| 12 | Poloxamer 188 (0.1 %) | Na Deoxycholate, 0.1% | Glycerin, 2.2% | $Na_2HPO_4$, 0.142% |
| 13 | Solutol HS-15 (1.5%) | - | Glycerin, 2.2% | $Na_2HPO_4$, 0.142% |
| 14 | E80, 1.2% | Hetastarch, 1% | Glycerin, 2.2% | TRIS, 0.06% |

Example 10:

Comparison Study of the Bioavailability and Pharmacokinetics of Compositions 4, 5 and 6

The study was performed in 6 cynomolgus monkeys (3 males and 3 females) in a crossover design. The test drug compositions were administered both orally and intravenously.

The following dosing regimen was followed:

A: Composition 6, p.o., 5 mg/kg/dose
B: Composition 4, p.o., 5 mg/kg/dose
C: Composition 4, i.v., 5 mg/kg/dose
D: Composition 5, i.v, 0.2 mg/kg/dose

Blood samples from all animals were taken at the following times:

Oral: before as well as 0.5, 1, 2, 4, 6, 8, 10, 12, 16, 20, 24, 30, 36, 42, 48 and 54 h after administration. Additional blood samples were taken 60 h post dose (Composition 4).
Intravenous: before as well as 0.033, 0.083, 0.17, 0.25, 0.5, 0.75, 1, 2, 3, 4, 5 and 6 h after administration. Additional blood samples were taken 10, 16, 24, 36, 48 and 60 h post dose (Composition 4).
Sample Collection: Blood samples were collected in tubes containing Li-heparin and were centrifuged to obtain plasma. For the intravenous Composition 4 dosed animals, samples were divided in two similar aliquots. One sample was centrifuged to produce plasma and the other sample of whole blood was stored together with the test plasma samples at approx. -20°. The plasma and the blood concentrations of Indibulin were determined by a validated HPLC method. The limit of quantification (LOQ) is 2 ng/ml. The obtained volume of the test samples was about 100-300 μl. The obtained plasma and blood concentrations were used for non-compartmental pharmacokinetic evaluations.

The median plasma and blood concentration-time profiles of **D-24851 after** oral and intravenous administration are given in Tables 1 and 2:

**Table 3**

**Table 3 Pharmacokinetic parameters of D-24851 after intravenous or oral administration (plasma concentrations)**

**Pharmacokinetic parameters of D-24851 after intravenous or oral administration *Plasma concentrations***

| | | Mean$_{geo}$ (95% CI$_{in}$) | | | | | | | Median (Min - Max) | |
|---|---|---|---|---|---|---|---|---|---|---|
| **Compositiontion** | **Route** | **$C_{max}$** [ng/ml] | **$AUC_{o\text{-}tlast}$** [ng·h/ml] | **AUC** [ng·h/ml] | **CL** [ml/mm/kg] | **$V_{ss}$** [l/kg] | **$V_z$** [l/kg] | **MRT** [h] | **$t_{max}$** [h] | **$t_{1/2}$** [h] |
| **solu/prop.[1)]0.2 mg/kg** | i.v. | **401** (279.576) | **287** (228-360) | **319** (249-409) | **10.5** (8.16-13.4) | **1.14** (0.82-1.59) | **1.73** (1.06-2.80) | **1.82** (1.18-2.80) | **0.06** (0.03-0.08) | **1.85** (1.01-3.47) |
| **Composition 4 -D-24851 nanosuspension[2)] 5 mg/kg** | i.v. | **586** (349-985) | **5501** (3947-7666) | **6374** (4357-9325) | - | - | **27.4*** (15.5-48.2) | **25.8** (17.8-37.6) | **0.06** (0.03-0.08) | **26.7** (23.7-50.0) |
| **lactic acid[3)] 5 mg/kg** | p.o. | **59.1** (22.2-157) | **676** (356-1284) | **803** (405-1592) | - | - | **10.3** (4.05-26.3) | **15.2** (8.73-26.6) | **4.00** (2.00-16.0) | **12.8** (6.18-15.3) |
| **Composition 4 - D-24851 nanosuspension[2)] 5 mg/kg** | p.o. | **27.8** (15.3-50.4) | **182** (119-281) | - | - | - | - | - | 6.00 (4.00-6.00) | - |

[1)] n=6, [2)] n=5, [3)] n=4

* The plasma concentrations showed an untypical curve progression with an absorption phase. Therefore the apparent volume f distribution was calculated by the use of the fraction of the administered dose which was systemically available.

**Table 4**

**Table 4 Pharmacokinetic parameters of D-24851 after intravenous or oral administration (Blood concentrations)**

**Pharmacokinetic parameters of D-24851 after intravenous or oral administration *Blood concentrations***

| | | **Mean$_{geo}$ (95% $CI_{in}$)** | | | | | | | **Median (Min - Max)** | |
|---|---|---|---|---|---|---|---|---|---|---|
| **Formul.** | **Route** | **$C_{max}$** [ng/ml] | **$AUC_{o\text{-}tlast}$** [ng·h/ml] | **AUC** (ng·h/ml] | **CL** [ml/mm/kg] | **$V_{ss}$** [l/kg] | **$V_z$** [l/kg] | **MRT** [h] | **$t_{max}$** [h] | **$t_{1/2}$** [h] |
| **Composition 4 - D-24851 nanosuspension[2)]5 mg/kg** | i.v. | **47516** (35571-63472) | **13375** (9233-19374) | **14023** (9736-20198) | **5.94** (4.13-8.56) | **2.60** (1.02-6.65) | **11.6** (5.93-22.7) | **7.30** (3.27-16.3) | **0.03** (0.03-0.03) | **20.0** (11.2-41.8) |
| **Composition 4 D-24851 nanosuspension[1)]) 5 mg/kg** | p.o. | **17.2** (12.0-24.6) | **131.5** (81.5-212) | - | - | - | - | - | **6.00** (4.00-12.0) | - |

[1)] n = 5; [2)] n = 6

Under the regimen described in Example 10, the nanosuspension formulation of D-24851, preferably Composition 4, is characterized by a sustained-release pharmacokinetic after I.V. injection. As shown in Tables 1 and 2 and as illustrated in FIG. 1, intravenous injection of Composition 4 does not lead to a typical i.v. plasma curve as compared to Composition 5. Instead of a high $c_{max}$ value and a rapid exponential decrease of the plasma concentration of D-24851, a sustained released profile was found. As the effective concentration for D-24851 is expected to be above 100 mg/ml, the nanosuspension (Composition 4) will lead to an efficacy over more than 15 hours, whereas the solutol solution (Composition 5) will only be effective for less than 2 hours.

Calculation of the absolute bioavailability for the different compositions is based on their plasma AUC values relative to that for intravenous administration of the Composition 5 Solutol/Propanediol solution at a dose of 0.2 mg/kg under the assumption of dose linearity in the range of 0.2 - 5 mg/kg.

The absolute bioavailability of Composition 4 after a single oral administration of 5 mg/kg as a 10% aqueous lactic acid solution was calculated to be 11.5%.

Because of its high lactic acid content, the lactic acid solution (Composition 6) is very bitter, causes emesis and is poorly tolerated. The nanosuspension (Composition 4), on the other hand, offers an attractive alternative because all lactic acid is removed, and thus the nanosuspension is much better tolerated.

Due to the shown pharmacokinetic properties and therefore increased plasma half-life of D-24851 after i.v. injection of Composition 4, better tolerability is achieved after injection because of lower $C_{max}$ values. The overall tolerability of Composition 4 is also improved because the total dosage amount of D-24851 administered to a mammal can be reduced over the entire therapeutic cycle. Also, a prolonged dosing interval is achieved because Composition 4 shows more than seven times longer effective plasma levels than Composition 5; the frequency of administration to a mammal can be reduced over the entire therapeutic cycle and still achieve equivalent efficacy in terms of tumor inhibition, but with significantly fewer side effects, compared to solutions administered more frequently.

<u>Example 11: Comparison of the toxicity profiles of Composition 4</u>

To evaluate the subchronic toxicity of Composition 4, dogs (3 male and 3 female) were treated over a time frame of 4 weeks. Composition 4 was injected intravenously at different dose levels of 2.61 mg/kg, 5.62 mg/kg and 12.1 mg/kg.

Blood samples from all animals were taken at the following times: 1h, 2 h, 4 h, 8 h, 16 h, 24 h, 36 h and 48 hours after application. The concentration levels of D-24851 were measured using HPLC.

As shown in Tables 3 and 4, D-24851 plasma concentrations depend from the dose. Plasma profiles were of similar magnitude at day 1 and day 27 dosings.

Table 5

**Table 5 Pharmacokinetic parameters of D-14851 (Day 1)**

**Pharmacokinetic parameters of D-24851**
$Mean_{ar}$ (n=3 for each sex)
(min-max)
**Day 1**

| **Dose** [mg/kg] | **Sex** | **$C_{max, sd}$** [ng/ml] | **$t_{max, sd}$** [h] | **$AUC_{sd}$** [ng·h/ml] | **$AUC_{\tau, sd}$** [ng-h/ml] | **$t_{1/2}$** [h] | **CL/f** [ml/(min·kg)] |
|---|---|---|---|---|---|---|---|
| 2.61 | Males | **147** (130-166) | **1.67** (1.00-200) | nc | nc | nc | nc |
| | Females | **210** (183-258) | **1.67** (1.00-2.00) | nc | **3403** (2945-3705) | **41.0*** (19.7-81.7) | nc |
| 5.62 | Males | **241** (190-267) | **2.00** (2.00-2.00) | **2468** (2347-2654) | **2593** (2488-2784) | **6.63** (6.04-7.28) | **38.1** (35.3-39.9) |
| | Females | **279** (271-289) | **2.00** (2.00-200) | nc | **3543** (2855-4633) | **20.00*** (4.49-45.4) | nc |

(continued)

| Table 5 Pharmacokinetic parameters of D-14851 (Day 1)<br>Pharmacokinetic parameters of D-24851<br>$Mean_{ar}$ (n=3 for each sex)<br>(min-max)<br>Day 1 | | | | | | | |
|---|---|---|---|---|---|---|---|
| **Dose** [mg/kg] | **Sex** | $C_{max, sd}$ [ng/ml] | $t_{max, sd}$ [h] | $AUC_{sd}$ [ng·h/ml] | $AUC_{\tau, sd}$ [ng-h/ml] | $t_{1/2}$ [h] | **CL/f** [ml/(min·kg)] |
| 12.1 | Males | **592** (552-618) | **2.67** (2.00-4.00) | **6981** (5994-8338) | **6874** (5914-7937) | **8.74** (5.26-12.0) | **295** (24.2-33.6) |
| | Females | **860** (414-1483) | **2.33** (1.00-4.00) | **8254** (3873-13082) | **7666** (4054-11217) | **11.6** (4.70-22.3) | **31.1** (15.4-52.1) |
| * these values are only for orientating, due to the unsufficient curve fitting | | | | | | | |

**Table 6**

| Table 6 PK parameters of D-24851 (Day 27)<br>Pharmacokinetic parameters of D-24851<br>$Mean_{or}$ (n=3 for each sex)<br>(min-max)<br>Day 27 | | | | | | | |
|---|---|---|---|---|---|---|---|
| **Dose** [mg/kg] | **Sex** | $C_{max, md}$ [ng/ml] | $t_{max, md}$ [h] | $AUC_{o-tlast,md}$ [ng·h/ml] | $AUC_{\tau, md}$ [ng·h/ml] | $t_{in}$ [h] | **CL/f** [ml/(min·kg)] |
| 2.61 | Males | **224** (147-290) | **1.33** (1.00-2.00) | **1447** (1240-1586) | **1736** (1574-1865) | **40.7** (35.0-46.7) | nc |
| | Females | **148** (138-164) | **2.33** (1.00-400) | **1104** (1049-1178) | **1413** (1356-1485) | **283*** (22.3-31.7) | nc |
| 5.62 | Males | **186** (176-200) | **2.33** (1.00-4.00) | **1323** (1065-1460) | **1852**** (1840-1864) | **5.10**** (4.99-5.22) | **38.1** (35.3-39.9) |
| | Females | **315** (271-376) | **2.33** (1.00-4.00) | **2737** (2265-3085) | **2963** (2616-3189) | **14.8** (7.02-30.3) | nc |
| 12.1 | Males | **435** (396-460) | **2.67** (2.00-4.00) | **5558** (4935-6738) | **5621** (4935-6738) | **11.9** (10.1-12.9) | **29.5** (24.2-33.6) |
| | Females | **329** (286-390) | **2.67** (2.00-4.00) | **4853** (4059-5564) | **4853** (4059-5564) | **24.2** (22.4-27.6) | **31.1** (15.4-52.1) |
| * these values are only for orientating, due to the insufficient curve fitting | | | | | | | |

The obtained sustained release profile is of special interest for D-24851 and other tubulin inhibitors of the present invention because of its mode of action. For tubulin inhibitors it is important to provide an effective drug concentration in a special cycle of proliferating cells. Due to the fact that not all cells are in the same cell cycle at the same time it is necessary to provide a sufficient plasma concentration over a long period of time to therapeutically affect as many cancer cells as possible. The present invention is particularly useful for highly toxic antineoplastic agents such as D-24851 because it may enable the reduction of total dosing, and therefore may provide an altered treatment regimen. Therefore the pharmacokinetic profile advantages of parenterally administered Composition 4 should lead to a higher efficacy of the drug versus traditional compositions.

The present compositions may be used in a method of treating a mammal, preferably a human being, by administering to the mammal a therapeutically effective amount of a composition. In general, such an amount will be from about 0.01mg/kg to about 100 mg/kg of tubulin inhibitor, administered in bolus or by controlled rate. Preferably, the dosing amount will be from about 0.1 mg/kg to about 10 mg/kg.

The route of administration (e.g., topical, parenteral or oral) and the dosage regimen will be determined by skilled clinicians, based on factors such as the exact nature of the condition being treated, the severity of the condition, the age and general physical condition of the patient, and so on. The specific type of formulation selected will depend on various factors, such as the compound, the dosage frequency, and the disease being treated.

As indicated above, use of the compositions of the present invention to treat cancer is a particularly important use for the compositions. Types of cancer to be treated include metastasizing carcinoma, including the spread of metastases, anti-tumor agent resistant tumors, tumors sensitive to tubulin inhibitors, or combinations thereof. Other medical disorders which may be treated include autoimmune diseases, asthma and allergic reactions and inflammatory disorders, including, pancreatitis, septic shock, allergic rhinitis, and rheumatoid arthritis. The compositions of the present invention can also be administered as an immuno-suppresant and for other immunomodulating activity.

**Example 12: IV Pharmacokinetics Comparision Study in Rats of Compositions 4 & 5**

D-24851 nanosuspension (Composition 4) intravenous pharmacokinetics were studied in rats. The dosing schedule was optimized by altering both dose and frequency with a Yoshida® AH13 sarcoma transplanted SC into a rat model, noting subsequent tumor growth. IV treatment into the tail vein was started at 0.1g tumor weight. Pharmacokinetics in the rat were determined in a 1 month study, dosing IV q2d with 2, 5, and 10mg/kg, analyzing both plasma and whole blood samples by HPLC. Tissue distribution was determined with $^{14}$C-D-24851 after 10mg/kg IV administration in male rats (n=3), compared with 0.25 mg/kg IV D-24851 in an organic solution (n=4), also used for PK comparison.

Mean particle size of the nanosuspension was 260nm, with 99% < 0.540 μm. Dose frequency could be reduced to twice per week, by simultaneously increasing dose level, resulting in 98% tumor inhibition, Table 7. At this optimized schedule, the importance of drug level is shown in Fig. 6.

**Table** 7

| Table 7. Dependence of tumor inhibition on dose frequency and dose. | | | |
|---|---|---|---|
| Schedule | Dose | Total Dose | Tumor Inhibition |
| doses/14d | (mg/kg) | (mg/kg) | (%) |
| 14 | 5 | 70 | 66 |
| 7 | 10 | 70 | 100 |
| 6 | 10 | 60 | 88 |
| 4 | 15 | 60 | 98 |

Intravenous pharmacokinetics after a single dose revealed increasing plasma concentration to yield a $C_{max}$ at a $t_{max}$ of 2 hrs, followed by sustained levels over a number of hours, before onset of the excretion phase, Fig. 7. Dose proportionality is seen with $C_{max}$ while AUC increases to a greater extent, probably reflecting saturation of metabolizing enzymes, Table 8. The miniscule concentration in the organic solution gave a much reduced AUC, $t_{max}$, and $t_{½}$.

**Table 8**

| Table 8. PK Parameters of Single Dose IV Administration of D-24851 Nanosuspension (Composition 4) and Solutol/Propanediol Solution (Composition 5), to Rats | | | | | | | | | | |
|---|---|---|---|---|---|---|---|---|---|---|
| Form | Dose | $C_{max}$ | | $t_{max}$ | | AUC | | $t_{1/2}$ | |
| | (mg/kg) | (ng/ml) | | (h) | | (ng*h/ml) | | (h) | |
| | | M | F | M | F | M | F | M | F |
| D-24851 nanosuspension (Composition 4) | 2 | 80.4 | 90.8 | 2 | 2 | 517 | 663 | 12 | 6.4 |
| D-24851 nanosuspension (Composition 4) | 5 | 155 | 172 | 2 | 2 | 921 | 1775 | 3.6 | 7.2 |

(continued)

| Table 8. PK Parameters of Single Dose IV Administration of D-24851 Nanosuspension (Composition 4) and Solutol/Propanediol Solution (Composition 5), to Rats | | | | | | | | | | |
|---|---|---|---|---|---|---|---|---|---|---|
| Form | Dose | $C_{max}$ | | $t_{max}$ | | AUC | | $t_{1/2}$ | | |
| | (mg/kg) | (ng/ml) | | (h) | | (ng*h/ml) | | (h) | | |
| | | M | F | M | F | M | F | M | F | |
| D-24851 nanosuspension (Composition 4) | 10 | 297 | 373 | 2 | 2 | 2729 | 5016 | 5.7 | 9.5 | |
| Solutol/Propanediol solution (Composition 5) | 0.25 | 83.5 | 92.8 | 0.2 | 0.1 | 80.6 | 73 | 1.1 | 0.7 | |

Repeated IV administration of 10mg/kg q2d in rats indicated comparable AUC and $C_{max}$ after day 15 as after day 1, Fig. 8. Hence no measurable drug accumulation was observed. Female rats exhibit increased AUC and $t_{½}$ relative to male rats. In general, the prolonged pharmacokinetics with high loading supports the observed schedule dependency, involving frequent dosing of high drug amounts. In contrast, the Solutol/Propanediol solution formulation (Composition 5) offers limited dosing with very short duration drug levels.

The prolonged PK is consistent with the tissue distribution results seen for the $^{14}C$ ADME study. Initially after IV administration, high levels are found in the organs of the MPS, the liver and spleen, and decrease subsequently. In comparison, with the Solutol/Propanediol solution of the drug (Composition 5), liver levels slowly rise with time. As D-24851 nanosuspension formulated drug (Composition 4) is slowly released from the tissues of the MPS, levels rise in other organs, such as the fat and intestine. For Composition 5, by contrast, the drug levels initially peak in these other tissues, and decline subsequently, Table. 9. Only 0.25 mg/kg drug could be delivered to the rat in the Solutol/Propanediol solution vehicle, because of toxicity. By contrast, 10 mg/kg of drug in D-24851 nanosuspension was administered.

**Table 9**

| Table 9. Tissue Distribution after IV administration: D-24851 vs. Solutol/Propanediol Solution | | | | | | | | |
|---|---|---|---|---|---|---|---|---|
| 14C-D-24851 ADME Tissue Distribution (%) | | | | | | | | |
| | Composition 4 | | | | Composition 5 | | | |
| Tissue | 6h | 18h | 30h | 48h | 4h | 8h | 24h | 48h |
| Liver | 33 | 18 | 24 | 17 | 11 | 11 | 13 | 20 |
| Spleen | 6.7 | 3.2 | 2.7 | 2.6 | 1.2 | 1.2 | 1.3 | 1.6 |
| Sm Intestine | 4.8 | 4.4 | 6.7 | 4.7 | 9.9 | 4.4 | 3.8 | 3.1 |
| Fat | 5.9 | 18 | 11 | 24 | 19 | 22 | 15 | 11 |

The dose dependent anti-tumor effect observed for D-24851 requires a formulation with sufficient loading for IV delivery. This was satisfactorily accomplished with a crystal nanosuspension. Tissue distribution indicated initial targeting of the nanosuspension to the organs of the MPS, the liver and spleen. Subsequently, drug was apparently released and tissue levels of drug increased in other organs expected to have an affinity for hydrophobic drugs, e.g. fat. Pharmacokinetics revealed increasing levels in the plasma, subsequent to IV administration, consistent with release of soluble drug from an initial depot, to yield prolonged drug levels, required for efficacy.

In comparison with Composition 5, the Solutol/Propanediol solution formulation, the D-24851 nanosuspension, Composition 4, permitted considerably higher dosing (15 vs. 0.25 mg/kg), and gave a prolonged plasma concentration level. Based upon the mechanism of action of cell-cycle sensitive oncolytics, this sustained activity is expected to be highly efficacious, as indicated in preliminary efficacy studies. Tissue distribution studies were consistent with an IV depot effect, indicated by the pharmacokinetics.

By utilising compositions in accordance with the present invention, it has been found that drugs previously considered to present bioavailabilty problems may be presented in dosage forms with superior bioavailability.

## Claims

1. A particulate pharmaceutical composition comprising particles with an effective average size of from about 15 nm to about 50 microns of at least one tubulin inhibitor compound of formula (1):

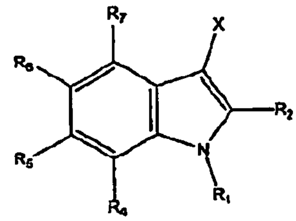

wherein X is

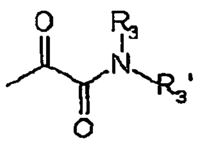

$R_3$ is aryl or heteroaryl,
$R_{3'}$ is hydrogen,
$R_4$, $R_5$, $R_6$ and $R_7$ are independently hydrogen, halogen or alkyl,
$R_1$ is arylalkyl; and
$R_2$ is hydrogen, alkyl, acyl, aryl, alkoxycarbonyl, aryloxycarbonyl, heteroaryloxycarbonyl, cycloalkoxycarbonyl, heterocycloalkoxycarbonyl, alkenyloxycarbonyl, cycloalkenyloxycarbonyl and heterocycloalkenyloxycarbonyl; and
at least one surfactant selected from the group consisting of non-ionic surfactants, anionic surfactants, cationic surfactants, biologically-derived surfactants, zwitterionic surfactants and amino acids.

2. The composition of Claim 1, wherein $R_1$ is a halogenated benzyl group, $R_2$, $R_4$, $R_5$, $R_6$ and $R_7$ are hydrogen and $R_3$ is a pyridine.

3. The composition of Claim 1, wherein the tubulin inhibitor compound is

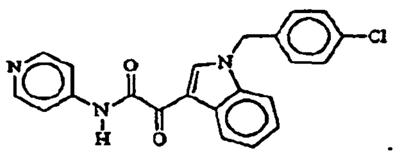

.

4. The composition of Claim 1, wherein the tubulin inhibitor compound is selected from the group consisting of:

N-(Pyridin-4-yl)-[1-(4-fluorobenzyl)indol-3-yl]glyoxylamide;
N-(Pyridin-3-yl)-[1-(4-fluorobenzyl)-indo1-3-yl]glyoxylamide;
N-(Pyridin-3-yl)-(1-benzylindol-3-yl)glyoxylamide;
N-(Pyridin-3-yl)-[1-(2-chlorobenzyl)indol-3-yl]glyoxylamide;
N-(4-Fluorophenyl)-[1-(4-fluorobenzyl)indol-3-yl]glyoxylamide;
N-(4-Nitrophenyl)-[1-(4-fluorobenzyl)indol-3-yl]glyoxylamide;
N-(2-Chloropyridine-3-yl)-[1-(4-fluorobenzyl)indol-3-yl]glyoxylamide;
N-(Pyridin-4-yl)-(1-benzylindol-3-yl)glyoxylamide;
N-(Pyridin-4-yl)-[1-(3-pyridylmethyl)indol-3-yl]glyoxylamide;
N-(4-Fluorophenyl)-[1-(2-pyridylmethyl)indol-3-yl]glyoxylamide;
N-(4-Fluorophenyl)-[1-(3-pyridylmethyl)indol-3-yl]glyoxylamide;
N-(Pyridin-4-yl)-[1-(4-chlorobenzyl)indol-3-yl]glyoxylamide;
N-(Pyridin-4-yl)-[1-(2-chlorobenzyl)indol-3-yl]glyoxylamide;

N-(Pyridin-2-yl)-[1-(4-fluorobenzyl)indol-3-yl]glyoxylamide;
N-(Pyridin-4-yl)-[1-(2-pyridylmethyl)indol-3-yl]glyoxylamide;
N-(Pyridin-2-yl)-(1-benzylindo1-3-yl)glyoxylamide;
N-(Pyridin-4-yl)-[1-(4-fluorobenzyl)-6-ethoxycarbonylaminoindol-3-yl]glyoxylamide;
N-(Pyridin-4-yl)-[1-(4-fluorobenzyl)-5-ethoxycarbonylaminoindol-3-yl]glyoxylamide;
N-(Pyridin-4-yl)-[1-(4-fluorobenzyl)-6-cyclopentyloxycarbonylaminoindol-3-yl] glyoxylamide;
N-(Pyridin-4-yl)-[1-(4-fluorobenzyl)-5-methoxyindol-3-yl]glyoxylamide; and
N-(Pyridin-4-yl-[1-(4-fluorobenzyl)-5-ethoxycarbonylaminomethylindol-3-yl] glyoxylamide.

5. The composition of any one of the preceding claims, wherein the surfactant comprises a non-ionic surfactant selected from the group consisting of polyoxyethylene fatty alcohol ethers, polyoxyethylene sorbitan fatty acid esters, polyoxyethylene fatty acid esters, sorbitan esters, glyceryl esters, glycerol monostearate, polyethylene glycols, polypropylene glycols, polypropylene glycol esters, cetyl alcohol, cetostearyl alcohol, stearyl alcohol, aryl alkyl polyether alcohols, polyoxyethylene-polyoxypropylene copolymers, poloxamers, poloxamines, methylcellulose, hydroxycellulose, hydroxymethylcellulose, hydroxypropylcellulose, hydroxypropylmethylcellulose, noncrystalline cellulose, polysaccharides, starch, hydroxyethylstarch, polyvinyl alcohol, polyvinylpyrrolidone, triethanolamine stearate, amine oxides, dextran, glycerol, gum acacia, cholesterol, tragacanth, cetomacrogol emulsifying wax, polyoxyethylene alkyl ethers, polyoxyethylene stearates, hydroxypropyl methylcellulose, methylcellulose, hydroxyethylcellulose, hydroxypropylmethylcellulose phthalate, noncrystalline cellulose, polyvinyl alcohol, polyvinylpyrrolidone, 4-(1,1,3,3-tetramethylbutyl)phenol polymer with ethylene oxide and formaldehyde, poloxamers, alkyl aryl polyether sulfonates, mixtures of sucrose stearate and sucrose distearate, $C_{18}H_{37}CH_2C(O)N(CH_3)CH_2(CHOH)_4(CH_2OH)_2$, p-isononylphenoxypoly(glycidol), decanoyl-N-methylglucamide, n-decyl-β-D-glucopyranoside, n-β-decyl-D-maltopyranoside, n-dodecyl-β-glucopyranoside, n-dodecyl-β-D-maltoside, heptanoyl-N-methylglucamide, n-heptyl-β-glucopyranoside, n-heptyl-β-D-thioglucoside, n-hexyl-β-D-glucopyranoside; nonanoyl-N-methylglucamide, n-nonyl-β-D-glucopyranoside, octanoyl-N-methylglucamide, n-octyl-β-D -glucopyranoside, octyl-β-D-thioglucopyranoside. PEG-cholesterol, PEG-vitamin A, PEG-vitamin E, and random copolymers of vinyl acetate and vinyl pyrrolidone.

6. The composition of any one of claims 1 to 4, wherein the surfactant comprises an anionic surfactant selected from the group consisting of alkyl sulfonates, aryl sulfonates, alkyl phosphates, alkyl phosphonates, potassium laurate, sodium lauryl sulfate, sodium dodecylsulfate, alkyl polyoxyethylene sulfates, sodium alginate, dioctyl sodium sulfosuccinate, phosphatidic acid and their salts, sodium carboxymethylcellulose, bile acids and their salts, cholic acid, deoxycholic acid, glycocholic acid, taurocholic acid, glycodeoxycholic acid, calcium carboxymethylcellulose, stearic acid and its salts, calcium stearate, phosphates, sodium dodecylsulfate, carboxymethylcellulose calcium, carboxymethylcellulose sodium, dioctylsulfosuccinate, dialkylesters of sodium sulfosuccinic acid, sodium lauryl sulfate, and phospholipids.

7. The composition of Claim 6, wherein the surfactant comprises a natural or synthetic phospholipid.

8. The composition of Claim 7, wherein the surfactant comprises a phospholipid selected from the group consisting of phosphatides, anionic phospholipids, phosphatidylserine, phosphatidylinositol, phosphatidylglycerol, phosphatidylinosine, phosphatidic acid, lysophospholipids, polyethylene glycol-phospholipid conjugates, egg phospholipids, soybean phospholipids, anionic PEG-phospholipids, and anionic methoxy PEG-phospholipids.

9. The composition of any one of claims 1 to 4, wherein the surfactant comprises a cationic surfactant selected from the group consisting of quaternary ammonium compounds, benzalkonium chloride, cetyltrimethylammonium bromide, chitosans, lauryldimethylbenzylammonium chloride, acyl carnitine hydrochlorides, alkyl pyridinium halides, cetyl pyridinium chloride, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, $C_{12-15}$-dimethyl hydroxyethyl ammonium chloride, $C_{12-15}$-dimethyl hydroxyethyl ammonium bromide, coconut dimethyl hydroxyethyl ammonium chloride, coconut dimethyl hydroxyethyl ammonium bromide, myristyl trimethyl ammonium methyl sulfate, lauryl dimethyl benzyl ammonium chloride, lauryl dimethyl benzyl ammonium bromide, lauryl dimethyl $(ethenoxy)_4$ ammonium chloride, lauryl dimethyl $(ethenoxy)_4$ ammonium bromide, N-alkyl $(C_{12-18})$dimethylbenzyl ammonium chloride, N-alkyl $(C_{14-18})$ dimethyl-benzyl ammonium chloride, N-tetradecyldimethylbenzyl ammonium chloride monohydrate, dimethyl didecyl ammonium chloride, N-alkyl $(C_{12-14})$ dimethyl 1-naphthylmethyl ammonium chloride, trimethylammonium halide

alkyl-trimethylammonium salts, dialkyl-dimethylammonium salts, lauryl trimethyl ammonium chloride, ethoxylated alkylamidoalkyldialkylammonium salts, ethoxylated trialkyl ammonium salts, dialkylbenzene dialkylammonium chloride, N-didecyldimethyl ammonium chloride, N-tetradecyldimethylbenzyl ammonium chloride monohydrate, N-alkyl ($C_{12-14}$) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, $C_{12}$ trimethyl ammonium bromides, $C_{15}$ trimethyl ammonium bromides, $C_{17}$ trimethyl ammonium bromides, dodecylbenzyl triethyl ammonium chloride, poly-diallyldimethylammonium chloride (DADMAC™), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT™, tetrabutylammonium bromide, benzyl trimethylammonium bromide, choline esters, stearalkonium chloride, cetyl pyridinium bromide, cetyl pyridinium chloride, halide salts of quatemized polyoxyethylalkylamines, MIRAPOL™, ALKAQUAT™, alkyl pyridinium salts, amines, amine salts, imide azolinium salts, protonated quaternary acrylamides, methylated quaternary polymers, cationic guar gum, dodecyl trimethyl ammonium bromide, triethanolamine, and poloxamines.

**10.** The composition of any one of claims 1 to 4, wherein the surfactant comprises a zwitterionic surfactant selected from the group consisting of zwitterionic phospholipids, phosphatidylcholine, diacyl-glycero-phosphoethanolamine, phosphatidylethanolamine, dimyristoyl-glycero-phosphoethanolamine (DMPE), dipalmitoyl-glycero-phosphoethanolamine (DPPE), distearoyl-glycero phosphoethanolamine (DSPE), dioleolyl-glycero-phosphoethanolamine (DOPE), pegylated phospholipids, PEGphosphatidylcholine, PEG-dlacyl-glycero phosphoethanolamine, PEG-phosphatidylethanolamine, PEG-dimyristoyl-glycero-phosphoethanolamine, PEG-dipalmitoyl-glycero-phosphoethanolamine, PEG-distearoyl-glycero-phosphoethanolamine, PEG-dioleolyl-glycero-phosphoethanolamine, methoxy polyethylene glycol (mPEG)-phospholipids, mPEG-phosphatidylcholine, mPEG diacyl-glycero-phosphoethanolamine, mPEG-phosphatidylethanolamine, mPEG-dimyristoyl-glycero-phosphoethanolamine, mPEG-dipalmitoyl-glycero-phosphoethanolamine, mPEG-distearoyl-glycero-phosphoethanolamine, and mPEG-dioleolyl-glycero-phosphoethanolamine.

**11.** The composition of any one of claims 1 to 4, wherein the surfactant comprises a biologkally-derived surfactant selected from the group consisting of lipoproteins, gelatin, casein, lysozyme, albumin, heparin, hirudin or other proteins.

**12.** The composition of any one of claims 1 to 4, wherein the surfactant comprises an amino acid selected from the group consisting of leucine, alanine, valine, isoleucine, lysine, aspartic acid, glutamic acid, methionine, and phenylalanine.

**13.** The composition of any one of the preceding claims, further comprising a pH adjusting agent

**14.** The composition of Claim 13, wherein the pH adjusting agent is selected from the group consisting of sodium hydroxide, hydrochloric acid, tris buffer, mono-, di-, tricarboxylic acids and their salts, citrate buffer, phosphate, acetate, lactate, tris(hydroxymethyl)aminomethane, aminosaccharides, mono-, di- and trialkylated amines, meglumine (N-methylglucosamine), and amino adds.

**15.** The composition of any one of the preceding claims, further comprising an osmotic pressure adjusting agent.

**16.** The composition of Claim 15, wherein the osmotic pressure adjusting agent is selected from the group consisting of glycerin, monosaccharides, inorganic salts, and sugar alcohols.

**17.** The composition of any one of the preceding claims, wherein the tubulin inhibitor compound is present in an amount of 0.1 mg/g to 200 mg/g.

**18.** The composition of Claim 17, wherein the tubulin inhibitor compound is present in an amount between about 0.5 mg/g to 50 mg/g.

**19.** The composition of Claim 17, wherein the tubulin inhibitor compound is present in an amount between about 1 mg/g to 50 mg/g.

**20.** The composition of any one of the preceding claims, wherein the particles have an effective average particle size of about 10 microns or less.

**21.** The composition of Claim 20, wherein the particles have an effective average particle size of about 2 microns or less.

**22.** The composition of any one of the preceding claims, wherein said composition is administrable by a route selected from the group consisting of parenteral, oral, buccal, periodontal, rectal, nasal, pulmonary, topical, transdermal, intravenous, intramuscular, subcutaneous, intradermal, intraoccular, intracerebral, intralymphatic, pulmonary, intraarticular, intrathecal and intraperitoneal.

**23.** The composition of any one of the preceding claims, wherein said composition is formulated into a liquid dispersion form selected from the group consisting of injectable formulations, solutions, delayed release formulations, controlled release formulations, extended release formulations, pulsatile release formulations and immediate release formulations.

**24.** The composition of any one of Claims 1 to 22, wherein said composition is formulated into a solid dosage form selected from the group consisting of tablets, coated tablets, capsules, ampoules, suppositories, lyophilized formulations, delayed release formulations, controlled release formulations, extended release formulations, pulsatile release formulations, immediate release and controlled release formulations.

**25.** The composition of Claim 22, wherein said composition is formulated into a form selected from the group consisting of patches, powder preparations which can be inhaled, suspensions, creams and ointments.

**26.** A method of making a particulate pharmaceutical composition according to Claim 1 comprising:

(i) dissolving an effective amount of at least one tubulin inhibitor compound as defined in Claim 1 in a water miscible first solvent to form a solution;
(ii) adding a surfactant or combination of surfactants;
(iii) mixing the solution with a second solvent to form a pre-suspension; and
(iv) adding energy to the pre-suspension to form a suspension of the particulate pharmaceutical composition.

**27.** The method of Claim 26, wherein the energy step includes sonication, homogenization, milling, high-shear extrusion, or microfluidization.

**28.** The method of Claim 26 or 27, wherein the first solvent is selected from the group consisting of N-methyl-2-pyrrolidinone, lactic acid, 2-pyrrolidone, dimethyl sulfoxide, dimethylacetamide, lactic acid, methanol, ethanol, Isopropanol, 3-pentanol, n-propanol, glycerol, butylene glycol, methylene glycol, propylene glycol, mono- and diacylated monoglycerides, dimethyl isosorbide, acetone, dimethylformamide, 1,4-dioxane, polyethylene glycol, polyethylene glycol esters, polyethylene glycol sorbitans, polyethylene glycol monoalkyl ethers, polypropylene glycol, polypropylene alginate, PPG-10 butanediol, PPG-10 methyl glucose ether, PPG-20 methyl glucose ether, PPG-15 stearyl ether, propylene glycol dicaprylate, propylene glycol dicaprate, propylene glycol laurate, propylene glycol carbonate, lactic acid, and acetic acid.

**29.** The method of any one of claims 26 to 28, wherein the second solvent is selected from the group consisting of water, buffers, salts, surfactant(s), water-soluble polymers, and combinations of excipients.

**30.** The method of any one of claims 26 to 29, wherein the surfactant is selected from the group consisting of non-ionic surfactants, anionic surfactants, cationic surfactants, biologically-derived surfactants, zwitterionic surfactants, and amino adds.

**31.** The method of Claim 30, wherein the surfactant comprises a nonionic surfactant selected from the group consisting of polyoxyethylene fatty alcohol ethers, polyoxyethylene sorbitan fatty acid esters, polyoxyethylene fatty acid esters, sorbitan esters, glyceryl esters, glycerol monostearate, polyethylene glycols, polypropylene glycols, polypropylene glycol esters, cetyl alcohol, cetostearyl alcohol, stearyl alcohol, aryl alkyl polyether alcohols, polyoxyethylene-polyoxypropylene copolymers, poloxamers, poloxamines, methylcellulose, hydroxycellulose, hydroxymethylcellulose, hydroxypropylcellulose, hydroxypropylmethylcellulose, noncrystalline cellulose, polysaccharides, starch, hydroxyethylstarch, polyvinyl alcohol, polyvinylpyrrolidone, triethanolamine stearate, amine oxides, dextran, glycerol, gum acacia, cholesterol, tragacanth, cetomacrogol emulsifying wax, polyoxyethylene alkyl ethers, polyoxyethylene stearates, hydroxypropyl celluloses, hydroxypropyl methylcellulose, methylcellulose, hydroxyethylcellulose, hydroxypropylmethylcellulose phthalate, noncrystalline cellulose, polyvinyl alcohol, polyvinylpyrrolidone, 4-(1,1,3,3-tetramethylbutyl)phenol polymer with ethylene oxide and formaldehyde, poloxamers, alkyl aryl polyether sulfonates, mixtures

of sucrose stearate and sucrose distearate, $C_{18}H_{37}CH_2C(O)N(CH_3)$ $CH_2(CHOH)_4(CH_2OH)_2$, p-isononylphenoxy-poly(glycidol), decanoyl-N-methylglucamide, n-decyl-β-glucopyranoside, n-decyl-β-D-maltopyranoside, n-dodecyl-β-D-glucopyranoside, n-dodecyl-β-D-maltoside, heptanoyl-N-methylglucamide, n-heptyl-β-D-glucopyranoside, n-heptyl-β-D-thioglucoside, n-hexyl-β-D-glucopyranoside; nonanoyl-N-methylglucamide, n-nonyl-β-D-glucopyranoside, octanoyl-N-methylglucamide, n-octyl-β-D-glucopyranoside, octyl-β-D-thioglucopyranoside, PEG-cholesterol, PEG-vitamin A, PEG-vitamin E, and random copolymers of vinyl acetate and vinyl pyrrolidone.

**32.** The method of Claim 30, wherein the surfactant comprises an anionic surfactant selected from the group consisting of alkyl sulfonates, aryl sulfonates, alkyl phosphates, alkyl phosphonates, potassium laurate, sodium lauryl sulfate, sodium dodecylsulfate, alkyl polyoxyethylene sulfates, sodium alginate, dioctyl sodium sulfosuccinate, phosphatidic acid and their salts, sodium carboxymethylcellulose, bile acids and their salts, cholic acid, deoxycholic acid, glycocholic acid, taurocholic acid, glycodeoxycholic acid, calcium carboxymethylcellulose, stearic add and its salts, calcium stearate, phosphates, sodium dodecylsulfate, carboxymethylcellulose calcium, carboxymethylcellulose sodium, dioctylsulfosuccinate, dialkylesters of sodium sulfosuccinic acid, sodium lauryl sulfate, and phospholipids.

**33.** The method of Claim 32, wherein the surfactant comprises a phospholipid that is natural or synthetic.

**34.** The method of Claim 33, wherein the surfactant comprises a phospholipid selected from the group consisting of phosphatides, anionic phospholipids, phosphatidylserine, phosphatidylinositol, phosphatidylglycerol, phosphatidylinosine, phosphatidic acid, lysophospholipids, polyethylene glycol-phospholipid conjugates, egg phospholipid, soybean phospholipids, anionic PEG-phospholipids, and anionic methoxy PEG-phospholipids.

**35.** The method of Claim 30, wherein the surfactant comprises a cationic surfactant selected from the group consisting of quaternary ammonium compounds, benzalkonium chloride, cetyltrimethylarnmonium bromide, chitosans, lauryldimethylbenzylammonium chloride, acyl carnitine hydrochlorides, alkyl pyridinium halides, cetyl pyridinium chloride, cationic lipids, polymethylmethacrylate trimethylammonium bromide, sulfonium compounds, polyvinylpyrrolidone-2-dimethylaminoethyl methacrylate dimethyl sulfate, hexadecyltrimethyl ammonium bromide, phosphonium compounds, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, $C_{12-15}$-dimethyl hydroxyethyl ammonium chloride, $C_{12-15}$-dimethyl hydroxyethyl ammonium bromide, coconut dimethyl hydroxyethyl ammonium chloride, coconut dimethyl hydroxyethyl ammonium bromide, myristyl trimethyl ammonium methyl sulfate, lauryl dimethyl benzyl ammonium chloride, lauryl dimethyl benzyl ammonium bromide, lauryl dimethyl $(ethenoxy)_4$ ammonium chloride, lauryl dimethyl $(ethenoxy)_4$ ammonium bromide, N-alkyl ($C_{12-18}$) dimethylbenzyl ammonium chloride, N-alkyl ($C_{14-18}$) dimethyl-benzyl ammonium chloride, N- tetradecyldimethylbenzyl ammonium chloride monohydrate, dimethyl didecyl ammonium chloride, N-alkyl and ($C_{12-14}$) dimethyl 1-naphthylmethyl ammonium chloride, trimethylammonium halide alkyl-trimethylammonium salts, dialkyl-dimethylammonium salts, lauryl trimethyl ammonium chloride, ethoxylated alkylamidoalkyldialkylammonium salts, ethoxylated trialkyl ammonium salts, dialkylbenzene dialkylammonium chloride, N-didecyldimethyl ammonium chloride, N-tetradecyldimethylbenzyl ammonium chloride monohydrate, N-alkyl($C_{12-14}$) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, $C_{12}$ trimethyl ammonium bromides, $C_{15}$ trimethyl ammonium bromides, $C_{17}$ trimethyl ammonium bromides, dodecylbenzyl triethyl ammonium chlorine, poly-diallyldimethylammonium chloride (DADMAC™), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT™ 10, tetrabutylammonium bromide, benzyl trimethylammonium bromide, choline esters, stearalkonium chloride, cetyl pyridinium bromide, cetyl pyridinium chloride, halide salts of quaternized polyoxyethylalkylamines, MIRAPOL™, ALKAQUAT™, alkyl pyridinium salts, amines, amine salts, imide azolinium salts, protonated quaternary acrylamides, methylated quaternary polymers, cationic guar gum, dodecyl trimethyl ammonium bromide, triethanolamine, and poloxarnines.

**36.** The method of Claim 30, wherein the surfactant comprises a zwitterionic surfactant selected from the group consisting of zwitterionic phospholipids, phosphatidylcholine, diacyl-glycero-phosphoethanolamine, phosphatidylethanolamine, dimyristoyl-glycero-phosphoethanolamine (DMPE), dipalmitoyl-glycero-phosphoethanolamine (DPPE), distearoyl-glycero phosphoethanolamine (DSPE), dioleolyl-glycero-phosphoethanolamine (DOPE), pegylated phospholipids, PEG-phosphatidylcholine, PEG-diacyl-glycero-phosphoethanolamine, PEG-phosphatidylethanolamine, PEG-dimyristoyl-glycero-phosphoethanolamine, PEG-dipalmitoyl-glycero-phosphoethanolamine, PEG-distearoyl-glycero-phosphoethanolamine, PEG-dioleolyl-glycero-phosphoethanolamine, methoxy polyethylene glycol (mPEG)-phospholipids, mPEG-phosphatidylcholine, mPEG-diacyl-glycero-phosphoethanolamine, mPEG-phosphatidylethanolamine, mPEG-dimyristoyl-glycero-phosphoethanolamine, mPEG-dipalmitoyl-glycero-phosphoethanolamine, mPEG-distearoyl-glycero-phosphoethanolamine, and mPEG-dioleolyl-glycero-phosphoethanolamine.

**37.** The method of Claim 34, wherein the surfactant comprises a biologically-derived surfactant selected from the group consisting of lipoproteins, gelatin, casein, lysozyme. albumin, heparin, hirudin or other proteins.

**38.** The method of Claim 30, wherein the surfactant comprises an amino acid selected from the group consisting of leucine, alanine, valine, isoleucine, lysine, aspartic acid, glutamic acid, methionine, and phenylalanine.

**39.** The method of any one of Claims 29 to 38, wherein the tubulin inhibitor compound is a compound as claimed in any one of Claims 2 to 4.

**40.** The method of any one of Claims 29 to 39, wherein the particles have an average particle size of about 10 microns or less.

**41.** The method of claim 40, wherein the particles have an average particle size of about 2 microns or less.

**42.** Use of particles of from about 15 nm to about 50 microns of at least one tubulin inhibitor compound of Claim 1 in the manufacture of a medicament for the treatment of mammals.

**43.** The use of Claim 42, wherein the medicament is for treating a medical disorder selected from the group consisting of immunological disorders, inflammatory disorders, antitumor agent resistant tumors, metastasizing carcinoma including development and spread of metastases, tumors sensitive to tubulin inhibitors or tumors that are both antitumor agent resistant and sensitive to tubulin inhibitors, pancreatitis, septic shock allergic rhinitis, and rheumatoid arthritis, and autoimmune diseases.

**44.** The use of Claim 42 or 43, wherein the tubulin compound is a compound as claimed in any one of Claims 2 to 4.

**45.** The use of Claim 42, wherein the medicament has anti-tumor, anti-asthmatic, anti-allergic, immunosuppressant or immunomodulating activity.

**46.** A particulate pharmaceutical composition according to any one of claims 1 to 4 for use in the treatment of mammals in need thereof.

**47.** A particulate pharmaceutical composition for the use according to claim 46 for use in treating a medical disorder selected from the group consisting of immunological disorders, inflammatory disorders, antitumor agent resistant tumors, metastasizing carcinoma including development and spread of metastases, tumors sensitive to tubulin inhibitors or tumors that are both antitumor agent resistant and sensitive to tubulin inhibitors, pancreatitis, septic shock allergic rhinitis, and rheumatoid arthritics, and autoimmune diseases.

**48.** A particulate pharmaceutical composition for the use according to claim 46 having antitumor, anti-asthmatic, anti-allergic, immunosuppressant or immunomodulating activity.

## Patentansprüche

**1.** Teilchenförmige pharmazeutische Zusammensetzung, die Teilchen mit einer effektiven durchschnittlichen Größe von etwa 15 nm bis etwa 50 Mikrometer von mindestens einer Tubulin-Hemmstoffverbindung der Formel (1)

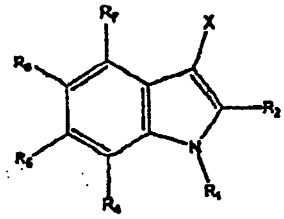

worin X

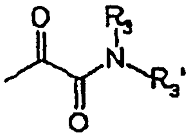

ist,
$R_3$ Aryl oder Heteroaryl ist,
$R_{3'}$ Wasserstoff ist,
$R_4$, $R_5$, $R_6$ und $R_7$ unabhängig Wasserstoff, Halogen oder Alkyl sind,
$R_1$ Arylalkyl ist und
$R_2$ Wasserstoff, Alkyl, Acyl, Aryl, Alkoxycarbonyl, Aryloxycarbonyl, Heteroaryloxycarbonyl, Cycloalkoxycarbonyl, Heterocycloalkoxycarbonyl, Alkenyloxycarbonyl, Cycloalkenyloxycarbonyl und Heterocycloalkenyloxycarbonyl ist, und
mindestens ein grenzflächenaktives Mittel, ausgewählt aus der Gruppe, bestehend aus nicht-ionischen grenzflächenaktiven Mitteln, anionischen grenzflächenaktiven Mitteln, kationischen grenzflächenaktiven Mitteln, biologisch abgeleiteten grenzflächenaktiven Mitteln, zwitterionischen grenzflächenaktiven Mitteln und Aminosäuren, umfasst.

2. Zusammensetzung nach Anspruch 1, bei der $R_1$ eine halogenierte Benzylgruppe ist, $R_2$, $R_4$, $R_5$, $R_6$ und $R_7$ Wasserstoff sind und $R_3$ ein Pyridin ist.

3. Zusammensetzung nach Anspruch 1, bei der die Tubulin-Hemmstoffverbindung

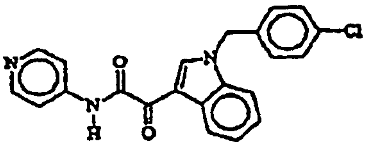

ist.

4. Zusammensetzung nach Anspruch 1, bei der die Tubulin-Hemmstoffverbindung aus der Gruppe, bestehend aus
N-(Pyridin-4-yl)-[1-(4-fluorbenzyl)indol-3-yl]glyoxylamid,
N-(Pyridin-3-yl)-[1-(4-fluorbenzyl)-indol-3-yl]glyoxylamid,
N-(Pyridin-3-yl)-[1-benzylindol-3-yl]glyoxylamid,
N-(Pyridin-3-yl)-[1-(2-chlorbenzyl)indol-3-yl]glyoxylamid,
N-(4-Fluorphenyl)-[1-(4-fluorbenzyl)indol-3-yl]glyoxylamid,
N-(4-Nitrophenyl)-[1-(4-fluorbenzyl)indol-3-yl]glyoxylamid,
N-(2-Chlorpyridin-3-yl)-[1-(4-fluorbenzyl)indol-3-yl]glyoxylamid,
N-(Pyridin-4-yl)-[1-benzylindol-3-yl]glyoxylamid,
N-(Pyridin-4-yl)-[1-(3-pyridylmethyl)indol-3-yl]glyoxylamid,
N-(4-Fluorphenyl)-[1-(2-pyridylmethyl)indol-3-yl]glyoxylamid,
N-(4-Fluorphenyl)-[1-(3-pyridylmethyl)indol-3-yl]glyoxylamid,
N-(Pyridin-4-yl)-[1-(4-chlorbenzyl)indol-3-yl]glyoxylamid,
N-(Pyridin-4-yl)-[1-(2-chlorbenzyl)indol-3-yl]glyoxylamid,
N-(Pyridin-2-yl)-[1-(4-fluorbenzyl)indol-3-yl]glyoxylamid,

N-(Pyridin-4-yl)-[1-(2-pyridylmethyl)indol-3-yl]glyoxylamid,
N-(Pyridin-2-yl)-[1-benzylindol-3-yl]glyoxylamid,
N-(Pyridin-4-yl)-[1-(4-fluorbenzyl)-6-ethoxycarbonylaminoindol-3-yl]glyoxylamid,
N-(Pyridin-4-yl)-[1-(4-fluorbenzyl)-5-ethoxycarbonylaminoindol-3-yl]glyoxylamid,
N-(Pyridin-4-yl)-[1-(4-fluorbenzyl)-6-cyclopentyloxycarbonylaminoindol-3-yl]glyoxylamid,
N-(Pyridin-4-yl)-[1-(4-fluorbenzyl)-5-methoxyindol-3-yl]glyoxylamid und
N-(Pyridin-4-yl)-[1-(4-fluorbenzyl)-5-ethoxycarbonylaminomethylindol-3-yl]glyoxylamid,
ausgewählt ist.

5. Zusammensetzung nach einem der vorhergehenden Ansprüche, bei der das grenzflächenaktive Mittel ein nichtionisches grenzflächenaktives Mittel umfasst, das aus der Gruppe, bestehend aus Polyoxyethylenfettalkoholethern, Polyoxyethylensorbitanfettsäureestern, Polyoxyethylenfettsäureestern, Sorbitanestem, Glycerylestem, Glycerinmonostearat, Polyethylenglykolen, Polypropylenglykolen, Polypropylenglykolestern, Cetylalkohol, Cetostearylalkohol, Stearylalkohol, Arylalkylpolyetheralkoholen, Polyoxyethylen-Polyoxypropylen-Copolymeren, Poloxameren, Poloxaminen, Methylcellulose, Hydroxycellulose, Hydroxymethylcellulose, Hydroxypropylcellulose, Hydroxypropylmethylcellulose, nicht-kristalliner Cellulose, Polysacchariden, Stärke, Hydroxyethylstärke, Polyvinylalkohol, Polyvinylpyrrolidon, Triethanolaminstearat, Aminoxiden, Dextran, Glycerin, Akaziengummi, Cholesterin, Tragant, emulgierendem Cetomakrogol-Wachs, Polyoxyethylenalkylethem, Polyoxyethylenstearaten, Hydroxypropylmethylcellulose, Methylcellulose, Hydroxyethylcellulose, Hydroxypropylmethylcellulosephthalat, nicht-kristalliner Cellulose, Polyvinylalkohol, Polyvinylpyrrolidon, 4-(1,1,3,3-Tetramethylbutyl)phenolpolymer mit Ethylenoxid und Formaldehyd, Poloxameren, Alkylarylpolyethersulfonaten, Gemischen von Saccharosestearat und Saccharosedistearat, $C_{18}H_{37}CH_2C(O)N(CH_3)CH_2(CHOH)_4(CH_2OH)_2$, p-Isononylphenoxypoly(glycidol), Decanoyl-N-methylglukamid, n-Decyl-β-D-glukopyranosid, n-β-Decyl-D-maltopyranosid, n-Dodecyl-β-D-glukopyranosid, n-Dodecyl-β-D-maltosid, Heptanoyl-N-methylglukamid, n-Heptyl-β-D-glukopyranosid, n-Heptyl-β-D-thioglukosid, n-Hexyl-β-D-glukopyranosid, Nonanoyl-N-methylglukamid, n-Nonyl-β-D-glukopyranosid, Octanoyl-N-methylglukamid, n-Octyl-β-D-glukopyranosid, Octyl-β-D-thioglukopyranosid, PEG-Cholesterin, PEG-Vitamin A, PEG-Vitamin E und statistischen Copolymeren von Vinylacetat und Vinylpyrrolidon, ausgewählt ist.

6. Zusammensetzung nach einem der Ansprüche 1 bis 4, bei der das grenzflächenaktive Mittel ein anionisches grenzflächenaktives Mittel umfasst, das aus der Gruppe, bestehend aus Alkylsulfonaten, Arylsulfonaten, Alkylphosphaten, Alkylphosphonaten, Kaliumlaurat, Natriumlaurylsulfat, Natriumdodecylsulfat, Alkylpolyoxyethylensulfaten, Natriumalginat, Dioctylnatriumsulfosuccinat, Phosphatidsäure und deren Salzen, Natriumcarboxymethylcellulose, Gallensäuren und deren Salzen, Cholsäure, Desoxycholsäure, Glykocholsäure, Taurocholsäure, Glykodesoxycholsäure, Calciumcarboxymethylcellulose, Stearinsäure und deren Salzen, Calciumstearat, Phosphaten, Natriumdodecylsulfat, Calcium-Carboxymethylcellulose, Natrium-Carboxymethylcellulose, Dioctylsulfosuccinat, Dialkylestem von Natriumsulfobernsteinsäure, Natriumlaurylsulfat und Phospholipiden, ausgewählt ist.

7. Zusammensetzung nach Anspruch 6, bei der das grenzflächenaktive Mittel ein natürliches oder synthetisches Phospholipid umfasst.

8. Zusammensetzung nach Anspruch 7, bei der das grenzflächenaktive Mittel ein Phospholipid umfasst, das aus der Gruppe, bestehend aus Phosphatiden, anionischen Phospholipiden, Phosphatidylserin, Phosphatidylinosit, Phosphatidylglycerin, Phosphatidylinosin, Phosphatidsäure, Lysophospholipiden, Polyethylenglykol-Phospholipid-Konjugaten, Ei-Phospholipiden, Sojabohnen-Phospholipiden, anionischen PEG-Phospholipiden und anionischen Methoxy-PEG-Phospholipiden, ausgewählt ist.

9. Zusammensetzung nach einem der Ansprüche 1 bis 4, bei der das grenzflächenaktive Mittel ein kationisches grenzflächenaktives Mittel umfasst, das aus der Gruppe, bestehend aus quaternären Ammoniumverbindungen, Benzalkoniumchlorid, Cetyltrimethylammoniumbromid, Chitosanen, Lauryldimethylbenzylammoniumchlorid, Acylcarnitinhydrochloriden, Alkylpyridiniumhalogeniden, Cetylpyridiniumchlorid, kationischen Lipiden, Polymethylmethacrylattrimethylammoniumbromid, Sulfoniumverbindungen, Polyvinylpyrrolidon-2-dimethylaminoethylmethacrylatdimethylsulfat, Hexadecyltrimethylammoniumbromid, Phosphoniumverbindungen, Benzyl-di(2-chlorethyl) ethylammoniumbromid, Kokosnusstrimethylammoniumchlorid, Kokosnusstrimethylammoniumbromid, Kokosnussmethyldihydroxyethylammoniumchlorid, Kokosnussmethyldihydroxyethylammoniumbromid, Decyltriethylammoniumchlorid, Decyldimethylhydroxyethylammoniumchlorid, Decyldimethylhydroxyethylammoniumbromid, $C_{12-15}$-Dimethylhydroxyethylammoniumchlorid, $C_{12-15}$-Dimethylhydroxyethyl-ammoniumbromid, Kokosnussdimethylhydroxyethylammoniumchlorid, Kokosnussdimethylhydroxyethylammoniumbromid, Myristyltrimethylammoniummethylsulfat, Lauryldimethylbenzylammoniumchlorid, Lauryldimethylbenzylammoniumbromid, Lauryldimethyl(ethen-

oxy)$_4$-ammoniumchlorid, Lauryldimethyl(ethenoxy)$_4$-ammonium-bromid, N-Alkyl($C_{12-18}$)dimethylbenzylammoniumchlorid, N-Alkyl($C_{14-18}$)dimethylbenzylammoniumchlorid, N-Tetradecyldimethylbenzylammoniumchloridmonohydrat, Dimethyldidecylammoniumchlorid, N-Alkyl($C_{12-14}$)dimethyl-1-naphthylmethylammoniumchlorid, Trimethylammoniumhalogenidalkyltrimethylammoniumsalzen, Dialkyldimethylammoniumsalzen, Lauryltrimethylammoniumchlorid, ethoxylierten Alkylamidoalkyldialkylammoniumsalzen, ethoxylierten Trialkylammoniumsalzen, Dialkylbenzoldialkylammoniumchlorid, N-Didecyldimethylammoniumchlorid, N-Tetradecyldimethylbenzylammoniumchloridmonohydrat, N-Alkyl($C_{12-14}$)dimethyl-1-naphthylmethylammoniumchlorid, Dodecyldimethylbenzylammoniumchlorid, Dialkylbenzolalkylammoniumchlorid, Lauryltrimethylammoniumchlorid, Alkylbenzylmethylammoniumchlorid, Alkylbenzyldimethylammoniumbromid, $C_{12}$-Trimethylammoniumbromiden, $C_{15}$-Trimethylammoniumbromiden, $C_{17}$-Trimethylammoniumbromiden, Dodecylbenzyltriethylammoniumchlorid, Polydiallyldimethylammoniumchlorid (DADMAC™), Dimethylammoniumchloriden, Alkyldimethylammoniumhalogeniden, Tricetylmethylammoniumchlorid, Decyltrimethylammoniumbromid, Dodecyltriethylammoniumbromid, Tetradecyltrimethylammoniumbromid, Methyltrfoctylammoniumchlorid, POLYQUAT™, Tetrabutylammoniumbromid, Benzyltrimethylammoniumbromid, Cholinestern, Stearalkoniumchlorid, Cetylpyridiniumbromid, Cetylpyridiniumchlorid, Halogenidsalzen von quatemisierten Polyoxyethylalkylaminen, MIRAPOL™, ALKAQUAT™, Alkylpyridiniumsalzen, Aminen, Aminsalzen, Imidazoliniumsalzen, protonierten quaternären Acrylamiden, methylierten quaternären Polymeren, kationischem Guargummi, Dodecyltrimethylammoniumbromid, Triethanolamin und Poloxaminen, ausgewählt ist.

**10.** Zusammensetzung nach einem der Ansprüche 1 bis 4, bei der das grenzflächenaktive Mittel ein zwitterionisches grenzflächenaktives Mittel umfasst, das aus der Gruppe, bestehend aus zwitterionischen Phospholipiden, Phosphatidylcholin, Diacylglycerophosphoethanolamin, Phosphatidylethanolamin, Dimyristoylglycerophosphoethanolamin (DMPE), Dipalmitoylglycerophosphoethanolamin (DPPE), Distearoylglycerophosphoethanolamin (DSPE), Dioleoylglycerophosphoethanolamin (DOPE), pegylierten Phospholipiden, PEG-Phosphatdiylcholin, PEG-Diacylglycerophosphoethanolamin, PEG-Phosphatidylethanolamin, PEG-Dimyristoylglycerophosphoethanolamin, PEG-Dipalmitoylglycerophosphoethanolamin, PEG-Distearoylglycerophosphoethanolamin, PEG-Dioleoylglycerophosphoethanolamin, Methoxypolyethylenglykol(mPEG)-phospholipiden, mPEG-Phosphatidylcholin, mPEG-Diacylglycerophosphoethanolamin, mPEG-Phosphatidylethanolamin, mPEG-Dimyristoylglycerophosphoethanolamin, mPEG-Dipalmitoylglycerophosphoethanolamin, mPEG-Distearoylglycerophosphoethanolamin und mPEG-Dioleoylglycerophosphoethanolamin, ausgewählt ist.

**11.** Zusammensetzung nach einem der Ansprüche 1 bis 4, bei der das grenzflächenaktive Mittel ein biologisch abgeleitetes grenzflächenaktives Mittel umfasst, das aus der Gruppe, bestehend aus Lipoproteinen, Gelatine, Kasein, Lysozym, Albumin, Heparin, Hirudin oder anderen Proteinen, ausgewählt ist.

**12.** Zusammensetzung nach einem der Ansprüche 1 bis 4, bei der das grenzflächenaktive Mittel eine Aminosäure umfasst, die aus der Gruppe, bestehend aus Leucin, Alanin, Valin, Isoleucin, Lysin, Asparaginsäure, Glutaminsäure, Methionin und Phenylalanin, ausgewählt ist.

**13.** Zusammensetzung nach einem der vorhergehenden Ansprüche, die ferner ein pH-Einstellmittel umfasst.

**14.** Zusammensetzung nach Anspruch 13, bei der das pH-Einstellmittel aus der Gruppe, bestehend aus Natriumhydroxid, Chlorwasserstoffsäure, Tris-Puffer, Mono-, Di-, Tricarbonsäuren und deren Salzen, Citratpuffer, Phosphat, Acetat, Lactat, Tris(hydroxymethyl)aminomethan, Aminosacchariden, mono-, di- und trialkylierten Aminen, Meglumin (N-Methylglukosamin) und Aminosäuren, ausgewählt ist.

**15.** Zusammensetzung nach einem der vorhergehenden Ansprüche, die ferner ein Mittel zum Einstellen des osmotischen Drucks umfasst.

**16.** Zusammensetzung nach Anspruch 15, bei der das Mittel zum Einstellen des osmotischen Drucks aus der Gruppe, bestehend aus Glycerin, Monosacchariden, anorganischen Salzen und Zuckeralkoholen, ausgewählt ist.

**17.** Zusammensetzung nach einem der vorhergehenden Ansprüche, bei der die Tubulin-Hemmstoffverbindung in einer Menge von 0,1 mg/g bis 200 mg/g vorliegt.

**18.** Zusammensetzung nach Anspruch 17, bei der die Tubulin-Hemmstoffverbindung in einer Menge zwischen etwa 0,5 mg/g bis 50 mg/g vorliegt.

**19.** Zusammensetzung nach Anspruch 17, bei der die Tubulin-Hemmstoffverbindung in einer Menge zwischen etwa 1

mg/g bis 50 mg/g vorliegt.

**20.** Zusammensetzung nach einem der vorhergehenden Ansprüche, bei der die Teilchen eine effektive durchschnittliche Teilchengröße von etwa 10 Mikrometer oder weniger aufweisen.

**21.** Zusammensetzung nach Anspruch 20, bei der die Teilchen eine effektive durchschnittliche Teilchengröße von etwa 2 Mikrometer oder weniger aufweisen.

**22.** Zusammensetzung nach einem der vorhergehenden Ansprüche, wobei die Zusammensetzung über einen Weg verabreicht werden kann, der aus der Gruppe, bestehend aus parenteral, oral, bukkal, periodontal, rektal, nasal, pulmonal, topisch, transdermal, intravenös, intramuskulär, subkutan, intradermal, intraokular, intracerebral, intralymphatisch, pulmonal, intraartikulär, intrathekal und intraperitoneal, ausgewählt ist.

**23.** Zusammensetzung nach einem der vorhergehenden Ansprüche, wobei die Zusammensetzung zu einer flüssigen Dispersionsform formuliert ist, die aus der Gruppe, bestehend aus injizierbaren Formulierungen, Lösungen, Formulierungen mit verzögerter Freisetzung, Formulierungen mit kontrollierter Freisetzung, Formulierungen mit verlängerter Freisetzung, Formulierungen mit diskontinuierlicher Freisetzung und Formulierungen mit sofortiger Freisetzung, ausgewählt ist.

**24.** Zusammensetzung nach einem der Ansprüche 1 bis 22, wobei die Zusammensetzung zu einer festen Dosierungsform formuliert ist, die aus der Gruppe, bestehend aus Tabletten, beschichteten Tabletten, Kapseln, Ampullen, Zäpfchen, lyophilisierten Formulierungen, Formulierungen mit verzögerter Freisetzung, Formulierungen mit kontrollierter Freisetzung, Formulierungen mit verlängerter Freisetzung, Formulierungen mit diskontinuierlicher Freisetzung und Formulierungen mit sofortiger Freisetzung und kontrollierter Freisetzung, ausgewählt ist.

**25.** Zusammensetzung nach Anspruch 22, wobei die Zusammensetzung zu einer Form formuliert ist, die aus der Gruppe, bestehend aus Pflastern, Pulverpräparaten, die eingeatmet werden können, Suspensionen, Cremes und Salben, ausgewählt ist.

**26.** Verfahren zur Herstellung einer teilchenförmigen pharmazeutischen Zusammensetzung nach Anspruch 1, umfassend:

(i) Lösen einer effektiven Menge mindestens einer Tubulin-Hemmstoffverbindung gemäß Anspruch 1 in einem wassermischbaren ersten Lösungsmittel zur Bildung einer Lösung,
(ii) Zusetzen eines grenzflächenaktiven Mittels oder einer Kombination von grenzflächenaktiven Mitteln,
(iii) Mischen der Lösung mit einem zweiten Lösungsmittel zur Bildung einer Vorsuspension und
(iv) Einbringen von Energie in die Vorsuspension zur Bildung einer Suspension der teilchenförmigen pharmazeutischen Zusammensetzung.

**27.** Verfahren nach Anspruch 26, bei dem der Energieschritt ein Ultraschallbehandeln, Homogenisieren, Mahlen, Extrudieren mit hoher Scherung oder Mikrofluidisieren umfasst.

**28.** Verfahren nach Anspruch 26 oder 27, bei dem das erste Lösungsmittel aus der Gruppe, bestehend aus N-Methyl-2-pyrrolidinon, Milchsäure, 2-Pyrrolidon, Dimethylsulfoxid, Dimethylacetamid, Milchsäure, Methanol, Ethanol, Isopropanol, 3-Pentanol, n-Propanol, Glycerin, Butylenglykol, Ethylenglykol, Propylenglykol, mono- und diacylierten Monoglyceriden, Dimethylisosorbid, Aceton, Dimethylformamid, 1,4-Dioxan, Polyethylenglykol, Polyethylenglykolestem, Polyethylenglykolsorbitanen, Polyethylenglykolmonoalkylethem, Polypropylenglykol, Polypropylenalginat, PPG-10-Butandiol, PPG-10-Methylglukoseether, PPG-20-Methylglukoseether, PPG-15-Stearylether, Propylenglykoldicaprylat, Propylenglykoldicaprat, Propylenglykollaurat, Propylenglykolcarbonat, Milchsäure und Essigsäure, ausgewählt ist.

**29.** Verfahren nach einem der Ansprüche 26 bis 28, bei dem das zweite Lösungsmittel aus der Gruppe, bestehend aus Wasser, Puffern, Salzen, einem grenzflächenaktiven Mittel oder grenzflächenaktiven Mitteln, wasserlöslichen Polymeren und Kombinationen von Trägem, ausgewählt ist.

**30.** Verfahren nach einem der Ansprüche 26 bis 29, bei dem das grenzflächenaktive Mittel aus der Gruppe, bestehend aus nicht-ionischen grenzflächenaktiven Mitteln, anionischen grenzflächenaktiven Mitteln, kationischen grenzflächenaktiven Mitteln, biologisch abgeleiteten grenzflächenaktiven Mitteln, zwitterionischen grenzflächenaktiven Mitteln und Aminosäuren, ausgewählt ist.

**31.** Verfahren nach Anspruch 30, bei dem das grenzflächenaktive Mittel ein nicht-ionisches grenzflächenaktives Mittel umfasst, das aus der Gruppe, bestehend aus Polyoxyethylenfettalkoholethern, Polyoxyethylensorbitanfettsäureestern, Polyoxyethylenfettsäureestern, Sorbitanestern, Glycerylestern, Glycerinmonostearat, Polyethylenglykolen, Polypropylenglykolen, Polypropylenglykolestern, Cetylalkohol, Cetostearylalkohol, Stearylalkohol, Arylalkylpolyetheralkoholen, Polyoxyethylen-Polyoxypropylen-Copolymeren, Poloxameren, Poloxaminen, Methylcellulose, Hydroxycellulose, Hydroxymethylcellulose, Hydroxypropylcellulose, Hydroxypropylmethylcellulose, nicht-kristalliner Cellulose, Polysacchariden, Stärke, Hydroxyethylstärke, Polyvinylalkohol, Polyvinylpyrrolidon, Triethanolaminstearat, Aminoxiden, Dextran, Glycerin, Akaziengummi, Cholesterin, Tragant, emulgierendem Cetomakrogol-Wachs, Polyoxyethylenalkylethern, Polyoxyethylenstearaten, Hydroxypropylcellulosen, Hydroxypropylmethylcellulose, Methylcellulose, Hydroxyethylcellulose, Hydroxypropylmethylcellulosephthalat, nicht-kristalliner Cellulose, Polyvinylalkohol, Polyvinylpyrrolidon, 4-(1,1,3,3-Tetramethylbutyl)phenolpolymer mit Ethylenoxid und Formaldehyd, Poloxameren, Alkylarylpolyethersulfonaten, Gemischen von Saccharosestearat und Saccharosedistearat, $C_{18}H_{37}CH_2C(O)N(CH_3)CH_2(CHOH)_4(CH_2OH)_2$, p-Isononylphenoxypoly(glycidol), Decanoyl-N-methylglukamid, n-Decyl-β-D-glukopyranosid, n-Decyl-β-D-maltopyranosid, n-Dodecyl-β-D-glukopyranosid, n-Dodecyl-β-D-maltosid, Heptanoyl-N-methylglukamid, n-Heptyl-β-D-glukopyranosid, n-Heptyl-β-D-thioglukosid, n-Hexyl-β-D-glukopyranosid, Nonanoyl-N-methylglukamid, n-Nonyl-β-D-glukopyranosid, Octanoyl-N-methylglukamid, n-Octyl-β-D-glukopyranosid, Octyl-β-D-thioglukopyranosid, PEG-Cholesterin, PEG-Vitamin A, PEG-Vitamin E und statistischen Copolymeren von Vinylacetat und Vinylpyrrolidon, ausgewählt ist.

**32.** Verfahren nach Anspruch 30, bei dem das grenzflächenaktive Mittel ein anionisches grenzflächenaktives Mittel umfasst, das aus der Gruppe, bestehend aus Alkylsulfonaten, Arylsulfonaten, Alkylphosphaten, Alkylphosphonaten, Kaliumlaurat, Natriumlaurylsulfat, Natriumdodecylsulfat, Alkylpolyoxyethylensulfaten, Natriumalginat, Dioctylnatriumsulfosuccinat, Phosphatidsäure und deren Salzen, Natriumcarboxymethylcellulose, Gallensäuren und deren Salzen, Cholsäure, Desoxycholsäure, Glykocholsäure, Taurocholsäure, Glykodesoxycholsäure, Calciumcarboxymethylcellulose, Stearinsäure und deren Salzen, Calciumstearat, Phosphaten, Natriumdodecylsulfat, Calcium-Carboxymethylcellulose, Natrium-Carboxymethylcellulose, Dioctylsulfosuccinat, Dialkylestern von Natriumsulfobernsteinsäure, Natriumlaurylsulfat und Phospholipiden, ausgewählt ist.

**33.** Verfahren nach Anspruch 32, bei dem das grenzflächenaktive Mittel ein Phospholipid umfasst, das natürlich oder synthetisch ist.

**34.** Verfahren nach Anspruch 33, bei dem das grenzflächenaktive Mittel ein Phospholipid umfasst, das aus der Gruppe, bestehend aus Phosphatiden, anionischen Phospholipiden, Phosphatidylserin, Phosphatidylinosit, Phosphatidylglycerin, Phosphatidylinosin, Phosphatidsäure, Lysophospholipiden, Polyethylenglykol-Phospholipid-Konjugaten, Ei-Phospholipiden, Sojabohnen-Phospholipiden, anionischen PEG-Phospholipiden und anionischen Methoxy-PEG-Phospholipiden, ausgewählt ist.

**35.** Verfahren nach Anspruch 30, bei dem das grenzflächenaktive Mittel ein kationisches grenzflächenaktives Mittel umfasst, das aus der Gruppe, bestehend aus quaternären Ammoniumverbindungen, Benzalkoniumchlorid, Cetyltrimethylammoniumbromid, Chitosanen, Lauryldimethylbenzylammoniumchlorid, Acylcamitinhydrochloriden, Alkylpyridiniumhalogeniden, Cetylpyridiniumchlorid, kationischen Lipiden, Polymethylmethacrylattrimethylammoniumbromid, Sulfoniumverbindungen, Polyvinylpyrrolidon-2-dimethyl-aminoethylmethacrylatdimethylsulfat, Hexadecyltrimethylammoniumbromid, Phosphoniumverbindungen, Benzyl-di(2-chlorethyl)ethylam-moniumbromid, Kokosnusstrimethylammoniumchlorid, Kokosnusstrimethylammoniumbromid, Kokosnussmethyldihydroxyethylammoniumchlorid, Kokosnussmethyldihydroxyethylammoniumbromid, Decyltriethylammoniumchlorid, Decyldimethylhydroxyethylammoniumchlorid, Decyldimethylhydroxyethylammoniumbromid, $C_{12-15}$-Dimethylhydroxyethylammoniumchlorid, $C_{12-15}$-Dimethylhydroxyethylammonium-bromid, Kokosnussdimethylhydroxyethylammoniumchlorid, Kokosnussdimethylhydroxyethylammoniumbromid, Myristyltrimethylammoniummethylsulfat, Lauryldimethylbenzylammoniumchlorid, Lauryldimethylbenzylammoniumbromid, Lauryldimethyl(ethenoxy)$_4$-ammoniumchlorid, Lauryldimethyl(ethenoxy)$_4$-ammoniumbromid, N-Alkyl($C_{12-18}$)dimethylbenzylammoniumchlorid, N-Alkyl($C_{14-18}$)dimethylbenzylammoniumchlorid, N-Tetradecyldimethylbenzylammoniumchloridmonohydrat, Dimethyldidecylammoniumchlorid, N-Alkyl($C_{12-14}$)dimethyl-1-naphthylmethylammoniumchlorid, Trimethylammoniumhalogenidalkyltrimethylammoniumsalzen, Dialkyldimethylammoniumsalzen, Lauryltrimethylammoniumchlorid, ethoxylierten Alkylamidoalkyldialkylammoniumsalzen, ethoxylierten Trialkylammoniumsalzen, Dialkylbenzoldialkylammoniumchlorid, N-Didecyldimethylammoniumchlorid, N-Tetradecyldimethylbenzylammoniumchloridmonohydrat, N-Alkyl ($C_{12-14}$)dimethyl-1-naphthylmethylammoniumchlorid, Dodecyldimethylbenzylammoniumchlorid, Dialkylbenzolalky-

lammoniumchlorid, Lauryltrimethylammoniumchlorid, Alkylbenzylmethylammoniumchlorid, Alkylbenzyldimethylammoniumbromid, $C_{12}$-Trimethylammoniumbromiden, $C_{15}$-Trimethylammoniumbromiden, $C_{17}$-Trimethylammoniumbromiden, Dodecylbenzyltriethylammoniumchlorid, Polydiallyldimethylammoniumchlorid (DADMAC™), Dimethylammoniumchloriden, Alkyldimethylammoniumhalogeniden, Tricetylmethylammoniumchlorid, Decyltrimethylammoniumbromid, Dodecyltriethylammoniumbromid, Tetradecyltrimethylammoniumbromid, Methyltrioctylammoniumchlorid, POLYQUAT™, Tetrabutylammoniumbromid, Benzyltrimethylammoniumbromid, Cholinestem, Stearalkoniumchlorid, Cetylpyridiniumbromid, Cetylpyridiniumchlorid, Halogenidsalzen von quaternisierten Polyoxyethylalkylaminen, MIRAPOL™, ALKAQUAT™, Alkylpyridiniumsalzen, Aminen, Aminsalzen, Imidazoliniumsalzen, protonierten quaternären Acrylamiden, methylierten quaternären Polymeren, kationischem Guargummi, Dodecyltrimethylammoniumbromid, Triethanolamin und Poloxaminen, ausgewählt ist.

**36.** Verfahren nach Anspruch 30, bei dem das grenzflächenaktive Mittel ein zwitterionisches grenzflächenaktives Mittel umfasst, das aus der Gruppe, bestehend aus zwitterionischen Phospholipiden, Phosphatidylcholin, Diacylglycerophosphoethanolamin, Phosphatidylethanolamin, Dimyristoylglycerophosphoethanolamin (DMPE), Dipalmitoylglycerophosphoethanolamin (DPPE), Distearoylglycerophosphoethanolamin (DSPE), Dioleoylglycerophosphoethanolamin (DOPE), pegylierten Phospholipiden, PEG-Phosphatdiylcholin, PEG-Diacylglycerophosphoethanolamin, PEG-Phosphatidylethanolamin, PEG-Dimyristoylglycerophosphoethanolamin, PEG-Dipalmitoylglycerophosphoethanolamin, PEG-Distearoylglycerophosphoethanolamin, PEG-Dioleoylglycerophosphoethanolamin, Methoxypolyethylenglykol(mPEG)-phospholipiden, mPEG-Phosphatidylcholin, mPEG-Diacylglycerophosphoethanolamin, mPEG-Phosphatidylethanolamin, mPEG-Dimyristoylglycerophosphoethanolamin, mPEG-Dipalmitoylglycerophosphoethanolamin, mPEG-Distearoylglycerophosphoethanolamin und mPEG-Dioleoylglycerophosphoethanolamin, ausgewählt ist.

**37.** Verfahren nach Anspruch 34, bei dem das grenzflächenaktive Mittel ein biologisch abgeleitetes grenzflächenaktives Mittel umfasst, das aus der Gruppe, bestehend aus Lipoproteinen, Gelatine, Kasein, Lysozym, Albumin, Heparin, Hirudin oder anderen Proteinen, ausgewählt ist.

**38.** Verfahren nach Anspruch 30, bei dem das grenzflächenaktive Mittel eine Aminosäure umfasst, die aus der Gruppe, bestehend aus Leucin, Alanin, Valin, Isoleucin, Lysin, Asparaginsäure, Glutaminsäure, Methionin und Phenylalanin, ausgewählt ist.

**39.** Verfahren nach einem der Ansprüche 29 bis 38, bei dem die Tubulin-Hemmstoffverbindung eine Verbindung gemäß einem der Ansprüche 2 bis 4 ist.

**40.** Verfahren nach einem der Ansprüche 29 bis 39, bei dem die Teilchen eine durchschnittliche Teilchengröße von etwa 10 Mikrometer oder weniger aufweisen.

**41.** Verfahren nach Anspruch 40, bei dem die Teilchen eine durchschnittliche Teilchengröße von etwa 2 Mikrometer oder weniger aufweisen.

**42.** Verwendung von Teilchen von etwa 15 nm bis etwa 50 Mikrometer von mindestens einer Tubulin-Hemmstoffverbindung nach Anspruch 1 bei der Herstellung eines Medikaments zur Behandlung von Säugern.

**43.** Verwendung nach Anspruch 42, bei der das Medikament zur Behandlung einer medizinischen Störung dient, die aus der Gruppe, bestehend aus immunologischen Störungen, Entzündungsstörungen, Antitumormittel-resistenten Tumoren, metastasierenden Karzinomen, einschließlich der Entwicklung und Ausbreitung von Metastasen, Tumoren, die bezüglich Tubulin-Hemmstoffen sensibel sind oder Tumoren, die sowohl Antitumormittel-resistent als auch bezüglich Tubulin-Hemmstoffen sensibel sind, Pankreatitis, septischem Schock, allergischer Rhinitis und rheumatoider Arthritis und Autoimmunerkrankungen, ausgewählt ist.

**44.** Verwendung nach Anspruch 42 oder 43, bei der die Tubulinverbindung eine Verbindung nach einem der Ansprüche 2 bis 4 ist.

**45.** Verwendung nach Anspruch 42, bei der das Medikament eine Antitumor-Aktivität, Antiasthma-Aktivität, antiallergische Aktivität, immununterdrückende Aktivität oder immunmodulierende Aktivität aufweist.

**46.** Teilchenförmige pharmazeutische Zusammensetzung nach einem der Ansprüche 1 bis 4 zur Verwendung bei der Behandlung von Säugern, die einer solchen Zusammensetzung bedürfen.

**47.** Teilchenförmige pharmazeutische Zusammensetzung nach Anspruch 46 zur Verwendung bei der Behandlung einer medizinischen Störung, die aus der Gruppe, bestehend aus immunologischen Störungen, Entzündungsstörungen, Antitumormittel-resistenten Tumoren, metastasierenden Karzinomen, einschließlich der Entwicklung und Ausbreitung von Metastasen, Tumoren, die bezüglich Tubulin-Hemmstoffen sensibel sind oder Tumoren, die sowohl Antitumormittel-resistent als auch bezüglich Tubulin-Hemmstoffen sensibel sind, Pankreatitis, septischem Schock, allergischer Rhinitis und rheumatoider Arthritis und Autoimmunerkrankungen, ausgewählt ist.

**48.** Teilchenförmige pharmazeutische Zusammensetzung nach Anspruch 46, die eine Antitumor-Aktivität, Antiasthma-Aktivität, antiallergische Aktivität, immununterdrückende Aktivität oder immunmodulierende Aktivität aufweist.

## Revendications

**1.** Composition pharmaceutique particulaire comprenant des particules présentant une taille efficace moyenne d'environ 15 nm à environ 50 microns d'au moins un inhibiteur de la tubuline de formule (1) :

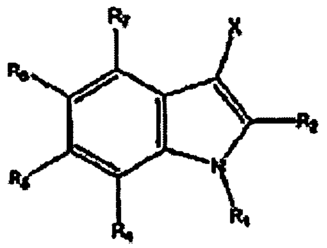

dans laquelle X représente

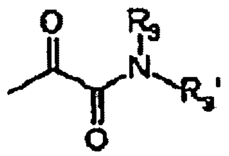

$R_3$ représente un groupe aryle ou hétéroaryle,
$R_{3'}$ représente un atome d'hydrogène,
$R_4$, $R_5$, $R_6$ et $R_7$ représentent indépendamment un atome d'hydrogène, d'halogène ou un groupe alkyle,
$R_1$ représente un groupe arylalkyle ; et
$R_2$ représente un atome d'hydrogène, un groupe alkyle, acyle, aryle, alcoxycarbonyle, aryloxycarbonyle, hétéroaryloxycarbonyle, cycloalcoxycarbonyle, hétérocycloalcoxycarbonyle, alcényloxycarbonyle, cycloalcényloxycarbonyle et hétérocycloalcényloxy-carbonyle ; et
au moins un surfactant choisi dans le groupe constitué de surfactants non ioniques, de surfactants anioniques, de surfactants cationiques, de surfactants d'origine biologique, de surfactants zwittérioniques et d'acides aminés.

**2.** Composition selon la revendication 1, où $R_1$ représente un groupe benzyle halogéné, $R_2$, $R_4$, $R_5$, $R_6$ et $R_7$ représentent un atome d'hydrogène et $R_3$ représente un groupe pyridine.

**3.** Composition selon la revendication 1, dans laquelle le composé inhibiteur de la tubuline est

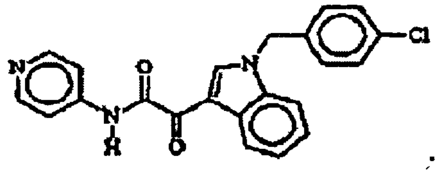

**4.** Composition selon la revendication 1, dans laquelle le composé inhibiteur de la tubuline est choisi dans le groupe

constitué par :

le N-(pyridin-4-yl)-[1-(4-fluorobenzyl)indol-3-yl]glyoxylamide ;
le N-(pyridin-3-yl)-[1-(4-fluorobenzyl)indol-3-yl]glyoxylamide ;
le N-(pyridin-3-yl)-(1-benzylindol-3-yl)glyoxylamide ;
le N-(pyridin-3-yl)-[1-(2-chlorobenzyl)indol-3-yl]glyoxylamide ;
le N-(4-fluorophényl)-[1-(4-fluorobenzyl)indol-3-yl]glyoxylamide ;
le N-(4-nitrophényl)-[1-(4-fluorobenzyl)indol-3-yl]glyoxylamide ;
le N-(2-chloropyridin-3-yl)-[1-(4-fluorobenzyl)-indol-3-yl]glyoxylamide ;
le N-(pyridin-4-yl)-(1-benzylindol-3-yl)glyoxylamide ;
le N-(pyridin-4-yl)-[1-(3-pyridylméthyl)indol-3-yl]glyoxylamide ;
le N-(4-fluorophényl)-[1-(2-pyridylméthyl)indol-3-yl]glyoxylamide ;
le N-(4-fluorophényl)-[1-(3-pyridylméthyl)indol-3-yl]glyoxylamide ;
le N-(pyridin-4-yl)-[1-(4-chlorobenzyl)indol-3-yl]glyoxylamide ;
le N-(pyridin-4-yl)-[1-(2-chlorobenzyl)indol-3-yl]glyoxylamide ;
le N-(pyridin-2-yl)-[1-(4-fluorobenzyl)indol-3-yl]glyoxylamide ;
le N-(pyridin-4-yl)-[1-(2-pyridylméthyl)indol-3-yl]glyoxylamide ;
le N-(pyridin-2-yl)-(1-benzylindol-3-yl)glyoxylamide ;
le N-(pyridin-4-yl)-[1-(4-fluorobenzyl)-6-éthoxy-carbonylaminoindol-3-yl]glyoxylamide ;
le N-(pyridin-4-yl)-[1-(4-fluorobenzyl)-5-éthoxy-carbonylaminoindol-3-yl]glyoxylamide ;
le N-(pyridin-4-yl)-[1-(4-fluorobenzyl)-6-cyclopentyloxycarbonylaminoindol-3-yl]glyoxylamide ;
le N-(pyridin-4-yl)-[1-(4-fluorobenzyl)-5-méthoxy-indol-3-yl]glyoxylamide ; et
le N-(pyridin-4-yl)-[1-(4-fluorobenzyl)-5-éthoxy-carbonylaminométhylindol-3-yl]glyoxylamide.

**5.** Composition selon l'une quelconque des revendications précédentes, dans laquelle le surfactant comprend un surfactant non ionique choisi dans le groupe constitué d'éthers d'alcools gras polyoxyéthylénés, d'esters d'acides gras de sorbitane polyoxyéthylénés, d'esters d'acides gras polyoxyéthylénés, d'esters de sorbitane, d'esters de glycéryle, de monostéarate de glycérol, de polyéthylène glycols, de polypropylène glycols, d'esters de polypropylène glycols, d'alcool cétylique, d'alcool cétostéarylique, d'alcool stéarylique, alcools polyéthers d'arylalkyle, de copolymères de polyoxyéthylène-polyoxypropylène, de poloxamères, de poloxamines, de méthylcellulose, d'hydroxycellulose, d'hydroxyméthylcellulose, d'hydroxypropylcellulose, d'hydroxypropylméthylcellulose, de cellulose non cristalline, de polysaccharides, d'amidon, d'hydroxyéthyl-amidon, de polyalcool vinylique, de polyvinylpyrrolidone, de stéarate de triéthanolamine, d'oxydes d'amines, de dextrane, de glycérol, de gomme arabique, de cholestérol, de gomme adragante, de cire émulsifiante au cétomacrogol, d'éthers d'alkyle polyoxyéthylénés, de stéarates de polyoxyéthylène, d'hydroxypropylméthylcellulose, de méthylcellulose, d'hydroxyéthylcellulose, de phtalate d'hydroxypropylméthylcellulose, de cellulose non cristalline, de polyalcool vinylique, de polyvinylpyrrolidone, de polymère de 4-(1,1,3,3-tétraméthylbutyl)phénol avec de l'oxyde d'éthylène et du formaldéhyde, des poloxamères, de sulfonates polyéthers d'alkylaryle, des mélanges de stéarate de saccharose et de distéarate de saccharose, de $C_{18}H_{37}CH_2C(O)N(CH_3)CH_2(CHOH))_4(CH_2OH)_2$, de p-isononyl-phénoxypoly(glycidol), de décanoyl-N-méthylglucamide, de n-décyl-β-D-glucopyranoside, de n-β-décyl-D-maltopyranoside, de n-dodécyl-β-D-glucopyranoside, de n-dodécyl-β-D-maltoside, de heptanoyl-N-méthylglucamide, de n-heptyl-β-D-glucopyranoside, de n-heptyl-β-D-thioglucoside, de n-hexyl-β-D-glucopyranoside ; de nonanoyl-N-méthylglucamide, de n-nonyl-β-D-glucopyranoside, d'octanoyl-N-méthylglucamide, de n-octyl-β-D-glucopyranoside, d'octyl-β-D-thioglucopyranoside, de PEG-cholestérol, de PEG-vitamine A, de PEG-vitamine E et de copolymères aléatoires d'acétate de vinyle et de vinylpyrrolidone.

**6.** Composition selon l'une quelconque des revendications 1 à 4, dans laquelle le surfactant comprend un surfactant anionique choisi dans le groupe constitué de sulfonates d'alkyle, de sulfonates d'aryle, de phosphates d'alkyle, de phosphonates d'alkyle, de laurate de potassium, de laurylsulfate de sodium, de dodécylsulfate de sodium, de sulfates d'alkyle polyoxyéthylénés, d'alginate de sodium, de sulfosuccinate de dioctyle sodique, d'acide phosphatidique et de ses sels, de carboxyméthylcellulose sodique, d'acides biliaires et de leurs sels, d'acide cholique, d'acide désoxycholique, d'acide glycocholique, d'acide taurocholique, d'acide glycodésoxycholique, de carboxyméthylcellulose calcique, d'acide stéarique et de ses sels, de stéarate de calcium, de phosphates, de dodécylsulfate de sodium, de carboxyméthylcellulose calcique, de carboxyméthylcellulose sodique, de dioctyl-sulfosuccinate, de dialkylesters de sodium, d'acide sulfosuccinique, de laurylsulfate de sodium et de phospholipides.

**7.** Composition selon la revendication 6, dans laquelle le surfactant comprend un phospholipide naturel ou synthétique.

**8.** Composition selon la revendication 7, dans laquelle le surfactant comprend un phospholipide choisi dans le groupe

constitué de phosphatides, de phospholipides anioniques, de phosphatidylsérine, de phosphatidylinositol, de phosphatidylglycérol, de phosphatidylinosine, d'acide phosphatidique, de lysophospholipides, de conjugués de polyéthylène glycol-phospholipide, de phospholipides d'oeuf, de phospholipides de soja, de PEG-phospholipides anioniques et de méthoxy-PEG-phospholipides anioniques.

**9.** Composition selon l'une quelconque des revendications 1 à 4, dans laquelle le surfactant comprend un surfactant cationique choisi dans le groupe constitué de composés d'ammonium quaternaire, de chlorure de benzalkonium, de bromure de cétyltriméthylammonium, de chitosanes, de chlorure de lauryldiméthylbenzylammonium, de chlorhydrates d'acyl-carnitine, d'halogénures d'alkyl-pyridinium, de chlorure de cétyl-pyridinium, de lipides cationiques, de bromure de polyméthylméthacrylate de triméthylammonium, de composés de sulfonium, de diméthylsulfate de méthacrylate de polyvinylpyrrolidone-2-diméthylaminoéthyle, de bromure d'hexadécyltriméthyl-ammonium, de composés de phosphonium, de bromure de benzyl-di(2-chloroéthyl)éthylammonium, de chlorure de triméthylammonium de noix de coco, de bromure de triméthylammonium de noix de coco, de chlorure de méthyl-dihydroxyéthyl-ammonium de noix de coco, de bromure de méthyl-dihydroxyéthyl-ammonium de noix de coco, de chlorure de décyl-triéthyl-ammonium, de chlorure de décyl-diméthyl-hydroxyéthyl-ammonium, de bromure de décyl-diméthyl-hydroxyéthyl-ammonium, de chlorure de diméthyl en $C_{12}$ à $C_{15}$-hydroxyéthyl-ammonium, de bromure diméthyl en $C_{12}$ à $C_{15}$-hydroxyéthyl-ammonium, de chlorure de diméthyl-hydroxyéthyl-ammonium de noix de coco, de bromure de diméthyl-hydroxyéthyl-ammonium de noix de coco, de méthylsulfate de myristyl-triméthyl-ammonium, de chlorure de lauryl-diméthyl-benzyl-ammonium, de bromure de lauryl-diméthyl-benzyl-ammonium, de chlorure de lauryl-diméthyl-(éthénoxy)$_4$-ammonium, de bromure de lauryl-diméthyl-(éthénoxy)$_4$-ammonium, de chlorure de N-alkyl-diméthyl en $C_{12}$ à $C_{18}$-benzyl-ammonium, de chlorure de N-alkyl-diméthyl en $C_{14}$ à $C_{18}$-benzyl-ammonium, de chlorure de N-tétradécyldiméthylbenzyl-ammonium monohydraté, de chlorure de diméthyl-didécyl-ammonium, de chlorure de N-alkyl-diméthyl en $C_{12}$ à $C_{14}$-1-naphtylméthyl-ammonium, de sels d'alkyl-triméthylammonium d'halogénures de triméthylammonium, de sels de dialkyl-diméthylammonium, de chlorure de lauryl-triméthyl-ammonium, de sels d'alkylamidoalkyldialkylammonium éthoxylés, de sels de trialkylammonium éthoxylés, de chlorure de dialkyl-benzène-dialkyl-ammonium, de chlorure de N-didécyl-diméthyl-ammonium, de chlorure de N-tétradécyldiméthylbenzyl-ammonium monohydraté, de chlorure de N-alkyl-diméthyl en $C_{12}$ à $C_{14}$-1-naphtylméthyl-ammonium, de chlorure de dodécyldiméthylbenzyl-ammonium, de chlorure de dialkyl-benzène-alkyl-ammonium, de chlorure de lauryl-triméthyl-ammonium, de chlorure d'alkylbenzyl-méthyl-ammonium, de bromure d'alkyl-benzyl-diméthyl-ammonium, de bromures de triméthyl en $C_{12}$-ammonium, de bromures de triméthyl en $C_{15}$-ammonium, de bromure de triméthyl en $C_{17}$-ammonium, de chlorure de dodécylbenzyl-triéthyl-ammonium, de chlorure de poly-diallyldiméthylammonium (DADMAC™), de chlorures de diméthyl-ammonium, d'halogénures d'alkyldiméthyl-ammonium, de chlorure de tricétyl-méthyl-ammonium, de bromure de décyltriméthylammonium, de bromure de dodécyltriéthylammonium, de bromure de tétradécyl-triméthylammonium, de chlorure de méthyl-trioctylammonium, de POLYQUAT™, de bromure de tétrabutylammonium, de bromure de benzyl-triméthylammonium, d'esters de choline, de chlorure de stéaralkonium, de bromure de cétylpyridinium, de chlorure de cétylpyridinium, de sels halogénures de polyoxyéthylalkylamines quaternisées, de MIRAPOL™, de ALKAQUAT™, de sels d'alkyl-pyridinium, d'amines, de sels d'amines, de sels d'imide-azolinium, d'acrylamides quaternaires protonés, de polymères quaternaires méthylés, de gomme guar cationique, de bromure de dodécyl-triméthyl-ammonium, de triéthanolamine et de poloxamines.

**10.** Composition selon l'une quelconque des revendications 1 à 4, dans laquelle le surfactant comprend un surfactant zwittérionique choisi dans le groupe constitué de phospholipides zwittérioniques, de phosphatidylcholine, de diacyl-glycéro-phosphoéthanolamine, de phosphatidyléthanolamine, de dimyristoyl-glycéro-phosphoéthanolamine (DMPE), de dipalmitoyl-glycéro-phosphoéthanolamine (DPPE), de distéaroyl-glycéro-phosphoéthanolamine (DSPE), de dioléolyl-glycéro-phosphoéthanolamine (DOPE), de phospholipides pégylés, de PEG-phosphatidylcholine, de PEG-diacyl-glycéro-phosphoéthanolamine, de PEG-phosphatidyléthanolamine, de PEG-dimyristoyl-glycéro-phosphoéthanolamine, de PEG-dipalmitoyl-glycéro-phosphoéthanolamine, de PEG-distéaroyl-glycéro-phosphoéthanolamine, de PEG-dioléolyl-glycéro-phosphoéthanolamine, de méthoxy-polyéthylène glycol (mPEG)-phospholipides, de mPEG-phosphatidylcholine, de mPEG-diacyl-glycéro-phosphoéthanolamine, de mPEG-phosphatidyléthanolamine, de mPEG-dimyristoyl-glycéro-phosphoéthanolamine, de mPEG-dipalmitoyl-glycéro-phosphoéthanolamine, de mPEG-distéaroyl-glycéro-phosphoéthanolamine et de mPEG-dioléolyl-glycéro-phosphoéthanolamine.

**11.** Composition selon l'une quelconque des revendications 1 à 4, dans laquelle le surfactant comprend un surfactant d'origine biologique choisi dans le groupe constitué de lipoprotéines, de gélatine, de caséine, de lysozyme, d'albumine, d'héparine, d'hirudine ou d'autres protéines.

**12.** Composition selon l'une quelconque des revendications 1 à 4, dans laquelle le surfactant comprend un acide aminé choisi dans le groupe constitué de leucine, d'alanine, de valine, l'isoleucine, de lysine, d'acide aspartique, d'acide

glutamique, de méthionine et de phénylalanine.

13. Composition selon l'une quelconque des revendications précédentes, comprenant en outre un agent ajustant le pH.

14. Composition selon la revendication 13, dans laquelle l'agent ajustant le pH est choisi dans le groupe constitué d'hydroxyde de sodium, d'acide chlorhydrique, de tampon tris, d'acides mono-, di-, tricarboxyliques et de leurs sels, de tampon citrate, de phosphate, d'acétate, de lactate, de tris(hydroxy-méthyl)aminométhane, d'aminosaccharides, d'amines mono-, di- et trialkylées, de méglumine (N-méthylglucosamine) et d'acides aminés.

15. Composition selon l'une quelconque des revendications précédentes, comprenant en outre un agent ajustant la pression osmotique.

16. Composition selon la revendication 15, dans laquelle l'agent ajustant la pression osmotique est choisi dans le groupe constitué de glycérine, de monosaccharides, de sels inorganiques et d'alcools de sucres.

17. Composition selon l'une quelconque des revendications précédentes, dans laquelle le composé inhibiteur de la tubuline est présent dans une quantité de 0,1 mg/g à 200 mg/g.

18. Composition selon la revendication 17, dans laquelle le composé inhibiteur de la tubuline est présent dans une quantité entre environ 0,5 mg/g et 50 mg/g.

19. Composition selon la revendication 17, dans laquelle le composé inhibiteur de la tubuline est présent dans une quantité entre environ 1 mg/g et 50 mg/g.

20. Composition selon l'une quelconque des revendications précédentes, dans laquelle les particules présentent une taille de particule efficace moyenne d'environ 10 microns ou inférieure.

21. Composition selon la revendication 20, dans laquelle les particules présentent une taille de particule efficace moyenne d'environ 2 microns ou inférieure.

22. Composition selon l'une quelconque des revendications précédentes, où ladite composition peut être administrée par une voie choisie dans le groupe constitué des voies parentérale, orale, buccale, parodontale, rectale, nasale, pulmonaire, topique, transdermique, intraveineuse, intramusculaire, sous-cutanée, intradermique, intraoculaire, intracérébrale, intralymphatique, pulmonaire, intra-articulaire, intrathécale et intrapéritonéale.

23. Composition selon l'une quelconque des revendications précédentes, où ladite composition est formulée sous une forme de dispersion liquide choisie dans le groupe constitué de formulations injectables, de solutions, de formulations à libération retardée, de formulations à libération contrôlée, de formulations à libération prolongée, de formulations à libération pulsatile et de formulations à libération immédiate.

24. Composition selon l'une quelconque des revendications 1 à 22, où ladite composition est formulée sous une forme galénique solide choisie dans le groupe constitué de comprimés, de comprimés enrobés, de capsules, d'ampoules, de suppositoires, de formulations lyophilisées, de formulations à libération retardée, de formulations à libération contrôlée, de formulations à libération prolongée, de formulations à libération pulsatile, de formulations à libération immédiate et à libération contrôlée.

25. Composition selon la revendication 22, où ladite composition est formulée sous une forme choisie dans le groupe constitué de patches, de préparations en poudre qui peuvent être inhalées, de suspensions, de crèmes et de pommades.

26. Procédé de fabrication d'une composition pharmaceutique particulaire selon la revendication 1 comprenant :

(i) la dissolution d'une quantité efficace d'au moins un composé inhibiteur de la tubuline tel que défini dans la revendication 1 dans un premier solvant miscible à l'eau pour former une solution ;
(ii) l'addition d'un surfactant ou d'une combinaison de surfactants ;
(iii) le mélange de la solution avec un second solvant pour former une pré-suspension ; et
(iv) l'addition d'énergie à la pré-suspension pour former une suspension de la composition pharmaceutique particulaire.

**27.** Procédé selon la revendication 26, dans lequel l'étape énergétique comprend une sonication, une homogénéisation, un broyage, une extrusion à cisaillement élevé ou une microfluidisation.

**28.** Procédé selon la revendication 26 ou 27, dans lequel le premier solvant est choisi dans le groupe constitué de N-méthyl-2-pyrrolidinone, d'acide lactique, de 2-pyrrolidone, de sulfoxyde de diméthyle, de diméthylacétamide, d'acide lactique, de méthanol, éthanol, d'isopropanol, de 3-pentanol, de n-propanol, de glycérol, de butylène glycol, d'éthylène glycol, de propylène glycol, de monoglycérides mono- et diacylés, de diméthylisosorbide, d'acétone, de diméthylformamide, de 1,4-dioxane, de polyéthylène glycol, d'esters de polyéthylène glycol, de polyéthylène glycol-sorbitanes, de polyéthylène glycol-éthers de monoalkyle, de polypropylène glycol, d'alginate de polypropylène, de PPG-10-butanediol, de PPG-10-éther de méthyl-glucose, de PPG-20-éther de méthyl-glucose, de PPG-15-éther de stéaryle, de dicaprylate de propylène glycol, de dicaprate de propylène glycol, de laurate de propylène glycol, de carbonate de propylène glycol, d'acide lactique et d'acide acétique.

**29.** Procédé selon l'une quelconque des revendications 26 à 28, dans lequel le second solvant est choisi dans le groupe constitué d'eau, de tampons, de sels, de surfactant(s), de polymères hydrosolubles et de combinaisons d'excipients.

**30.** Procédé selon l'une quelconque des revendications 26 à 29, dans lequel le surfactant est choisi dans le groupe constitué de surfactants non ioniques, de surfactants anioniques, de surfactants cationiques, de surfactants d'origine biologique, de surfactants zwittérioniques et d'acides aminés.

**31.** Procédé selon la revendication 30, dans lequel le surfactant comprend un surfactant non ionique choisi dans le groupe constitué d'éthers d'alcools gras polyoxyéthylénés, d'esters d'acides gras de sorbitane polyoxyéthylénés, d'esters d'acides gras polyoxyéthylénés, d'esters de sorbitane, d'esters de glycéryle, de monostéarate de glycérol, de polyéthylène glycols, de polypropylène glycols, d'esters de polypropylène glycols, d'alcool cétylique, d'alcool cétostéarylique, d'alcool stéarylique, alcools polyéthers d'arylalkyle, de copolymères de polyoxyéthylène-polyoxypropylène, de poloxamères, de poloxamines, de méthylcellulose, d'hydroxycellulose, d'hydroxyméthylcellulose, d'hydroxypropylcellulose, d'hydroxypropylméthylcellulose, de cellulose non cristalline, de polysaccharides, d'amidon, d'hydroxyéthyl-amidon, de polyalcool vinylique, de polyvinylpyrrolidone, de stéarate de triéthanolamine, d'oxydes d'amines, de dextrane, de glycérol, de gomme arabique, de cholestérol, de gomme adragante, de cire émulsifiante au cétomacrogol, d'éthers d'alkyle polyoxyéthylénés, de stéarates de polyoxyéthylène, d'hydroxypropylméthylcellulose, de méthylcellulose, d'hydroxyéthylcellulose, de phtalate d'hydroxypropylméthylcellulose, de cellulose non cristalline, de polyalcool vinylique, de polyvinylpyrrolidone, de polymère de 4-(1,1,3,3-tétraméthylbutyl)phénol avec de l'oxyde d'éthylène et du formaldéhyde, des poloxamères, de sulfonates polyéthers d'alkylaryle, des mélanges de stéarate de saccharose et de distéarate de saccharose, de $C_{18}H_{37}CH_2C(O)N(CH_3)CH_2(CHOH)_4(CH_2OH)_2$, de p-isononyl-phénoxypoly(glycidol), de décanoyl-N-méthylglucamide, de n-décyl-β-D-glucopyranoside, de n-β-décyl-D-maltopyranoside, de n-dodécyl-β-D-glucopyranoside, de n-dodécyl-β-D-maltoside, de heptanoyl-N-méthylglucamide, de n-heptyl-β-D-glucopyranoside, de n-heptyl-β-D-thioglucoside, de n-hexyl-β-D-glucopyranoside ; de nonanoyl-N-méthylglucamide, de n-nonyl-β-D-glucopyranoside, d'octanoyl-N-méthylglucamide, de n-octyl-β-D-glucopyranoside, d'octyl-β-D-thioglucopyranoside, de PEG-cholestérol, de PEG-vitamine A, de PEG-vitamine E et de copolymères aléatoires d'acétate de vinyle et de vinylpyrrolidone.

**32.** Procédé selon la revendication 30, dans lequel le surfactant comprend un surfactant anionique choisi dans le groupe constitué de sulfonates d'alkyle, de sulfonates d'aryle, de phosphates d'alkyle, de phosphonates d'alkyle, de laurate de potassium, de laurylsulfate de sodium, de dodécylsulfate de sodium, de sulfates d'alkyle polyoxyéthylénés, d'alginate de sodium, de sulfosuccinate de dioctyle sodique, d'acide phosphatidique et de ses sels, de carboxyméthylcellulose sodique, d'acides biliaires et de leurs sels, d'acide cholique, d'acide désoxycholique, d'acide glycocholique, d'acide taurocholique, d'acide glycodésoxycholique, de carboxyméthylcellulose calcique, d'acide stéarique et de ses sels, de stéarate de calcium, de phosphates, de dodécylsulfate de sodium, de carboxyméthylcellulose calcique, de carboxyméthylcellulose sodique, de dioctyl-sulfosuccinate, de dialkylesters de sodium, d'acide sulfosuccinique, de laurylsulfate de sodium et de phospholipides.

**33.** Procédé selon la revendication 32, dans lequel le surfactant comprend un phospholipide qui est naturel ou synthétique.

**34.** Procédé selon la revendication 33, dans lequel le surfactant comprend un phospholipide choisi dans le groupe constitué de phosphatides, de phospholipides anioniques, de phosphatidylsérine, de phosphatidylinositol, de phosphatidylglycérol, de phosphatidylinosine, d'acide phosphatidique, de lysophospholipides, de conjugués de polyéthylène glycol-phospholipide, de phospholipides d'oeuf, de phospholipides de soja, de PEG-phospholipides anioniques et de méthoxy-PEG-phospholipides anioniques.

35. Procédé selon la revendication 30, dans lequel le surfactant comprend un surfactant cationique choisi dans le groupe constitué de composés d'ammonium quaternaire, de chlorure de benzalkonium, de bromure de cétyltriméthylammonium, de chitosanes, de chlorure de lauryldiméthylbenzylammonium, de chlorhydrates d'acyl-carnitine, d'halogénures d'alkyl-pyridinium, de chlorure de cétyl-pyridinium, de lipides cationiques, de bromure de polyméthylméthacrylate de triméthylammonium, de composés de sulfonium, de diméthylsulfate de méthacrylate de polyvinylpyrrolidone-2-diméthylaminoéthyle, de bromure d'hexadécyltriméthyl-ammonium, de composés de phosphonium, de bromure de benzyl-di(2-chloroéthyl)éthylammonium, de chlorure de triméthylammonium de noix de coco, de bromure de triméthylammonium de noix de coco, de chlorure de méthyl-dihydroxyéthyl-ammonium de noix de coco, de bromure de méthyl-dihydroxyéthyl-ammonium de noix de coco, de chlorure de décyl-triéthyl-ammonium, de chlorure de décyl-diméthyl-hydroxyéthyl-ammonium, de bromure de décyl-diméthyl-hydroxyéthyl-ammonium, de chlorure de diméthyl en $C_{12}$ à $C_{15}$-hydroxyéthyl-ammonium, de bromure diméthyl en $C_{12}$ à $C_{15}$-hydroxyéthyl-ammonium, de chlorure de diméthyl-hydroxyéthyl-ammonium de noix de coco, de bromure de diméthyl-hydroxyéthyl-ammonium de noix de coco, de méthylsulfate de myristyl-triméthyl-ammonium, de chlorure de lauryl-diméthyl-benzyl-ammonium, de bromure de lauryl-diméthyl-benzyl-ammonium, de chlorure de lauryl-diméthyl-(éthénoxy)$_4$-ammonium, de bromure de lauryl-diméthyl-(éthénoxy)$_4$-ammonium, de chlorure de N-alkyl-diméthyl en $C_{12}$ à $C_{18}$-benzyl-ammonium, de chlorure de N-alkyl-diméthyl en $C_{14}$ à $C_{18}$-benzyl-ammonium, de chlorure de N-tétradécyldiméthylbenzylammonium monohydraté, de chlorure de diméthyl-didécyl-ammonium, de chlorure de N-alkyl-diméthyl en $C_{12}$ à $C_{14}$-1-naphtylméthyl-ammonium, de sels d'alkyl-triméthylammonium d'halogénures de triméthylammonium, de sels de dialkyl-diméthylammonium, de chlorure de lauryl-triméthyl-ammonium, de sels d'alkylamidoalkyldialkylammonium éthoxylés, de sels de trialkylammonium éthoxylés, de chlorure de dialkyl-benzène-dialkyl-ammonium, de chlorure de N-didécyl-diméthyl-ammonium, de chlorure de N-tétradécyldiméthylbenzyl-ammonium monohydraté, de chlorure de N-alkyl-diméthyl en $C_{12}$ à $C_{14}$-1-naphtylméthyl-ammonium, de chlorure de dodécyldiméthylbenzyl-ammonium, de chlorure de dialkyl-benzène-alkyl-ammonium, de chlorure de lauryl-triméthyl-ammonium, de chlorure d'alkylbenzyl-méthyl-ammonium, de bromure d'alkyl-benzyl-diméthyl-ammonium, de bromures de triméthyl en $C_{12}$-ammonium, de bromures de triméthyl en $C_{15}$-ammonium, de bromure de triméthyl en $C_{17}$-ammonium, de chlorure de dodécylbenzyl-triéthyl-ammonium, de chlorure de poly-diallyldiméthylammonium (DADMAC™), de chlorures de diméthyl-ammonium, d'halogénures d'alkyldiméthylammonium, de chlorure de tricétyl-méthyl-ammonium, de bromure de décyltriméthylammonium, de bromure de dodécyltriéthylammonium, de bromure de tétradécyl-triméthylammonium, de chlorure de méthyl-trioctylammonium, de POLYQUAT™, de bromure de tétrabutylammonium, de bromure de benzyl-triméthylammonium, d'esters de choline, de chlorure de stéaralkonium, de bromure de cétylpyridinium, de chlorure de cétylpyridinium, de sels halogénures de polyoxyéthylalkylamines quaternisées, de MIRAPOL™, de ALKAQUAT™, de sels d'alkyl-pyridinium, d'amines, de sels d'amines, de sels d'imide-azolinium, d'acrylamides quaternaires protonés, de polymères quaternaires méthylés, de gomme guar cationique, de bromure de dodécyl-triméthyl-ammonium, de triéthanolamine et de poloxamines.

36. Procédé selon la revendication 30, dans lequel le surfactant comprend un surfactant zwittérionique choisi dans le groupe constitué de phospholipides zwittérioniques, de phosphatidylcholine, de diacyl-glycéro-phospho-éthanolamine, de phosphatidyléthanolamine, de dimyristoyl-glycéro-phosphoéthanolamine (DMPE), de dipalmitoyl-glycérophosphoéthanolamine (DPPE), de distéaroyl-glycéro-phosphoéthanolamine (DSPE), de dioléolyl-glycéro-phosphoéthanolamine (DOPE), de phospholipides pégylés, de PEG-phosphatidylcholine, de PEG-diacyl-glycéro-phosphoéthanolamine, de PEG-phosphatidyléthanolamine, de PEG-dimyristoyl-glycéro-phosphoéthanolamine, de PEG-dipalmitoyl-glycéro-phosphoéthanolamine, de PEG-distéaroyl-glycéro-phosphoéthanolamine, de PEG-dioléolyl-glycéro-phosphoéthanolamine, de méthoxy-polyéthylène glycol (mPEG)-phospholipides, de mPEG-phosphatidylcholine, de mPEG-diacyl-glycéro-phosphoéthanolamine, de mPEG-phosphatidyléthanolamine, de mPEG-dimyristoyl-glycéro-phosphoéthanolamine, de mPEG-dipalmitoyl-glycéro-phosphoéthanolamine, de mPEG-distéaroyl-glycéro-phosphoéthanolamine et de mPEG-dioléolyl-glycéro-phosphoéthanolamine.

37. Procédé selon la revendication 34, dans lequel le surfactant comprend un surfactant d'origine biologique choisi dans le groupe constitué de lipoprotéines, de gélatine, de caséine, de lysozyme, d'albumine, d'héparine, d'hirudine ou d'autres protéines.

38. Procédé selon la revendication 30, dans lequel le surfactant comprend un acide aminé choisi dans le groupe constitué de leucine, d'alanine, de valine, l'isoleucine, de lysine, d'acide aspartique, d'acide glutamique, de méthionine et de phénylalanine.

39. Procédé selon l'une quelconque des revendications 29 à 38, dans lequel le composé inhibiteur de la tubuline est

un composé tel que revendiqué dans l'une quelconque des revendications 2 à 4.

**40.** Procédé selon l'une quelconque des revendications 29 à 39, dans lequel les particules présentent une taille de particule moyenne d'environ 10 microns ou inférieure.

**41.** Procédé selon la revendication 40, dans lequel les particules présentent une taille de particule moyenne d'environ 2 microns ou inférieure.

**42.** Utilisation de particules d'environ 15 nm à environ 50 microns d'au moins un composé inhibiteur de la tubuline selon la revendication 1 dans la fabrication d'un médicament destiné au traitement de mammifères.

**43.** Utilisation selon la revendication 42, où le médicament est destiné au traitement d'un trouble médical choisi dans le groupe constitué de troubles immunologiques, de troubles inflammatoires, de tumeurs résistantes à des agents antitumoraux, de carcinomes se métastasant y compris le développement et la propagation des métastases, de tumeurs sensibles aux inhibiteurs de la tubuline ou de tumeurs qui sont à la fois résistantes aux agents antitumoraux et sensibles aux inhibiteurs de la tubuline, d'une pancréatite, d'un choc septique, d'une rhinite allergique et de la polyarthrite rhumatoïde, et de maladies auto-immunes.

**44.** Utilisation selon la revendication 42 ou 43, où le composé inhibiteur de la tubuline est un composé tel que revendiqué dans l'une quelconque des revendications 2 à 4.

**45.** Utilisation selon la revendication 42, où le médicament possède une activité antitumorale, antiasthmatique, antiallergique, immunosuppressive ou immunomodulatrice.

**46.** Composition pharmaceutique particulaire selon l'une quelconque des revendications 1 à 4, pour une utilisation dans le traitement de mammifères en ayant besoin.

**47.** Composition pharmaceutique particulaire selon la revendication 46 pour une utilisation dans le traitement d'un trouble médical choisi dans le groupe constitué de troubles immunologiques, de troubles inflammatoires, de tumeurs résistantes à des agents antitumoraux, de carcinomes se métastasant est compris le développement et la propagation des métastases, de tumeurs sensibles aux inhibiteurs de la tubuline ou de tumeurs qui sont à la fois résistantes aux agents antitumoraux et sensibles aux inhibiteurs de la tubuline, d'une pancréatite, d'un choc septique, d'une rhinite allergique et de la polyarthrite rhumatoïde, et de maladies auto-immunes.

**48.** Composition pharmaceutique particulaire selon la revendication 46, possédant une activité antitumorale, antiasthmatique, antiallergique, immunosuppressive ou immunomodulatrice.

FIG. 1

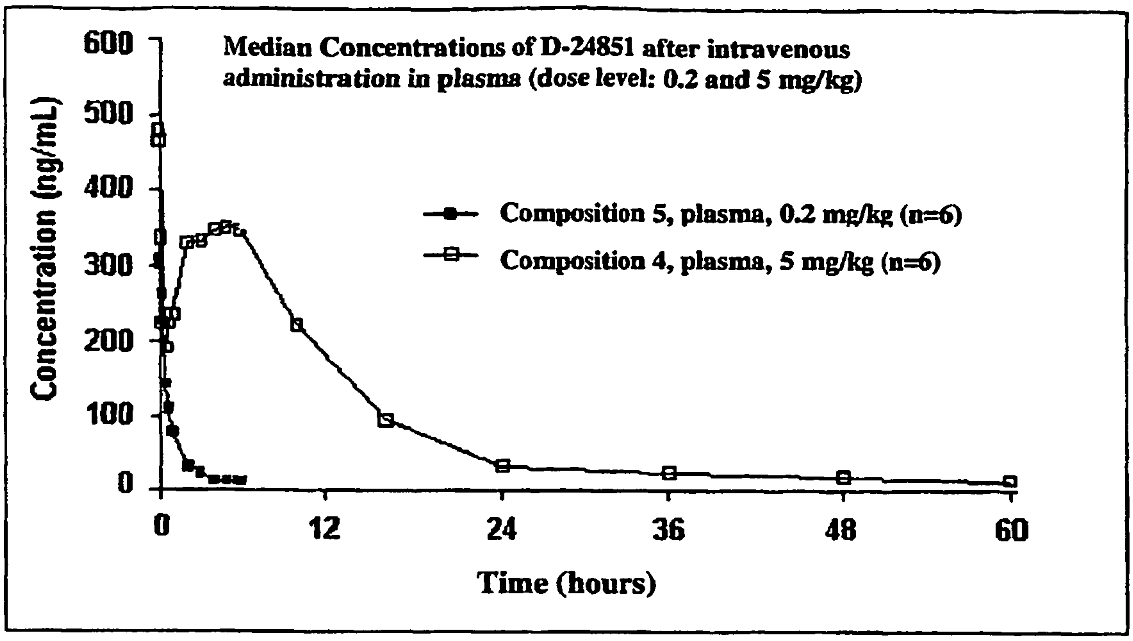
Median Concentrations of D-24851 after intravenous administration in plasma (dose level: 0.2 and 5 mg/kg)
Concentration (ng/mL)
600
500
400
300
200
100
0
Composition 5, plasma, 0.2 mg/kg (n=6)
Composition 4, plasma, 5 mg/kg (n=6)
0
12
24
36
48
60
Time (hours)

FIG. 2

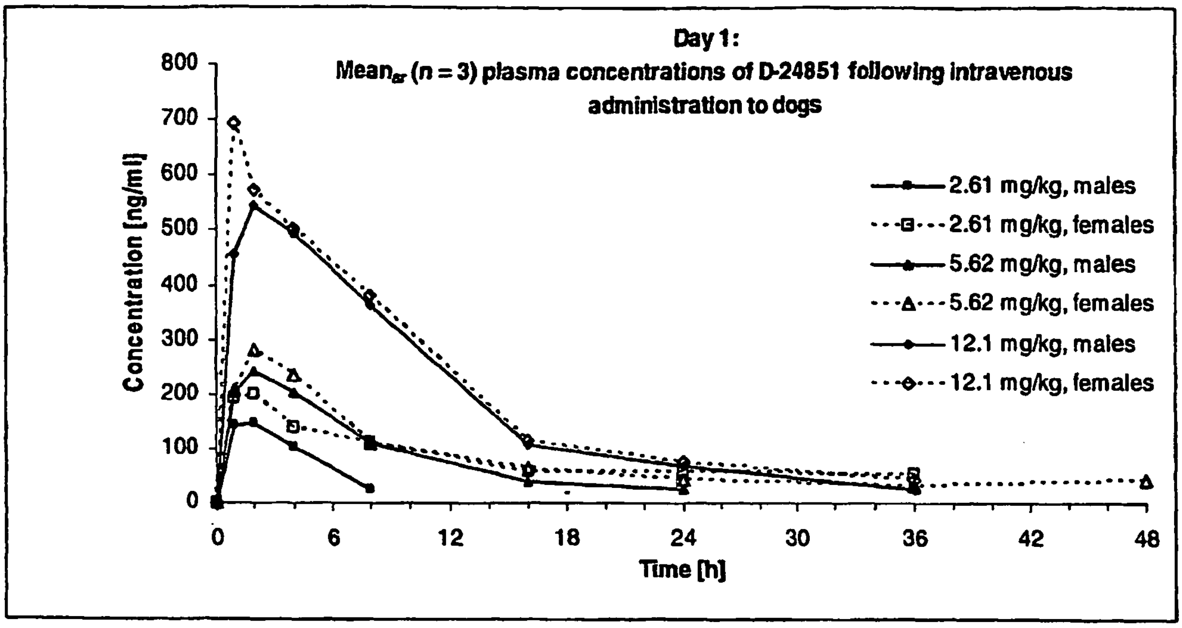
Day 1:
Mean$_{ar}$ (n = 3) plasma concentrations of D-24851 following intravenous administration to dogs
Concentration [ng/ml]
800
700
600
500
400
300
200
100
0
0
6
12
18
24
30
36
42
48
Time [h]
2.61 mg/kg, males
2.61 mg/kg, females
5.62 mg/kg, males
5.62 mg/kg, females
12.1 mg/kg, males
12.1 mg/kg, females

FIG. 3

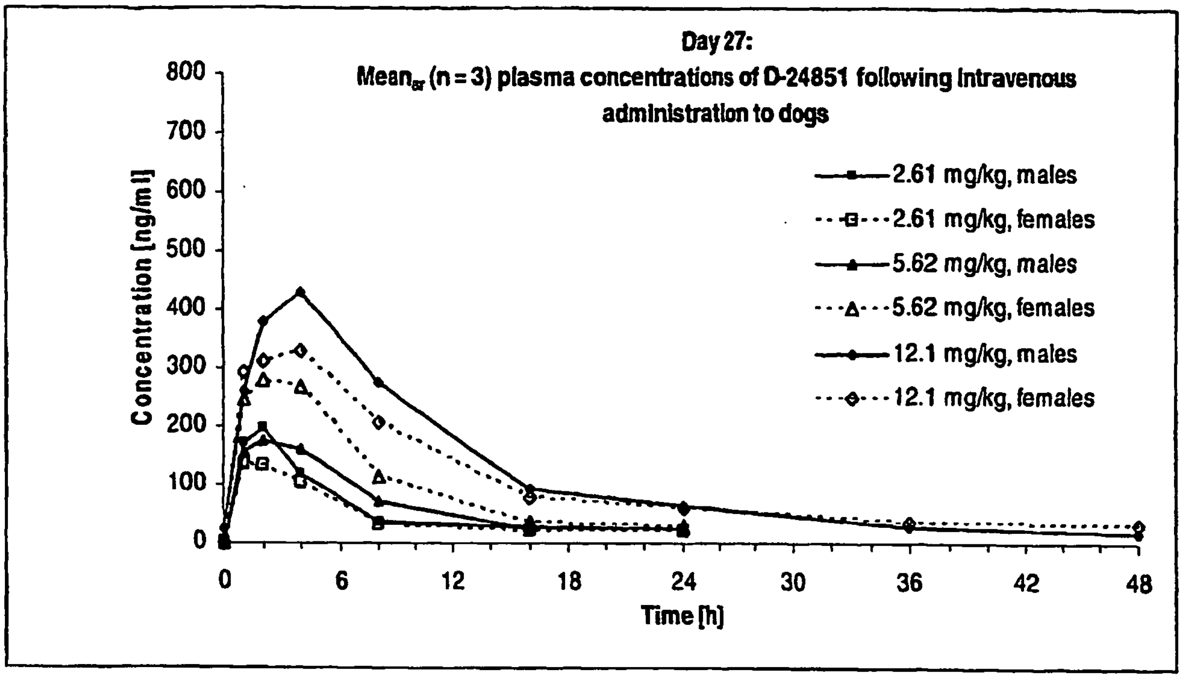
Day 27:
Mean$_{SF}$ (n = 3) plasma concentrations of D-24851 following intravenous administration to dogs
Concentration [ng/ml]
800
700
600
500
400
300
200
100
0
0
6
12
18
24
30
36
42
48
Time [h]
2.61 mg/kg, males
2.61 mg/kg, females
5.62 mg/kg, males
5.62 mg/kg, females
12.1 mg/kg, males
12.1 mg/kg, females

FIG. 4

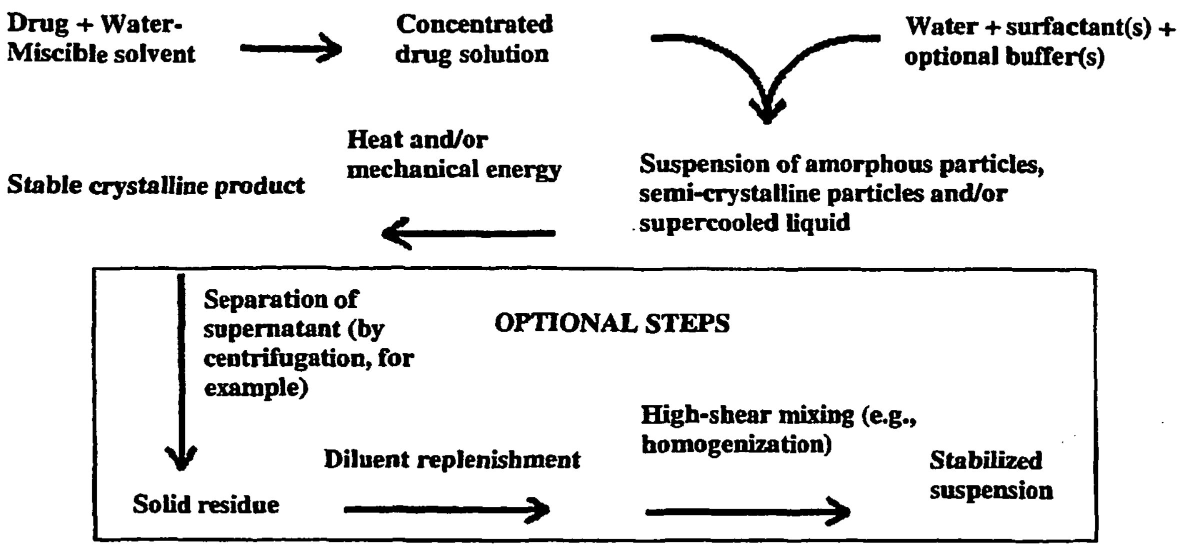
Drug + Water-Miscible solvent
Concentrated drug solution
Water + surfactant(s) + optional buffer(s)
Suspension of amorphous particles, semi-crystalline particles and/or supercooled liquid
Heat and/or mechanical energy
Stable crystalline product
Separation of supernatant (by centrifugation, for example)
OPTIONAL STEPS
Solid residue
Diluent replenishment
High-shear mixing (e.g., homogenization)
Stabilized suspension

FIG. 5

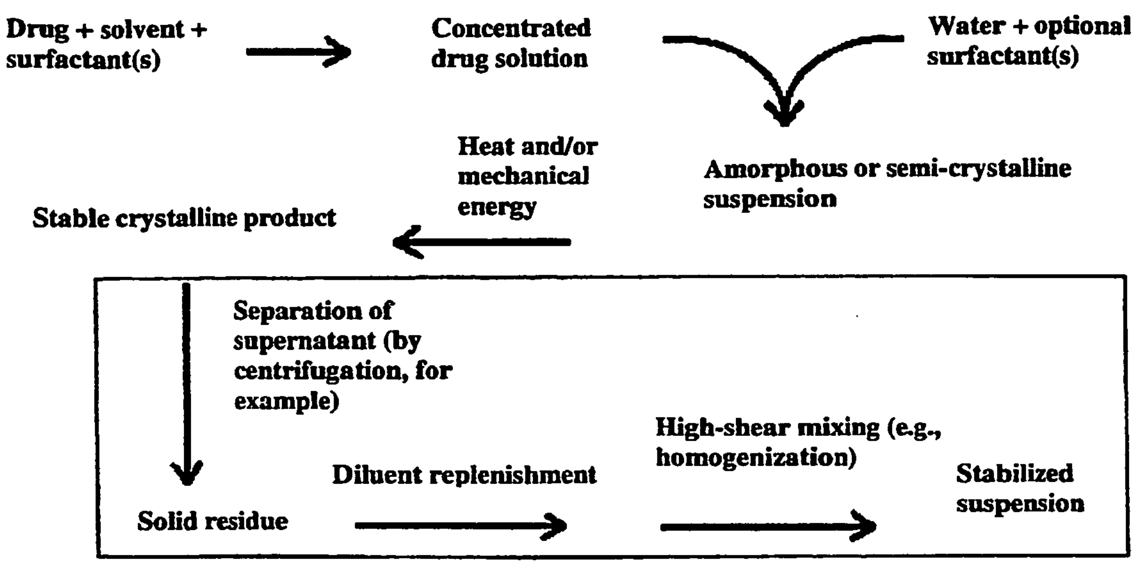
Drug + solvent + surfactant(s)
Concentrated drug solution
Water + optional surfactant(s)
Amorphous or semi-crystalline suspension
Heat and/or mechanical energy
Stable crystalline product
Separation of supernatant (by centrifugation, for example)
Solid residue
Diluent replenishment
High-shear mixing (e.g., homogenization)
Stabilized suspension

FIG. 6

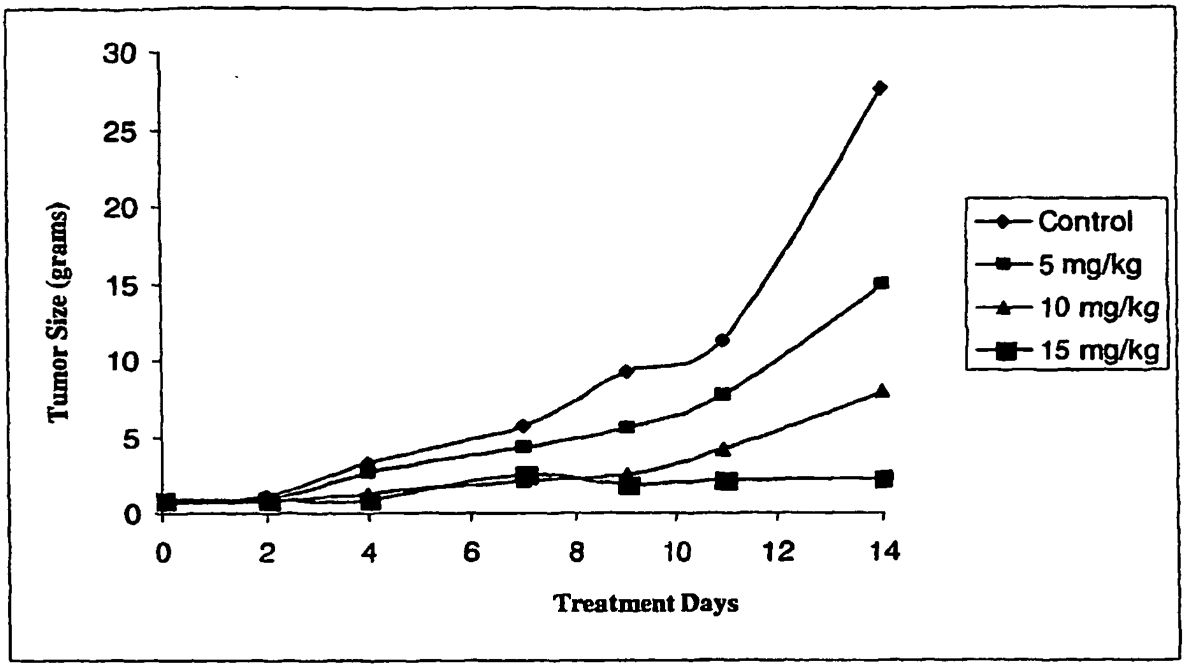
30
25
20
15
10
5
0
Tumor Size (grams)
0
2
4
6
8
10
12
14
Treatment Days
Control
5 mg/kg
10 mg/kg
15 mg/kg

FIG. 7

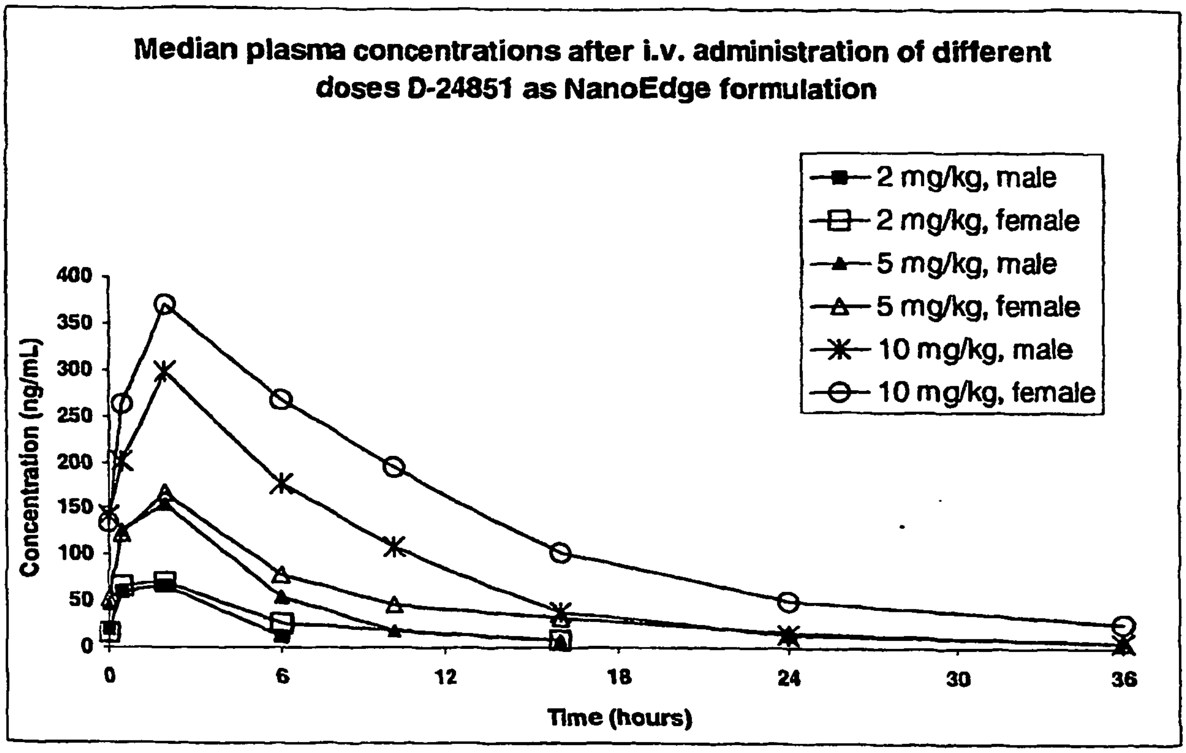
Median plasma concentrations after i.v. administration of different doses D-24851 as NanoEdge formulation
2 mg/kg, male
2 mg/kg, female
5 mg/kg, male
5 mg/kg, female
10 mg/kg, male
10 mg/kg, female
Concentration (ng/mL)
400
350
300
250
200
150
100
50
0
0
6
12
18
24
30
36
Time (hours)

FIG. 8

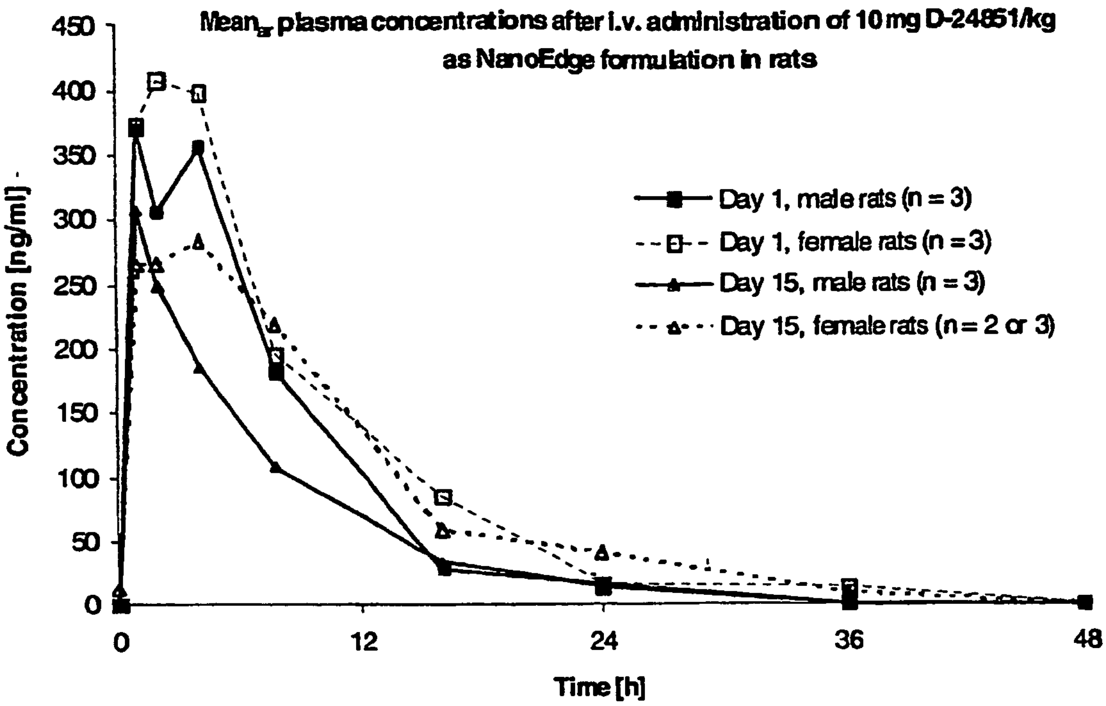
Mean plasma concentrations after i.v. administration of 10 mg D-24851/kg as NanoEdge formulation in rats
Concentration [ng/ml]
Time [h]
450
400
350
300
250
200
150
100
50
0
0
12
24
36
48
Day 1, male rats (n = 3)
Day 1, female rats (n = 3)
Day 15, male rats (n = 3)
Day 15, female rats (n = 2 or 3)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2745785 A
- US 5118528 A
- US 4826689 A
- US 5091188 A
- US 5091187 A
- US 4725442 A
- US 5145684 A
- US 5922355 A
- US 5780062 A
- US 5858410 A
- US 4997454 A
- US 5605785 A
- US 20020127278 A1
- US 6607784 B
- US 20020091124 A1
- US 6008231 A
- US 6232327 A
- US 6693119 A
- US 20030195244 A1
- US 20040033267 A1
- US 20030195244 A
- US 20030077329 A
- US 270267 A
- US 20020176935 A
- US 20020127278 A
- US 20020168402 A
- US 20050037083 A
- US 20030044433 A
- US 20030031719 A
- US 20030059472 A
- US 20030100568 A
- US 20030096013 A
- US 20030072807 A
- US 200377329 B
- US 2004022862 A
- US 258160 P
- US 09874799 P
- US 09874637 P
- US 09874499 P
- US 09964273 P
- US 10035821 P
- US 60347548 P
- US 10021692 P
- US 10183035 P
- US 10213352 P
- US 10246802 P
- US 10270268 P
- US 10270267 P
- US 10390333 P
- US 5720551 A
- US 5100591 A
- US 6235224 B
- US 6143211 B
- US 20010042932 A
- US 5665331 A
- US 5716642 A
- US 5662883 A
- US 5560932 A
- US 4608278 A
- US 5188837 A, Domb
- US 4973465 A
- US 6576264 B


### Non-patent literature cited in the description


- *Cancer Treatments - Chemotherapy,* 2003, www.cancerquest.org/index.cfm?page=520
- **BACHER et al.** *Pure Appl. Chem.,* 2001, vol. 73, 91459-1464
- **ROWINSKY ; DONEHOWER.** *Pharmac. Ther.,* 1991, vol. 52, 35-84
- New Small-Molecule Tubulin Ishibitors. *Pure Appl. Chem.,* 2001, vol. 73 (9
- International Union of Pure and Applied Chemistry. IUPAC Compendium of Chemical Terminology. 1997